# EUROPÄISCHE PATENTSCHRIFT

(11) **EP 3 115 023 B1**
(45) Veröffentlichungstag und Bekanntmachung des Hinweises auf die Patenterteilung: **20.12.2017**
(21) Anmeldenummer: 16178130.7
(22) Anmeldetag: 06.07.2016
(51) Int. Cl.: A61F 2/24, A61M 1/10, A61M 1/12

(54) **IMPLANTIERBARE VORRICHTUNG ZUR ORTSGENAUEN ZUFÜHRUNG UND APPLIKATION VON SUBSTANZEN IN DAS PERIKARD ODER AUF DIE HERZOBERFLÄCHE**
IMPLANTABLE DEVICE FOR PRECISE SUPPLY AND APPLICATION OF SUBSTANCES TO THE PERICARDIAL SAC OR THE SURFACE OF THE HEART
DISPOSITIF IMPLANTABLE D'INTRODUCTION A UN ENDROIT PRECIS ET D'APPLICATION DE SUBSTANCES DANS LE PERICARDE OU SUR LA SURFACE DU COEUR

(30) Priorität: 07.07.2015 DE 102015212699
(43) Veröffentlichungstag der Anmeldung: 11.01.2017
(73) Patentinhaber: AdjuCor GmbH, 85748 Garching b. München (DE)
(72) Erfinder: Wildhirt, Stephen Manuel, 80997 München (DE); Maier, Andreas, 85567 Grafing (DE); Müller, Kei Wieland, 69469 Weinheim (DE); Hofmann, Björn, 85661 Forstinning (DE)
(74) Vertreter: Peterreins Schley

(56) Entgegenhaltungen:
- WO-A1-2005/110514
- WO-A2-00/36995
- WO-A2-2005/091860
- US-A1- 2007 197 859

## Beschreibung

### Zusammenfassung

Die vorliegende Erfindung betrifft eine Vorrichtung zur Applikation von Substanzen auf die epikardiale Herzoberfläche. Die Vorrichtung umfasst eine Rahmenstruktur. Die Rahmenstruktur kann zumindest eine Hülle enthalten. Die Vorrichtung umfasst einen Substanzträger der geeignet ist die zu applizierende Substanz aufzunehmen.

### Hintergrund

Die Erfindung dient der Behandlung einer Herzinsuffizienz, z.B. nach vorangegangenem Myokardinfarkt. Ihr Zweck ist die Unterstützung der Pumpfunktion des Herzens, ihre Erhöhung sowie die Regeneration beschädigten Herzgewebes und somit die mittel- und langfristige Reduktion der Insuffizienz bzw. die Vermeidung des terminalen Stadiums.

Herz-Kreislauf-Erkrankungen wie die terminale Herzinsuffizienz oder der Myokardinfarkt stellen weltweit die Haupttodesursache dar (Behfar, A. and A. Terzic. Nature clinical practice Cardiovascular medicine 3 Suppl 1, S78-82, 2006). 32 % der weltweiten Todesfälle sind Folge von Herz-Kreislauf-Erkrankungen (World Health Statistics, http://apps.who.int/iris/ bitstream/10665/81965/1/9789241564588_eng.pdf (2013)2013). 40 % aller Todesfälle in Deutschland sind auf Herz-Kreislauf-Erkrankungen zurückzuführen (Statistisches Bundesamt, https://www.destatis.de/DE/Publikationen/ Thematisch/Gesundheit/Todesursachen/Todesursachen.html, 2013). In den USA erleiden jährlich ca. eine Million Menschen einen Herzinfarkt und sechs Millionen leiden an einer sich ergebenden Herzinsuffizienz; die direkten und indirekten Kosten werden jährlich auf 180 Milliarden Dollar geschätzt (Christoforou et al.. Plos One 8, 17, 2013). Die medizinischen, gesellschaftlichen und ökonomischen Implikationen sind enorm und werden weltweit in den kommenden Jahren aufgrund wachsenden Wohlstands, des damit verknüpften Lebensstils und der demographischen Entwicklung weiter an Gewicht gewinnen.

Als Herzinsuffizienz wird die Einschränkung der Pumpfunktion des Herzens bezeichnet. Das primäre Symptom ist eine verringerte Pumpleistung des Herzens. Kompensiert wird der Leistungsabfall durch Vergrößerung des Herzvolumens. Infolgedessen nimmt die Dicke der Herzwand ab, die Kontraktionsfähigkeit und somit die Pumpleistung sinkt weiter. Am Ende dieser sich selbst verstärkenden Entwicklung stehen Herzversagen oder auch der Ausfall anderer Organe aufgrund von Sauerstoff- und Nährstoffmangel.

Die verschiedenen Ausprägungen der Herzinsuffizienz können medikamentös (z.B. durch Verabreichung von Beta-Blockern) oder operativ behandelt werden. Die einzige Therapieoption bei terminaler Herzinsuffizienz ist die Herztransplantation. Der großen Nachfrage nach Spenderherzen steht jedoch ein ungenügendes Angebot gegenüber (Sasaki, D. and T. Okano. Embryonic Stem Cells - Recent Advances in Pluripotent Stem Cell-Based Regenerative Medicine, 65-80, 2011). Herzschrittmacher können Störungen in der Erregungsleitung ausgleichen, Prothesen defekte Herzklappen ersetzen und Blutpumpen (bspw. ventricular assist devices, VADs) die Leistung insuffizienter Herzen anheben. Durch die genannten Methoden wird die Entwicklung der Insuffizienz jedoch nur verzögert, nicht aber aufgehalten oder die Krankheit geheilt (Christoforou *et al.,* 2013). Ein die verschiedenen Ursachen der Herzinsuffizienz übergreifend behandelnder Ansatz ist die Unterstützung der Pumpfunktion des Herzens durch ein Implantat, das auf das Herz einen mechanischen Druck ausübt und damit dessen Pumpleistung unmittelbar und auch mittel- bis langfristig verbessert.

Die Herzinsuffizienz kann sich beispielsweise infolge eines Herzinfarkts ausprägen. Bei einem Herzinfarkt führt der plötzliche Verschluss zumindest einer Koronararterie zum Zelltod von Herzmuskelzellen. Die eingeschränkte Regenerationsfähigkeit des geschädigten Herzmuskelgewebes erlaubt keine volle und selbständige Genesung. Abgestorbenes Gewebe wird vielmehr durch kontraktionsunfähiges Narbengewebe ersetzt. Die Herzpumpleistung verringert sich und es erfolgt der Eintritt in die zuvor beschriebene Spirale der Degeneration. Medizinisch, gesellschaftlich und ökonomisch besteht der Bedarf zur Entwicklung effektiver, regenerativer Behandlungsformen, welche die vorhandene Pumpfunktion des Herzens unterstützen und erhöhen, die Bildung von Narbengewebe nach einem Herzinfarkt vermeiden, die Regeneration von Herzmuskelzellen fördern und in Konsequenz eine terminaler Herzinsuffizienz und Herzversagen verhindern.

Bisher bekannte mechanische Herzunterstützungsvorrichtungen sind bspw. in den US Patenten US 5,749,839 B1 und US 6,626,821 B1 und in der WO Anmeldung 00/25842 offenbart. Sie offenbaren Vorrichtungen, die den Nachteil haben, dass deren Implantation eine Operation am offenen Brustkorb erfordert. Des Weiteren sind sie komplex und können nur mit einer aufwendigen chirurgischen Operation implantiert werden. Sie werden in den Blutkreislauf der Patienten integriert. Verbesserte Zentrifugalpumpen oder magnetisch gelagerte Impellersysteme befördern das Blut kontinuierlich. Der Kontakt des Blutes mit der körperfremden Oberfläche der Systeme ist eine große technische und medizinische Herausforderung. Gängige Komplikationen dieser Systeme sind Schlaganfälle, Blutungen und Infektionen. Sie führen nicht selten zur Langzeithospitalisierung und zu häufigen Wiederaufnahmen bereits aus dem Krankenhaus entlassener Patienten.

Andere bekannte Herzunterstützungsvorrichtungen, wie z.B. die in US 2008/0021266 A1, DE 10 2009 043 795 A1, US 2004/0267329 A1, US 2005/0107661 A1, US 2006/0217774 A1, US 2007/0197859 A1, US 2009/0036730 A1, US 2011/0021864 A1, US 8944987 B2 und EP 2482865 B1 offenbarten Vorrichtungen haben den Nachteil, dass keine Ausführungsformen existieren, welche einer räumlichen Beeinträchtigung der Vena cava inferior durch die implantierte Vorrichtung vorbeugen. Die Vena cava inferior mündet von dorsal in den rechten Vorhof. Eine räumliche Beeinträchtigung der Vena cava inferior durch die Vorrichtung würde zu einer unteren Einflussstauung führen (Behinderung der Befüllung des rechten Vorhofs). Ein weiterer Nachteil bekannter Herzunterstützungssysteme ist, dass keine Vorkehrungen gegen eine Dislokation der Vorrichtung gegenüber dem Herzen getroffen werden. Eine Dislokation führt zu einer schlechteren Passform der Vorrichtung zum Herzen und zum Verlust der Unterstützungsleistung. Zudem ist keine dieser Vorrichtungen selbst-expandierende, d.h., sie können nur mithilfe weiterer Vorrichtungen in ihre Zielposition, in welcher sie ein Herz umfassen, gebracht werden.

Weitere bekannte Herzunterstützungssysteme, wie z.B. die in EP 2752208 A1 offenbarten Vorrichtungen, sind zwar selbst-expandierend und weisen eine Aussparung auf, welche verhindert, dass die Vena Cava inferior oder andere anatomische Merkmale in Herznähe räumlich beeinträchtig werden, jedoch fehlt Ihnen die Fähigkeit, ortsgenau und gezielt Substanzen, bspw. pharmazeutische Wirkstoffe, auf das Epikard zu applizieren.

Ein vielsprechender Ansatz zur regenerativen Therapie von ischämischen Gewebe nach einem Herzinfarkt stellt die Behandlung mit Stammzellen (Vizzardi, E. et al. J. Card. Surg., 53, 685-689, 2012) oder mit sogenannten Wachstumsfaktoren (Korf-Klingebiel, M. et al.. Nature Medicine 21, 52-61, 2015) dar. Stammzellen können unter bestimmten Bedingungen zu Kardiomyozyten ausdifferenzieren und abgestorbene Zellen ersetzen (Sasaki und Okano, 2011). Bislang wurden Stammzellen mit geringem Therapieerfolg vor allem in den Blutkreislauf injiziert (US 2009/0285787 A1) oder mittels Katheter in der Koronararterie freigesetzt (US 8372054 B2, US 20070065414 A1, US 20120157751 A1, US 20140072611 A1). Nur ein geringer Anteil der Zellen (∼3%) oder anderer therapeutischer Substanzen erreicht den Zielort (Hou, D. M. et al. Circulation 112, I150-I156, 2005). Die Injektion von Zellen in die Koronararterie kann zu einer Verstopfung der Arterie führen. Alle genannten Methoden arbeiten mit einer Freisetzung von Zellen oder therapeutischen Substanzen im Blutkreislauf, was zu Blutungen, Infektionen, Thrombusbildung und somit zu Herzinfarkten und Schlaganfällen führen kann. Eine andere Verabreichungsform ist die Injektion von Zellen in das Myokard (US 6659950 B2, US 20100168713 A1). Aufgrund der Herzbewegung gehen diese Methoden mit einem hohen Risiko für Gewebs- und Gefäßverletzungen sowie von Fehlinjektionen einher, ohne zu einer erfolgreicheren Therapie zu führen (Jawad, H., A. R. Lyon, S. E. Harding, N. N. Ali and A. R. Boccaccini, 2008, Myocardial tissue engineering. British Medical Bulletin, 87, 31-47.). Weiterhin ist es zur Erhöhung des therapeutischen Wirkungsgrades oftmals notwendig, zusätzlich chemische Signalstoffe zeitlich und örtlich definiert zu verabreichen, was mit den genannten Methoden nicht möglich ist. Keine der offenbarten Vorrichtungen können Substanzen über eine implantierbare Vorrichtung in die Perikardhöhle oder auf die Herzoberfläche applizieren. Auch kann keine offenbarte Vorrichtung bereits in der Perikardhöhle applizierte Substanzen nach der Implantation zu- und abführen.

Eine all diese Nachteile behebende Vorrichtung wird hier beschrieben. Die vorliegende Erfindung betrifft eine Vorrichtung zur Unterstützung und Wiederherstellung der Funktion eines Herzens. Ein Aspekt der Erfindung ist eine Vorrichtung zur ortsgenauen Zuführung von Substanzen auf die Herzoberfläche oder in das Perikardhöhle hinein, die nicht die Nachteile der bekannten Vorrichtungen und/oder Verabreichungsmethoden zur Applikation von therapeutischen Substanzen zur Regeneration von ischämischen Herzgewebe nach einem Myokardinfarkt aufweist. Die Vorrichtung kann minimalinvasiv mithilfe eines Katheters implantiert werden. Sie wird nicht in ein Gefäßsystem eingeführt und hat somit keinen dauerhaften unmittelbaren Blutkontakt, was zu einer erheblichen Reduzierung der Infektionsgefahr, der Thrombusbildung und von Blutungen im Vergleich zu anderen Applikationssytemen von Substanzen zur Regeneration von ischämischen Gewebe nach einem Herzinfarkt. Die Vorrichtung kann für eine bestimmte Zeit im Körper verbleiben und nach Abschluss der Therapie minimalinvasiv explantiert werden.

WO 2005/110514 A1 beschreibt eine Vorrichtung zur epikardialen Unterstützung und/oder Übernahme der Herztätigkeit mit einer Doppelmembran, einer elastischen inneren Membran und einer nicht dehnbaren äußeren Membran und einem dazwischen gebildeten geschlossenen und mittels eines Fluids inflatierbaren und deflatierbaren Hohlraum.

### Zusammenfassung der Erfindung

Die vorliegende Erfindung wird in den Ansprüchen definiert. Die Vorrichtung zur Applikation einer Substanz auf eine Herzoberfläche umfasst eine Rahmenstruktur, eine Hülle, ein Substanzträger und eine zu applizierende Substanz. Der Substanzträger ist eine Tasche und die zu applizierende Substanz ist in die Tasche eingebracht. Die Tasche hat einen entfernbaren Bereich auf der herzzugewandten Fläche der Tasche.

Die Vorrichtung kann weiterhin eine expandierbare Einheit umfassen, die an, in, oder auf der Hülle angebracht ist. Die expandierbare Einheit kann auch Teil der Hülle sein. Der Substanzträger kann auf der expandierbaren Einheit oder auf der Hülle angebracht sein. Anbringen des Substanzträgers auf der expandierbaren Einheit ermöglicht die Applikation der Substanz unter gleichzeitigem Ausüben von Druck auf die Herzoberfläche.

Die Rahmenstruktur kann reversibel selbst-expandierend sein. Sie kann minimalinvasiv in den Körper eingebracht werden und kann im expandierten Zustand ein Herz zumindest teilweise umschließen.

Der Substanzträger ist eine Tasche. Die zu applizierende Substanz ist in die Tasche eingebracht sein. Die Substanz kann direkt in die Tasche eingebracht werden oder auf einen Träger aufgebracht werden, der in die Tasche eingebracht wird.

Die Tasche hat einen entfernbaren Bereich auf der herzzugewandten Fläche der Tasche. In einigen Ausführungsformen kann der entfernbare Bereich nach der Implantation der Vorrichtung von außerhalb des Körpers entfernt werden kann. Der entfernbare Bereich kann zumindest teilweise bioabbaubar sein. Die Tasche kann auch einen permeablen Bereich umfassen.

Der Substanzträger kann eine Zuleitung zum Befüllen und Entleeren des Substanzträgers mit einer zu applizierenden Substanz aufweisen. Der Substanzträger kann eine schwammartige oder poröse Struktur haben. Dadurch wird die Oberfläche und Speicherkapazität des Substanzträgers erhöht. Bei der Applikation von Zellen ist eine derartige Struktur auch vorteilhaft, da Zellen in derartigen Strukturen besser haften und/oder proliferieren.

Die Hülle kann an der Rahmenstruktur befestigt sein. Die Vorrichtung minimalinvasiv implantiert werden kann. Die Rahmenstruktur kann aus Draht gefertigt sein, insbesondere aus einem Draht umfassend eine Formgedächtnislegierung.

Die zu applizierende Substanz kann ein pharmazeutischer Wirkstoff sein. Die zu applizierende Substanz kann zusätzlich oder alternativ Zellen umfassen.

Ausführungsformen der vorliegenden Erfindung umfassen eine Versorgungseinheit. Diese kann ein Substanz-Reservoir umfassen.

Ein weiterer Aspekt der Erfindung betrifft eine Vorrichtung zum Implantieren der Applikationsvorrichtung. Die Vorrichtung zum Implantieren kann ein rohrförmiges Zuführsystem umfassen. Das rohrförmige Zuführsystem kann eine distale Stirnfläche haben, die abgeschrägt ist. Dies kann die Implantation erleichtern.

Ein weiterer Aspekt der Erfindung betrifft eine Vorrichtung zum Explantieren der Applikationsvorrichtung. Die Vorrichtung zum Explantieren kann ein zylindrisches Rohr umfassen, wobei das zylindrische Rohr am distalen Ende eine radiale Aufweitung umfassen kann. Diese Explantationsvorrichtung kann eine minimalinvasive Explantation des Implantats aus der Perikardhöhle ermöglichen.

Ein weiterer Aspekt betrifft ein Verfahren zum Herstellen einer Vorrichtung zur Applikation einer Substanz auf eine Herzoberfläche. Das Verfahren umfasst die Schritte des Bereitstellens einer Rahmenstruktur, des Anbringens einer Hülle an der Rahmenstruktur, des Anbringens eines Substanzträgers und des Beladens des Substanzträgers mit einer zu applizierenden Substanz. Der Substanzträger ist eine Tasche und die zu applizierende Substanz ist in die Tasche eingebracht. Die Tasche hat einen entfernbaren Bereich auf der herzzugewandten Fläche der Tasche. Der Substanzträger kann auf der Hülle angebracht werden. Die Vorrichtung kann eine expandierbare Einheit umfassen. Der Substanzträger kann auf der expandierbaren Einheit angebracht werden.

Die Vorrichtung zur Applikation einer Substanz auf eine Herzoberfläche kann weitere Komponenten umfassen. Beispielsweise können Leitungen zur Zu- und Abführung der zu applizierenden Substanz vorgesehen sein. Weiterhin können mechanische Kräfte auf die applizierten Substanzen oder die epikardiale Herzoberfläche des Myokards ausgeübt werden. Die Kräfte können durch die expandierbaren Einheiten aufgebaut werden. Alternativ oder zusätzlich können Kräfte auf den Substanzträger aufgebaut werden, die die Applikation verbessern.

Ein weiterer Aspekt betrifft eine Motoreinheit, welche mittels eines Elektromotors einen Druckspeicher von veränderlichem Volumen ansteuert, um den Expansionsgrad der zumindest einen expandierbaren Einheit einzustellen.

Weiterhin können manche Ausführungsformen der erfindungsgemäßen Vorrichtung energetisch passiv sein, d.h. ohne Energiezufuhr von außerhalb und/oder innerhalb des Körpers funktionieren.

Des Weiteren ist ein Aspekt der erfindungsgemäßen Vorrichtung, dass diese über Vorrichtungen verfügen kann, welche das Verletzungsrisiko für das umliegende Gewebe durch fertigungsbedingte spitze oder scharfkantige Komponenten der Rahmenstruktur minimieren.

Weiterhin wird eine Schleuse zum Einbringen in einen chirurgischen Zugang beschrieben, wobei die Schleuse ein proximales und ein distales Ende hat und an dem proximalen Ende zumindest eine Lamelle hat. Die Schleuse kann eine vorwiegend rotationssymmetrische Form haben. Die Schleuse kann ferner eine Dichtlippe und eine Halsregion umfassen. Die Schleuse vereinfacht das Einsetzen von Implantaten durch den chirurgischen Zugang. Weitere Einzelheiten zur Schleuse ergeben sich aus der detaillierten Beschreibung.

Weitere Ausführungsformen und Vorteile ergeben sich aus der detaillierten Beschreibung.

### Beschreibung der Zeichnungen

**Figur 1** zeigt einen menschlichen Torso mit Komponenten der implantierten Vorrichtung mit einer extrakorporalen Versorgungseinheit.
**Figur 2** zeigt eine Ausführungsform des Implantats im expandierten und versiegelten Zustand, in welchem sie ein Herz teilweise umschließt und im Inneren des Perikards liegt.
**Figur 3** zeigt einen Schnitt durch ein Herz mit Infarkt, welches von einer inflatierbaren, pneumatischen Kammer und einem darauf befindlichen Substanzträger umfasst wird.
**Figur 4** zeigt eine Versorgungseinheit eines Implantats.
**Figur** 5 zeigt einen Multikonnektor, mit welchem der Kabelbaum eines Implantats mit einer Versorgungseinheit gekoppelt werden kann.
**Figur 6** zeigt den Einbringungsvorgang einer Schleuse in das Perikard
**Figur 7** zeigt einen Komplex aus Schleuse, einem in diese eingebrachten Zuführsystems und eine darin eingebrachte Ausführungsform des Implantats. Der Ausbringungsvorgang des Implantats in das Perikard ist dargestellt.
**Figur 8** zeigt eine räumliche und eine abgerollte Ansicht der expandierbaren Rahmenstruktur.
**Figur 9** zeigt ein geschlitztes Rohr mit angedeutetem Schnittmuster sowie den Vorgang des Aufziehens eines geschlitzten Rohres auf einen Dorn.
**Figur 10** zeigt eine Ausführungsform eines Implantats, bei welcher auf einer pneumatischen Kammer auf der Innenseite einer Hülle ein Substanzträger in Form einer Tasche angebracht ist.
**Figur 11** zeigt verschiedene mögliche Ausführungsformen des Substanzträgers
**Figur 12** zeigt zwei Gesamtansichten von Ausführungsformen, bei welchen der Substanzträger direkt mit der pneumatischen Kammer gekoppelt wird.
**Figur 13** zeigt einen auf der Vorderseite einer pneumatischen Kammer angebrachten Substanzträgers in Form eines befüll- und entleerbaren Beutels.
**Figur 14** zeigt verschiedene Ausführungsformen, welche der Erzeugung einer Spannung im Substanzträger dienen.
**Figur 15** zeigt Ausführungsformen einer Aktuatoreinheit zur Veränderung des Expansionsgrades einer expandierbaren Einheit des Implantats.
**Figur 16** zeigt eine vollständig implantierbare Ausführungsform der Vorrichtung ohne Kabelbaum und Versorgungseinheit
**Figur 17** zeigt verschiedene Ausführungsformen von Endstücken sowie eine Rahmenstrukturgeometrie, welche das Risiko von Verletzungen des Körpergewebes durch die Rahmenstruktur bzw. das Risiko eines Durchbruchs der Rahmenstruktur durch die sie zumindest teilweise umgebende Hülle minimieren.
**Figur 18** zeigt ein Gerät zur minimalinvasiven Explantation der Vorrichtung während des Explantationsprozesses.

### Detaillierte Beschreibung

Ausführungsformen der Erfindung umfassen mehrere Bauteile, die in den folgenden Abschnitten näher erläutert werden, wobei Ausführungsformen der einzelnen Bauteile miteinander kombiniert werden können.

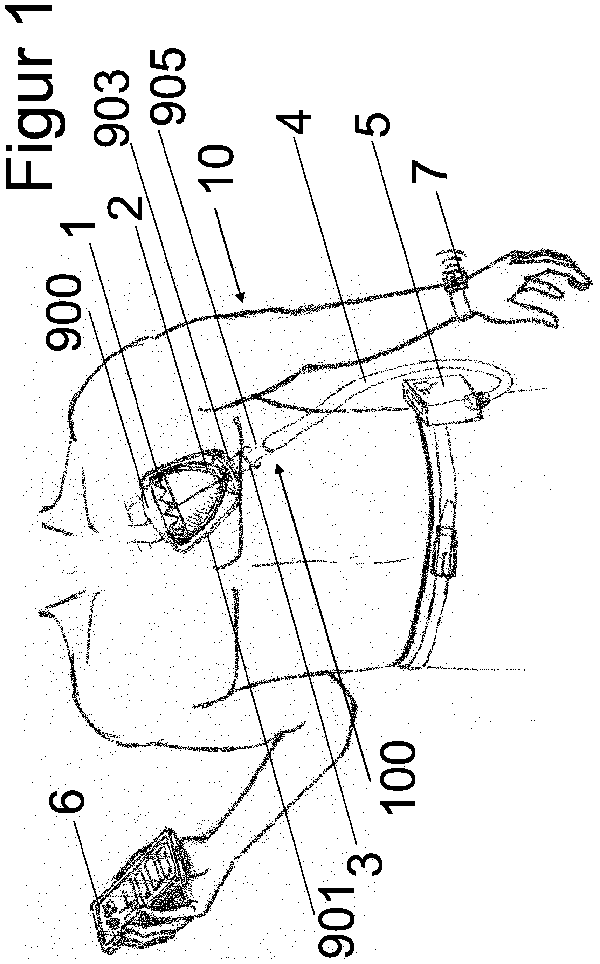

**Figur 1** zeigt einen menschlichen Torso mit einer Ausführungsform (1) der erfindungsgemäßen Vorrichtung im implantierten Zustand. Das Implantat (100) umfasst eine Rahmenstruktur (1), eine darin eingebrachte Hülle (2), zumindest eine expandierbare Einheit (auf der Hülle und/oder der Rahmenstruktur platziert) sowie zumindest ein Substanzträger, zumindest ein Sensor und zumindest eine Elektrode. Ebenfalls dem Implantat (100) zugeordnet werden eine Perikardschleuse (3), der implantierte Teil eines Kabelbaums (4) sowie pneumatische/hydraulische, Substanz- und elektrische Leitungen im Innern des Körpers. Des Weiteren umfasst die Ausführungsform (1) der erfindungsgemäßen Vorrichtung eine Versorgungseinheit (5). Zudem sind eine Steuerungseinheit (6) und eine Überwachungseinheit (7) dargestellt. Ausführungsformen der erfindungsgemäßen Vorrichtung können zumindest teilweise in einem menschlichen Körper implantiert werden. Sie kann jedoch auch in einem tierischen Körper implantiert werden. Ausführungsformen der erfindungsgemäßen Vorrichtung können in den Körper eines Säugetieres implantiert werden, z.B. dem eines Hundes, einer Katze, eines Nagetiers, eines Primaten, eines Paarhufers, eines Unpaarhufers oder eines Beuteltiers. Je nach Spezies können spezielle Anpassungen der Form und der Funktionsweise des Implantats (100) (100), des Kabelbaums (4) und der Versorgungseinheit (5) vonnöten sein, um der Anatomie und/oder der Physiologie der jeweiligen Spezies Rechnung zu tragen. Die Vorrichtung umfasst eine das Herz (900) zumindest teilweise umschließende Rahmenstruktur (1). Weiterhin ist ein ummantelter Kabelbaum (4) dargestellt, welcher aus dem Körperinnern nach außen tritt und an eine Versorgungseinheit (5) angeschlossen ist. In Figur 1 nicht dargestellt sind bspw. eine in diese Rahmenstruktur (1) einbringbare Hülle (2) oder weitere auf die Oberfläche der Hülle (2) aufgebrachte oder eingearbeitete Komponenten, welche eine therapeutische Wirkung haben können. Diese werden an anderer Stelle beschrieben.

Die Rahmenstruktur (1) kann zum Zwecke einer Implantation, die auch minimalinvasiv vorgenommen werden kann, von einem nicht expandierten Zustand in einen expandierten Zustand übergeführt werden. Im expandierten Zustand kann die Rahmenstruktur (1) ein Herz (900) zumindest teilweise umschließen. Im expandierten Zustand kann die Rahmenstruktur (1) eine formgebende, positionierende, oder stabilisierende Funktion oder eine Kombination daraus erfüllen. Im nicht expandierten Zustand kann die Rahmenstruktur (1) in ein Zuführsystem eingebracht werden. Die Rahmenstruktur (1) kann einteilig sein oder kann alternativ auch aus zwei, drei, vier, fünf, sechs oder mehr Teilen bestehen. Die Rahmenstruktur (1), welche aus zumindest einem Draht oder zumindest einer Strebe besteht, kann zumindest teilweise aus einer Formgedächtnislegierung gefertigt sein. Werkstoffe für Formgedächtnislegierungen sind bspw. NiTi und NiTiFe (Nickel-Titan und Nickel-Titan-Eisen; Nitinol), NiTiCu (Nickel-Titan-Kupfer), CuZn (Kupfer-Zink), CuZnAl (Kupfer-Zink-Aluminium), CuAINi (Kupfer-Aluminium-Nickel), FeNiAl (Eisen-Nickel-Aluminium) und FeMnSi (Eisen-Mangan-Silizium). Die Rahmenstruktur (1) kann auch aus Titan oder Titanlegierungen, Tantal oder Tantallegierungen, Edelstahl, Polymer, Polymerfaser-Werkstoff, Karbonfaserwerkstoff, Aramidfaserwerkstoff, Glasfaserwerkstoff und Kombinationen daraus bestehen. Die Rahmenstruktur (1) umschließt das Herz (900) im implantierten Zustand zumindest teilweise und ist im Inneren des Perikards verortet. Die Rahmenstruktur (1) kann auch außerhalb des Perikards angebracht werden. Derartige Ausführungsformen werden nicht separat erläutert, da die Beschreibung sowohl für innerhalb wie außerhalb des Perikards implantierte Ausführungsformen gilt (bis auf die im Falle einer außerhalb des Perikards befindlichen Ausführung entfallende Perikardschleuse (3)). Die Rahmenstruktur (1) kann über zumindest eine röntgenopake Markierung verfügen, mithilfe welcher die Positionierung und Orientierung der Rahmenstruktur (1) in Bezug auf ein Herz (900) während und nach der Implantation überprüft werden kann. Der Aufbau der Rahmenstruktur (1) wird in einem folgenden Abschnitt näher erläutert.

In die Rahmenstruktur (1) kann zumindest eine Hülle (2) eingebracht werden. Die Hülle (2) kann von der Rahmenstruktur (1) umfasst werden. Die Hülle (2) kann an die Rahmenstruktur (1) gekoppelt sein. Alternativ kann eine Rahmenstruktur (1) in eine Hülle (2) eingearbeitet sein. Die Hülle (2), deren Aufbau und die Kopplung zwischen Rahmenstruktur (1) und Hülle (2) werden in einem folgenden Abschnitt näher erläutert. In die Rahmenstruktur (1) oder in die Hülle (2) können Komponenten eingebracht werden, mit welchen eine therapeutische Wirkung erzielt werden kann. Beispielsweise kann die therapeutische Wirkung erzielt werden durch Aufbringen mechanischer Kräfte oder durch Zuführen von Substanzen auf das Epikard (die Oberfläche des Herzes (900)).

Figur 1 zeigt weiterhin eine Versorgungseinheit (5), die außerhalb des Körpers getragen werden kann. Die Versorgungseinheit (5) kann auch teilweise oder vollständig in den Körper implantiert werden. Wird die Versorgungseinheit (5) außerhalb des Körpers getragen, kann diese an einem Brustgurt, an einem Hüftgürtel oder an einem Bauchgurt befestigt werden. Alternativ kann sie in einem Halfter oder an/in einem Rucksack getragen werden. Auch andere Formen der Befestigung bzw. des Tragens sind denkbar. Figur 1 zeigt weiter einen Kabelbaum (4), der die Versorgungseinheit (5) mit der Rahmenstruktur (1), einer Hülle (2) und/oder darin eingebrachten Komponenten verbindet. Der Kabelbaum (4) ist in der vorliegenden Ausführungsform ummantelt dargestellt. Eine detaillierte Beschreibung des Kabelbaums (4) erfolgt in einem späteren Abschnitt. Wird die Versorgungseinheit (5) außerhalb des Körpers getragen, kann der Kabelbaum (4) an einer Stelle in den Körper eindringen. Befindet sich die Vorrichtung mit einer Rahmenstruktur (1), einer Hülle (2), zumindest einer expandierbaren Einheit und zumindest einer Komponente zur Erzielung einer therapeutischen Wirkung innerhalb der Perikardhöhle (902), kann der Kabelbaum (4) oder Teile davon bis in die Perikardhöhle (902) hineingeführt werden. Der Kabelbaum (4) kann an der Eintrittsstelle in den Körper (905) und/oder an der Eintrittsstelle (903) in die Perikardhöhle (902) durch eine Perikardschleuse (3) geführt werden, welche in einem späteren Abschnitt der Beschreibung näher erläutert wird.

Dient die eingebrachte zumindest eine Komponente mit therapeutischer Wirkung zur zumindest ortsgenauen Zuführung an und zur Applikation von Substanzen auf die Herzoberfläche, kann sich im Kabelbaum (4) zumindest eine Leitung befinden, die ein Nachführen und/oder Abführen einer Substanz und/oder ein Nachfüllen der Substanz in der eingebrachten Komponente ermöglicht.

Soll die eingebrachte zumindest eine Komponente mit therapeutischer Wirkung eine mechanische Kraft auf die Herzoberfläche ausüben, so erfordert dies das Zuführen von Energie von der Versorgungseinheit (5). Wird die Energie in Form von pneumatischer oder hydraulischer Energie zugeführt, so kann der Kabelbaum (4) zumindest eine Fluidleitung zum Transport von unter Druck stehendem Fluid enthalten. Für den Fall, dass elektrische Energie zugeführt werden muss, kann der Kabelbaum (4) zumindest einen elektrischen Leiter enthalten. Wird die Versorgungseinheit (5) außerhalb des Körpers getragen, kann die bereitzustellende elektrische Energie alternativ auch kabellos z.B. über elektromagnetische Induktion an die Vorrichtung in den Körper übertragen werden.

Alternativ können mit der eingebrachten Komponente auch Substanzen appliziert werden und mechanische Kraft auf die Substanz und/oder die Herzoberfläche aufgebracht werden. In diesem Fall können sich zumindest eine Leitung zum Nachführen und/oder Abführen einer Substanz und/oder ein Nachfüllen der Substanz und gleichzeitig zumindest eine Fluidleitung oder zumindest ein elektrischer Leiter befinden.

Beinhaltet die Vorrichtung weiterhin zumindest einen Sensor, zumindest eine Elektrode oder zumindest einen elektrischen Verbraucher (bspw. eine Heizwendel) in der Rahmenstruktur (1), in der Hülle (2), in der zumindest einen expandierbaren Einheit oder in der eingebrachten Komponente mit therapeutischer Wirkung, so kann sich die zumindest eine elektrische Leitung, die den zumindest einen Sensor, Elektrode oder Verbraucher bedient, ebenfalls in dem Kabelbaum (4) befinden.

Am Ende des Kabelbaums (4) kann ein Steckerteil angebracht sein, das über einen koppelbaren Anschluss an die Versorgungseinheit (5) verbunden werden kann. Alternativ befindet sich nur an der Versorgungseinheit (5) ein Kabelbaum (4) mit einem Steckerteil. In diesem Fall befindet sich der damit koppelbare Anschluss an der implantierten Vorrichtung.

Der Kabelbaum (4) kann ein einzelner durchgängiger Kabelbaum (4) sein oder ein mehrteiliger Kabelbaum (4) sein. Im Falle eines mehrteiligen Kabelbaums (4) kann sich an geeigneter Stelle im Kabelbaum (4) zumindest ein weiteres koppelbares Steckerpärchen befinden. Im Falle eines mehrteiligen Kabelbaums (4) kann zumindest ein Teil des Kabelbaums (4) entfernt werden und zumindest ein Teil des Kabelbaums (4) kann an der implantierten Vorrichtung oder der Versorgungseinheit (5) verbleiben. Der entfernbare Teil des Kabelbaums (4) kann als Verlängerungskabel dienen und kann bei Bedarf verwendet werden. Die Vorrichtung kann dann auch ohne das Verlängerungskabel verwendet werden und die verbleibenden Steckerteile können derart miteinander gekoppelt werden, dass die Vorrichtung betrieben werden kann. Bei einem mehrteiligen Kabelbaum (4) können die einzelnen Kabelbaumstücke kodiert sein, bspw. analog-elektronisch, wie z.B. über einen Widerstand, oder digital-elektronisch, wie z.B. mit einem ROM-Speicherchip im Steckerteil. Die Versorgungseinheit (5) kann die Kodierung der Kabelbäume (4) auslesen und wenn erforderlich Anpassungen im Betrieb der Vorrichtung vornehmen. Solche Anpassungen können bspw. eine Mehrförderung von Fluid sein, die aufgrund eines eingesetzten Verlängerungskabels notwendig ist.

Ausführungsformen der erfindungsgemäßen Vorrichtung können auch über getrennte Kabelbäume (4) für die Leitung zum Nach-/Abführen von Substanz, die Fluid- oder elektrische Leitung zur Energieversorgung der eingebrachten Komponente mit therapeutischer Wirkung und/oder die elektrische Leitung zum Bereitstellen von Energie für den zumindest einen Sensor, die zumindest eine Elektrode oder den zumindest einen elektrischen Verbraucher verfügen. Ist die Versorgungseinheit (5) direkt mit der zumindest einen eingebrachten Komponente mit therapeutischer Wirkung, dem zumindest einen Sensor, der zumindest einen Elektrode oder dem zumindest einen elektrischen Verbraucher verbunden, kann alternativ auf einen Kabelbaum (4) verzichtet werden.

Ein Teil des durchgängigen oder mehrteiligen, des einzelnen oder des in mehrere Kabelbäume (4) aufgeteilten Kabelbaums (4) kann im Körperinnern, ein anderer Teil außerhalb des Körpers verlaufen. Der im Körperinnern verlegte Teil des Kabelbaums (4) kann zwischen 0 cm und 70 cm lang sein, er kann zwischen 30 cm und 60 cm lang sein. Der außerhalb des Körpers verlaufende Teil des Kabelbaums (4) kann zwischen 0 cm und 90 cm lang sein, er kann zwischen 50 cm und 80 cm lang sein. Der Durchmesser des Kabelbaums (4) kann zwischen 4 mm und 25 mm liegen, er kann zwischen 6 mm und 12 mm liegen. Der mehrteilige Kabelbaum (4) kann gegenüber dem durchgängigen Kabelbaum (4) vorteilhaft sein, da er modular ist und daher bspw. eine veränderbare, u.U. größere Bewegungsfreiheit des Patienten ermöglichen kann.

Der Kabelbaum (4) kann zumindest teilweise entlang seiner Längsachse und zumindest teilweise umfänglich von einem Gewebe umgeben sein. Das Gewebe kann porös, faserig oder rau sein. Es kann das Einwachsen des Kabelbaums (4) in das umliegende Bindegewebe und dadurch die mechanische Verankerung der implantierten erfindungsgemäßen Vorrichtung im Körper verbessern. Es kann bspw. aus Filzgewebe, expandiertem PTFE, Dacron oder einem anderen geeigneten biokompatiblen synthetischen Stoffgewebe bestehen. Das Material des Gewebes kann je nach Anwendungszweck jedoch auch davon abweichen. Beispielsweise kann so auch eine zusätzliche Abschirmung des Kabelbaums (4) gegen die Umwelt ermöglicht werden, bspw. um den Ein- und Abstrahlschutz zu verbessern.

Des Weiteren zeigt Figur 1 eine Ausführungsform einer tragbaren Steuerungseinheit (6) und einer ebenfalls tragbaren Überwachungseinheit (7). Ausführungsformen der erfindungsgemäßen Vorrichtung können über eine Steuerungseinheit (6) und eine Überwachungseinheit (7) verfügen. Sie kann auch nur über eine Steuerungseinheit (6) verfügen. Sie kann auch nur über eine Überwachungseinheit (7) verfügen. Sie kann auch weder über eine Steuerungseinheit (6) noch über eine Überwachungseinheit (7) verfügen. Die Steuerungseinheit (6) und/oder die Überwachungseinheit (7) können bereits von der Versorgungseinheit (5) übernommene Funktionen in redundanter Art übernehmen. Sie können alternativ auch zusätzliche Funktionen bereitstellen.

Die Steuerungseinheit (6) kann als Hardware mit aufgespielter Software ausgeführt sein. Die Hardware kann hierbei zumindest eine Kommunikationsschnittstelle, zumindest ein Display und/oder auch die Möglichkeit zur Wiedergabe akustischer, visueller oder haptischer Signale bereitstellen. Die auf diese Hardware aufgespielte Software kann Steuerungs-, Überwachungs- und Kommunikationsfunktionen übernehmen. Die externe Steuerungseinheit (6) kann jedoch alternativ auch Drittanbieterhardware nutzen und lediglich in Form eines Softwarepaketes oder einer Applikation ("App") vorhanden sein. Ist letzteres der Fall, so können die durch die Drittanbieterhardware bereitgestellten Kommunikationsschnittstellen genutzt werden, um mit der Informations-/Steuerungseinheit (6) bzw. der Versorgungseinheit (5) zu kommunizieren. Die Kommunikation kann hierbei über eine kabellose P2P-Verbindung, eine Bluetooth-Verbindung, eine WLAN-Verbindung oder eine sonstige kabellose Form der Datenübertragung stattfinden. Alternativ kann die Kommunikation auch kabelgestützt erfolgen. Die Steuerungseinheit (6) kann auch eine zusätzliche oder aber auch eine ausschließliche Überwachungsfunktion übernehmen. Beispielsweise können die von dem zumindest einen Sensor des Implantats (100) erfassten Daten über die Versorgungseinheit (5) (oder auch direkt an die Steuerungseinheit (6)) an die Steuerungseinheit (6) gesendet, ausgewertet und in einer von Menschen lesbaren Form für den Nutzer dargestellt werden. Der Nutzer kann hierbei der Patient sein, der Nutzer kann auch ein behandelnder Arzt oder ein Techniker sein. Die Steuerungseinheit (6) kann so ausgelegt sein, dass die Funktion der Vorrichtung auch bei einem Ausfall der Steuerungseinheit (6) gewährleistet ist. Die Steuerungseinheit (6) kann auch rein informative Funktionen haben.

Die Überwachungseinheit (7) kann auch eine Steuerungseinheit (6) sein. Sie kann aber auch ausschließlich Überwachungsfunktionen wahrnehmen. Sie kann mit der Steuerungseinheit (6), aber auch mit der Versorgungseinheit (5) über eine der zuvor genannten Kommunikationsarten in Verbindung stehen. Die Überwachungseinheit (7) kann in Softwareform auf Hardware von Drittanbietern eingesetzt werden und dann deren Kommunikationsschnittstellen nutzen. Wie bei der Steuerungseinheit (6) kann jedoch die Überwachungseinheit (7) auch eine Ausführung als eigenständige Hardware mit aufgespielter Software sein. Für die Software der Überwachungseinheit (7) können dieselben Funktionalitäten wie bei der Steuerungseinheit (6) ausgeführt sein. Die Überwachungseinheit (7) kann insbesondere die Funktion der schnellen Informationsweitergabe an den Nutzer übernehmen. In der gezeigten Ausführungsform ist die Überwachungseinheit (7) am Handgelenk des Nutzers befestigt und kann daher rasch betrachtet werden. Im Falle eines technischen oder medizinischen Notfalls, welcher von der Versorgungseinheit (5) bzw. redundant von der Steuerungseinheit (6) und/oder der Überwachungseinheit (7) registriert wird, kann dies über die Überwachungseinheit (7) und/oder die Steuerungseinheit (6) an die Umwelt (den Nutzer und sein Umgebung) kommuniziert werden. Hierbei kann es sowohl lokale Hinweise (visueller, akustischer oder haptischer Natur) als auch solche Hinweise geben, welche direkt an bestimmte Stellen, bspw. den Notarzt oder eine andere dritte Stelle, weitergegeben werden können. Hierdurch kann sich die Reaktionszeit auf einen Notfall verkürzen lassen. Denkbar ist auch, dass, sollte es sich bei der Steuerungseinheit (6) um ein Mobiltelefon handeln, der Notarzt direkt über dieses Gerät mit dem Patienten, einem Ersthelfer oder einem Arzt vor Ort kommunizieren kann. Die Überwachungseinheit (7) kann auch zumindest einen Parameter des Körpers messen, bspw. die Sauerstoffsättigung des Blutes oder den Puls. Auch die Messung andere Parameter des Körpers sind denkbar.

Die gesamte Vorrichtung, bestehend aus einem Implantat (100) und einer damit verbundenen Versorgungseinheit (5), kann auch komplett in den Körper implantiert sein. Hierbei kann die Energieversorgung über elektromagnetische Induktion von außerhalb des Körpers stattfinden und so den zumindest einen Akkumulator oder die zumindest eine Batterie wieder aufladen. Vorteil dieser Ausführungsform ist die Vermeidung einer künstlichen Körperöffhung (905) zur Hinausführung des Kabelbaums (4) vom Implantat (100) zur Versorgungseinheit (5). Die Versorgungseinheit (5) kann oberhalb der Leber und des Zwerchfells in der Brusthöhle oder rechtslateral unterhalb des Zwerchfells in der Bauchhöhle implantiert sein. Auch andere Implantationsorte sind denkbar. Der Kabelbaum (4) kann im Falle einer Implantation der Versorgungseinheit (5) unterhalb des Zwerchfells das Zwerchfell durchstoßen, um zum Perikard (901) geführt zu werden.

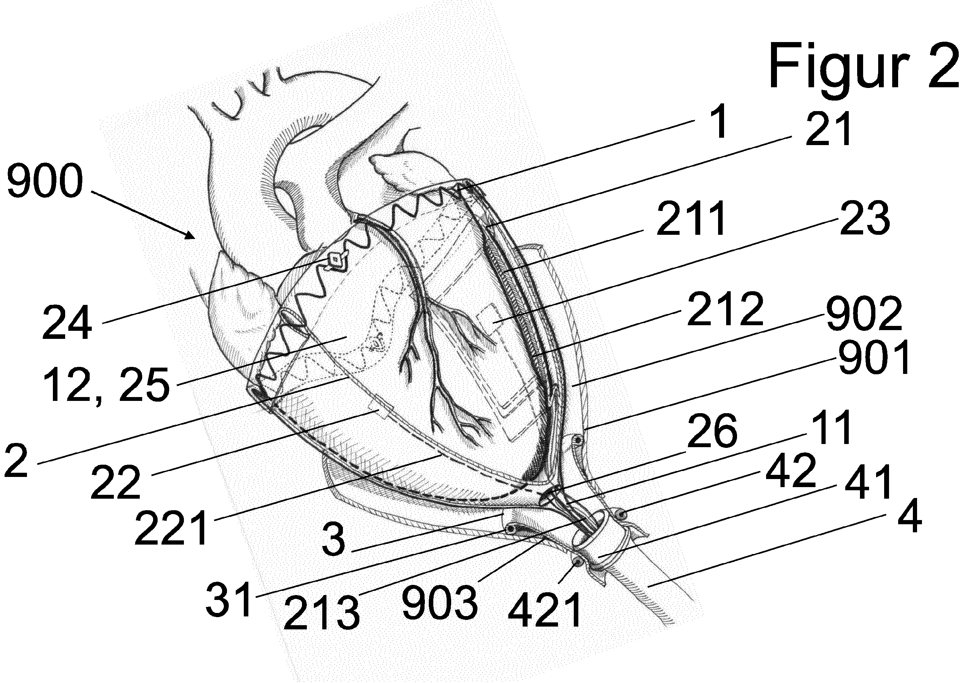

**Figur 2** zeigt eine Ausführungsform des Implantats im implantierten Zustand. Exemplarisch dargestellt sind ein menschliches Herz (900), eine Rahmenstruktur (1), eine Hülle (2), eine expandierbare Einheit (21) mit der zugehörigen Leitung (213), ein Substanzträger (211), die darauf applizierte Substanz (212), Sensoren und/oder Elektroden mit den zugehörigen Kabeln, eine Fixierhülse (41), ein Teil eines Kabelbaums (4), eine Perikardschleuse (3) und ein Fixierring (42) des Implantats.

Die Rahmenstruktur (1) in dieser Ausführungsform ist als Drahtgestell dargestellt. Die Rahmenstruktur (1) kann aus zumindest einem Draht geformt sein. Die Rahmenstruktur (1) kann auch aus zwei, drei, vier, fünf, sechs oder mehr Drähten geformt sein. Die Rahmenstruktur (1) kann auch eine aus Streben bestehende Struktur sein. In diesem Fall besteht die Rahmenstruktur (1) aus zumindest einer Strebe. Die Rahmenstruktur (1) kann auch aus zwei, drei, vier, fünf, sechs oder mehr Streben geformt sein. Eine Aufgabe der Rahmenstruktur (1) kann in der Gewährleistung mechanischer Stabilität im expandierten Zustand bestehen. Eine weitere Aufgabe der Rahmenstruktur (1) kann darin bestehen, die Expansion ausgehend vom nicht expandierten Zustand in einen expandierten Zustand zu ermöglichen. Eine weitere Aufgabe der Rahmenstruktur (1) kann die Aufspannung einer Hülle (2) sein, welche in oder an der Rahmenstruktur (1) befestigt werden kann, um sie in eine bestimmte Form zu bringen. Die Zwischenräume der Rahmenstruktur (1), welche durch den zumindest einen Draht oder die zumindest eine Strebe definiert werden, können Öffnungen in der Rahmenstruktur (1) sein. Sie können der Erhöhung der Flexibilität, der Gewichtsersparnis sowie der Vereinfachung des Flüssigkeitsaustauschs von außerhalb der Rahmenstruktur (1) in dessen innenliegende Kavität und umgekehrt dienen. Nähere Erläuterungen des Aufbaus und der Funktion der Rahmenstruktur (1) betreffenden Details erfolgen in einem nachfolgenden Abschnitt.

In die Rahmenstruktur (1) kann eine Hülle (2) eingebracht werden. Die Hülle (2) kann an der Rahmenstruktur (1) befestigt werden. Die Innenseite bzw. die Innenfläche ist definiert als die herzzugewandte Seite bzw. Fläche der Hülle (2) im implantierten Zustand der Vorrichtung. Die Außenseite/Außenfläche ist die entsprechend andere Seite/Fläche der Hülle (2). Die Hülle (2) kann zumindest teilweise die Form der Oberfläche eines Herzes (900) oder eines Abbilds eines Herzes (900) beschreiben. Die Hülle (2) kann patientenspezifisch angefertigt sein. Im implantierten Zustand kann die Hülle (2) einen Teil eines Herzes (900) zumindest teilweise umschließen. Die Befestigung der Hülle (2) an der Rahmenstruktur (1) kann derart stattfinden, dass die Hülle (2) am oberen Rand der Rahmenstruktur (1) über den Rand der Rahmenstruktur (1) gestülpt wird. In einem weiteren Schritt kann der übergestülpte Teil der Hülle (2) mit dem nicht gestülpten Teil durch vorhandene Öffnungen in der Rahmenstruktur (1) hindurch verbunden werden. Die beiden Teile können verschweißt, vernäht oder verklebt werden. Auch andere Kopplungsarten, etwa Formschlussverbindungen, sind denkbar. Die Hülle (2) kann an der Rahmenstruktur (1) auch durch Einhängen an dafür vorgesehenen Stellen befestigt werden. Sowohl auf der Innenseite der Hülle (2) als auch auf der Außenseite der Hülle (2) können zumindest ein Sensor und/oder zumindest eine Elektrode (23) angebracht werden. Die Hülle (2) kann aus Kunststoff, Polymer, Kautschuk, Gummi, Latex, Silikon oder Polyurethan bestehen. Die Hülle (2) kann eine Dicke von 0,1 mm bis 1 mm, vorzugsweise 0,2 mm bis 0,5 mm, aufweisen.

Das Herstellverfahren der Hülle (2) kann in mehrere Unterschritte gegliedert werden. Ausgangspunkt für eine patientenspezifisch geformte Hüllengeometrie kann ein medizinischer Bilddatensatz sein. Aus diesem kann die patientenspezifische Herzgeometrie extrahiert und in ein dreidimensionales Abbild überführt werden. Dieses Abbild kann Modell für den Vorgang der Formgebung der Hülle (2) stehen. Um die Hülle (2) zu formen, kann es eines Formkörpers bedürfen, welcher aus dem dreidimensionalen Abbild des Herzes (900) abgeleitet werden kann. Der Formkörper kann aus dem Vollen gefräst werden. Alternativ kann er auch mittels eines 3D-Druckverfahrens aus einem dreidimensionalen Computermodell hergestellt werden. Auch alternative Fertigungsverfahren zur Herstellung des Formkörpers, etwa Gießen, sind denkbar. Die Hülle (2) kann ein Herz (900) umhüllen und dabei einen bestimmten Abstand zur Herzoberfläche aufweisen. Der Abstand kann zwischen 0 mm und 3 mm betragen, er kann zwischen 0.4 mm und 2 mm betragen. Um diesen Abstand zu erzeugen, kann das dreidimensionale Abbild des Herzes (900) zentrisch gestreckt werden. Der Streckfaktor kann hierbei so gewählt werden, dass der Abstand zwischen Herzoberfläche und der Hülle (2) dem oben angegeben Werteintervall entspricht. Der Formkörper kann aus einem Metall bestehen. Der Formkörper kann auch aus einem Kunststoff bestehen. Metallische Werkstoffe können hierbei Aluminium und Aluminiumlegierungen, Stahl und Stahllegierungen oder Kupfer und Kupferlegierungen sein. Auch andere Metalle können infrage kommen. Bei einer Ausführungsform des Formkörpers aus Kunststoff können bspw. Polyurethan (PU), Polyamid (PA) oder Polyethylen (PE) infrage kommen. Auch andere Kunststoffe mit geeigneten mechanischen Eigenschaften können infrage kommen. Der Formkörper kann bereits Kennzeichnungen für die Lage zumindest einer expandierbaren Einheit (21) aufweisen, was den anschließenden Anbringungsvorgang der zumindest einen expandierbaren Einheit, bspw. bei manueller Fertigung und Anbringung der zumindest einen expandierbaren Einheit, vereinfacht. Ebenfalls bereits an dem Formkörper vorgesehen können Orte sein, an welchen Zuleitungen, etwa für eine pneumatische Aktuation der expandierbaren Einheiten (21) oder für die Substanzzuführung in den in einem folgenden Abschnitt näher beschriebenen Substanzträgern (211), ansetzen können. Die Hülle (2) kann über den Formkörper gespannt werden und wird unter Erhöhung der Temperatur plastisch verformbar. Die Hülle (2) kann sich an den Formkörper anschmiegen und nach dem Abkühlen die Form des Formkörpers annehmen. Auch die Vorgaben für die Position der expandierbaren Einheiten (21) und die Zuleitungsansätze können derart in die Hülle (2) eingeprägt werden. Schließlich kann ein Überstand des Materials der Hülle (2) über die eigentliche Geometrie der Hülle (2) hinaus dazu genutzt werden, die Hülle (2) in einem folgenden Fertigungsschritt mit der Rahmenstruktur (1), bspw. durch Umstülpen, zu koppeln.

Die Hülle (2) kann an die Rahmenstruktur (1) gekoppelt werden. Die Kopplung kann am oberen Rand der Rahmenstruktur (1) (dort, wo die zuvor beschriebene alternierende Draht- oder Strebenführung vorliegt) insbesondere durch Umschlagen der Hülle (2) und durch eine Kopplung der Hülle (2) an sich selbst durch die Öffnungen der Rahmenstruktur (1) hindurch realisiert werden. Die Kopplung der Hülle (2) mit sich selbst kann bspw. durch eine Klebeverbindung, eine Schweißverbindung, oder durch Vernähen ausgeführt sein. Auch andere Verbindungsformen, welche den gleichen Effekt erzielen, sind möglich. Bspw. kann die Hülle (2) an der Rahmenstruktur (1) an dafür vorgesehenen Stellen (bspw. an den Extrema der alternierenden Drahtführung) eingehängt werden. In einem solchen Fall kann die Hülle (2) zumindest eine Tasche aufweisen, die über zumindest eine Schlaufe oder zumindest einen Bügel gestülpt werden kann.

Die Rahmenstruktur (1) kann am unteren Ende eine Öffnung (26) haben. Ein Teil der Hülle (2) kann durch diese Öffnung (26) durchragen, bspw. können zwischen 3 mm und 2 cm der Hülle (2) durchragen. Die Hülle (2) kann ebenfalls unten geöffnet sein, womit der Flüssigkeitsaustausch zwischen dem Raum innerhalb der Hülle (2) und dem Raum zwischen Rahmenstruktur (1) und Perikard (901) und umgekehrt verbessert wird. So kann bspw. Perikardflüssigkeit durch die Öffnung (26) in der Hülle (2) ein- und ausfließen. Der obere Rand der Rahmenstruktur (1) verläuft bevorzugt parallel zur Herzklappenebene und kann zumindest einen Bereich mit zumindest einer Aussparung (12) aufweisen. Die Aussparung (12) in der Rahmenstruktur (1) kann aus anatomischen Gegebenheiten erforderlich sein, bspw. um die Vena cava inferior nicht räumlich zu beeinträchtigen, welche von dorsal in Richtung des rechten Vorhofs des Herzes (900) verläuft. Eine Räumliche Beeinträchtigung der Vena cava inferior würde zu einer unteren Einflussstauung führen (Behinderung der Befüllung des rechten Vorhofs). Eine Aussparung (12) kann auch aufgrund anderer anatomischer oder kardialer Strukturen erforderlich sein. Wird die Rahmenstruktur (1) als Gitterwerk oder Drahtgeflecht ausgeführt, so kann die Aussparung (12) durch nachträgliches Abtrennen von Streben oder Drähten hergestellt werden. Alternativ kann auch ein Fertigungsverfahren gewählt werden, bei dem die Aussparung (12) nicht im Nachhinein hergestellt werden muss. So kann zum Beispiel bei einer aus einem Drahtgeflecht hergestellten Rahmenstruktur (1) die Drahtwicklung so angepasst werden, dass die Drähte an der Stelle der Aussparung (12) kürzer gewählt werden und die Kreuzungspunkte näher zusammenrücken. Bei einer Rahmenstruktur (1), die aus einem Gitterwerk besteht, das aus einem geschlitzten Rohr ("slotted tube") gefertigt wurde, kann die Aussparung (12) durch geeigneten Zuschnitt des Rohres und entsprechend modifizierte Schlitzung angefertigt werden. Die Schlitzung kann so angepasst werden, dass trotz der Aussparung (12) die Anzahl der Zellen in der Rahmenstruktur (1) gleich bleibt. Das kann aus Stabilitätsgründen erforderlich sein. Wird mehr Flexibilität in der Rahmenstruktur (1) gewünscht, so kann die Schlitzung des Rohres derart modifiziert werden, dass an der Stelle der Aussparung (12) eine, zwei oder drei Zellreihen wegfallen. Die Hülle (2) kann an die Aussparung (12) angepasst werden und kann ihrerseits eine möglichst deckungsgleiche Aussparung (25) aufweisen. Entlang des oberen Rahmenstrukturrandes kann die Aussparung (12) eine Länge von 2 cm, 3 cm, 4 cm, 5 cm, 6 cm, 7 cm oder mehr haben. Die Tiefe der Aussparung (12), gemessen vom gedachten nicht-unterbrochenen oberen Rahmenstrukturrand zum Punkt der Aussparung (12) welcher der Herzspitze am nächsten liegt, kann zwischen 1 mm und 40 mm betragen, insbesondere zwischen 3 mm und 15 mm. In Umfangsrichtung ist die Aussparung (12) dort positioniert, wo die Vena cava inferior in den rechten Vorhof mündet. Die Aussparung (12) kann bogenförmig, halbkreisförmig, rechteckig oder polygonal sein. Die Aussparung (12) kann so lang sein, dass sie dem Umfang des oberen Randes der Rahmenstruktur (1) entspricht. Die Aussparung (12) kann dann an dem Punkt des oberen Randes der Rahmenstruktur (1) beginnen und enden, welcher der auszusparenden kardialen Struktur, bspw. der Vena cava inferior gegenüberliegt. In diesem Fall kann resultieren, dass die Aussparung (12) eine Ebene definiert, welche zur Herzklappebene verkippt ist und welche an der auszusparenden Stelle 1 mm bis 40 mm, insbesondere 3 mm bis 15 mm tiefer zu liegen kommt, als wenn der obere Rand der Rahmenstruktur (1) parallel zur Herzklappenebene verlaufen würde. Die Hülle (2) weist eine Aussparung (25) auf, die in ihrer Form, Lage und Dimension im Wesentlichen mit der Aussparung (12) in der Rahmenstruktur (1) übereinstimmt, welche den anatomischen Bereich um die Vena cava inferior freistellt. Eine Räumliche Beeinträchtigung der Vena cava inferior durch die Hülle (2) und eine daraus folgende Behinderung der Befüllung des rechten Vorhofs wird dadurch verhindert. Die Aussparungen (12, 25) in Hülle (2) und Rahmenstruktur (1) können miteinander in Deckung gebracht werden.
Die Rahmenstruktur (1) kann mehrere Markierungen aufweisen. Wie bereits beschrieben, können diese Markierungen verschiedene Formen oder Positionen einnehmen. In der vorliegenden Ausführungsform können die Markierungen am oberen Rand und an der unteren Ende Richtung Spitze der Rahmenstruktur (1) angebracht sein.

Am unteren Ende der Rahmenstruktur (1) können eine, zwei, drei, vier, fünf, sechs oder mehr Verlängerungsstreben (11) an der Rahmenstruktur (1) angebracht werden. Die Verlängerungsstreben (11) stellen eine Verlängerung der Rahmenstruktur (1) in Richtung der Herzlängsachse dar. Die Verlängerungsstreben (11) können in Richtung einer gedachten Herzspitze verlaufen, sich dort berühren oder auch nicht berühren und von dort aus in Richtung der Herzlängsachse vom Herz (900) wegragen. Die Verlängerungsstreben (11) können in einer gemeinsamen Fixierhülse (41) gesammelt werden. Die Verlängerungsstreben (11) und/oder die Fixierhülse (41) können mit dem Kabelbaum (4) der Vorrichtung verbunden werden, bspw. durch Kleben, Gießen oder eine formschlüssige oder kraftschlüssige Verankerung. Die Verlängerungsstreben (11) können dazu dienen, die Rahmenstruktur (1) in axialer Richtung zu fixieren. Die Verlängerungsstreben (11) schränken die Rotation der Rahmenstruktur (1) um die Herzlängsachse nur bedingt ein. Ist die Rahmenstruktur (1) aus einem Drahtgeflecht gefertigt, so können die Verlängerungsstreben (11) die verlängerten Enden von Drähten darstellen. Die Verlängerungsstreben (11) und das Drahtgeflecht können somit ein Teil sein. Besteht die Rahmenstruktur (1) aus einem Gitterwerk, das aus einem geschlitzten Rohr ("slotted tube") gefertigt wurde, so kann das Gitterwerk mit den Verlängerungsstreben (11) durch geeignete Schnittführung aus nur einem Rohr gefertigt werden. Das Gitterwerk und die Verlängerungsstreben (11) können somit ein Teil sein. Die Verlängerungsstreben (11) und die Rahmenstruktur (1) können auch mehrteilig sein, womit die Verlängerungsstreben (11) nach der Fertigung der Rahmenstruktur (1) an dieser befestigt werden, bspw. durch einen Einhängemechanismus oder durch Stoffschluss (Anschweißen, Ankleben). Bei gleichem oder ähnlichem Effekt sind auch alternative Kopplungsarten denkbar. Die Länge der Verlängerungsstreben (11) kann zwischen 1 cm und 10 cm, insbesondere zwischen 4 cm und 7 cm betragen.
Die Rahmenstruktur (1) kann nur die oben beschriebene Aussparung (12) und keine Verlängerungsstreben (11) umfassen. Alternativ kann die Rahmenstruktur (1) sowohl die oben beschriebene Aussparung (12) und die oben beschriebenen Verlängerungsstreben (11) umfassen. Die Rahmenstruktur (1) kann auch nur die oben beschriebenen Verlängerungsstreben (11) und keine Aussparung (12) umfassen.

Am unteren Ende der Rahmenstruktur (1) kann sich zumindest eine Verlängerungsstrebe (11) befinden, welche die Rahmenstruktur (1) in axialer Richtung zum Kabelbaum (4) fixiert und die Vorrichtung stabilisiert. Die zumindest eine Verlängerungsstrebe (11) kann mit dem Kabelbaum (4) der Vorrichtung gekoppelt sein. Mögliche Kopplungsarten sind Verkleben, Verklemmen, Eingießen, Steckverbindungen, Einhängen oder Verschweißen. Auch alternative Kopplungsarten sind denkbar. Ein Teil der zumindest einen Verlängerungsstrebe (11) auch nach der Implantation in einer Fixierhülse (41) verbleiben.

Des Weiteren kann das Implantat (100) eine Fixierhülse (41) umfassen. Die Fixierhülse (41) ist in der vorliegenden Aus führungs form als Hohlzylinder dargestellt, durch welchen pneumatische/hydraulische, elektrische und Substanzleitungen vom distalen Ende der Fixierhülse (41) her hindurchgeführt und im Kabelbaum (4) am proximalen Ende der Fixierhülse (41) zusammengefasst werden können. Der Kabelbaum (4) kann mit der Fixierhülse (41) gekoppelt sein, etwa mittels einer Klebeverbindung (Stoffschluss) oder einer kraftschlüssigen Verbindung (bspw. durch Aufschrumpfen des Kabelbaummantels auf die Fixierhülse (41)). Auch andere Kopplungsarten sind denkbar.

Die Fixierhülse (41) und der Kabelbaum (4) können alternativ auch über ein Zwischenstück gekoppelt sein. Dieses Zwischenstück kann bspw. aus zumindest einem Segment bestehen, welche eine zumindest teilweise rotatorische und/oder zumindest eine teilweise translatorische Relativbewegung zwischen dem Kabelbaum (4) und der Fixierhülse (41) zulassen kann. Der Vorteil einer solchen mittelbaren Kopplung ist die nahezu unabhängige Einstellbarkeit der Orientierung der Längsachse der Rahmenstruktur (1) und der Fixierhülse (41) sowie der Orientierung des Kabelbaums (4). Das Zwischenstück kann bspw. als Kugelgelenk ausgeführt sein. Das Zwischenstück kann alternativ aus einer zumindest ein Segment umfassenden Schlauchkonstruktion bestehen. Als Material des Zwischenstücks können Polymere, Kunststoffe oder Metalle und Metalllegierungen verwendet werden. Auch andere Materialien, die den mechanischen Anforderungen (insbesondere hinsichtlich der Dauerfestigkeit) genügen, können herangezogen werden.

Weiterhin kann ein Fixierring (42) im Zusammenspiel mit der Fixierhülse (41) zur Versiegelung des Perikards (901) genutzt werden. Zusammen mit der Fixierhülse (41), der Schleuse und einem Fixierring (42) kann die zumindest eine axiale Verlängerungsstrebe (11) die Vorrichtung zum Verschluss des Perikards (901) bilden. Der Fixierring (42) kann, bspw. zur Versteifung, einen aus einem steiferen Material als der übrige Fixierring (42) bestehenden Kern (421) aufweisen. Mögliche Materialien können, Stahl, Nitinol oder ein steifes Polymer sein. Auch andere Materialien sind denkbar. Auch Im Falle der Verwendung alternativer Vorrichtungen zur Versiegelung des Perikards (901), etwa durch Nutzung einer mehrteiligen Perikardschleuse (3), kann zumindest ein Teil der zumindest einen Verlängerungsstrebe (11) außerhalb des Perikards (901) und zumindest ein Teil der zumindest einen Verlängerungstrebe innerhalb der Perikardhöhle (902) liegen. Würde durch die Expansion der zumindest einen expandierbaren Einheiten (21) die Vorrichtung eine Kraft in Richtung der Herzlängsachse vom Herz (900) weg erfahren, so kann diese Kraft zum Teil über die Rahmenstruktur (1), die Verlängerungsstreben und den Kabelbaum (4) abgetragen werden, ohne dass sich eine Dislokation der Vorrichtung ergibt. Die Verlängerungsstreben (11) können an deren Enden Löcher, Bügel, Schlaufen oder abgewinkelte Enden haben, die eine minimalinvasive Explantation der Rahmenstruktur (1) und der Hülle (2) erleichtern. Die abgewinkelten Enden, die Löcher, Bügel oder Schlaufen behindern nicht die Fixierung der Verlängerungsstreben (11) in der Fixierhülse (41). Sie können zur Fixierung oder zur Zentrierung der Verlängerungsstreben (11) in der Fixierhülse (41) genutzt werden. Bei der Implantation oder während des Betriebs der Vorrichtung können sich Enden der Verlängerungsstreben (11) innerhalb des Kabelbaums (4) bzw. innerhalb der Fixierhülse (41) befinden. Implantation und Explantation des Implantats werden in nachfolgenden Abschnitten näher beschrieben.

Auf der Hülle (2) kann zumindest eine expandierbare Einheit (21) beispielsweise in Form einer balgförmigen Kammer angebracht sein. Die expandierbare Einheit (21) kann eine aktiv expandierbare Einheit (21) oder eine passiv expandierbare Einheit (21) sein. Auf der Hülle (2) können weiterhin ein Sensor und/oder eine Elektrode (23) angebracht sein. Die Hülle (2) kann die Unterseite der expandierbaren Einheiten (21) sein. Die expandierbaren Einheiten (21) können auf die Form des Herzes (900), der Rahmenstruktur (1) und/oder der Hülle (2) angepasst sein. Auf die Hülle (2) können eine, zwei, drei, vier, fünf, sechs, sieben oder mehr expandierbare Einheit (21)en aufgebracht werden. Beim Expandieren können die expandierbaren Einheiten (21) einen Druck auf den Herzmuskel ausüben. Dieser Druck wird vorzugsweise in Bereichen auf den Herzmuskel ausgeübt, unter denen sich eine Herzkammer befindet, vorzugsweise unterhalb der Herzklappenebene. In einer anderen Ausführungsform kann der Druck, bei gegebenem therapeutischem Nutzen, auch oberhalb der Herzklappenebene aufgebracht werden. Werden die expandierbaren Einheiten (21) im unteren Bereich der Hülle (2) und/oder Rahmenstruktur (1) nahe an der Herzspitze positioniert, so wird bei der Expansion der expandierbaren Einheiten (21) der Herzmuskel in Richtung der Herzlängsachse nach oben zur Herzklappenebene gedrückt. Werden die expandierbaren Einheiten (21) im oberen Bereich der Hülle (2) und der Rahmenstruktur (1) angeordnet, so wird bei Expansion der expandierbaren Einheiten (21) der Herzmuskel senkrecht zur Herzlängsachse gedrückt. Auf den Herzmuskel wird dann parallel zur Herzklappenebene (seitlich auf die Ventrikel) Druck ausgeübt. Die expandierbaren Einheiten (21) können auch an jeder Position zwischen dem unteren Rand und dem oberen Rand von Hülle (2) und Rahmenstruktur (1) positioniert werden, womit der Druck der expandierbaren Einheiten (21) bei deren Expansion anteilig in Richtung der Herzlängsachse und anteilig parallel zur Herzklappenebene wirkt. Bei Expansion der expandierbaren Einheiten (21) wirkt der Druck daher möglichst parallel zur Herzklappenebene. Die Gegenkraft auf die Vorrichtung in Richtung der Herzlängsachse wird dadurch gering gehalten. Eine Verschiebung der Vorrichtung in Richtung der Herzlängsachse vom Herz weg (Dislokation), verursacht durch Expansion der expandierbaren Einheiten (21), wird dadurch minimiert. Die selbst-expandierende Rahmenstruktur (1) und die Hülle (2) weisen in dieser Darstellung Aussparungen (12, 25) auf, um die Vena cava inferior im implantierten Zustand der Vorrichtung nicht räumlich zu beeinträchtigen. Die expandierbaren Einheiten (21) können in ihrer Größe, Form und Position an die Aussparungen (12, 25) in der Rahmenstruktur (1) und der Hülle (2) angepasst werden, so dass sie weder im expandierten, noch im nicht-expandierten Zustand die Aussparungen (12, 25) überdecken.

Die zumindest eine expandierbare Einheit (21) kann verschiedene Aufgaben innehaben. Sie kann einen zeitlich veränderlichen, vorzugsweise periodischen oder sich nach der aktuellen Phase des Herzzyklus richtenden Druck auf die Herzoberfläche ausüben. Ist das Ziel der Druckausübung die Erhöhung des Blutauswurfs, so kann die expandierbare Einheit (21) eine Augmentationseinheit sein. Unter dem Begriff der Augmentation kann also die unterstützende Erhöhung oder zumindest die Veränderung eines Herzparameters wie z.B. der Auswurfmenge verstanden werden. Alternativ kann die zumindest eine expandierbare Einheit (21) auch einen zeitlich veränderlichen Druck auf die Herzoberfläche ausüben, ohne dass dabei das primäre Ziel die Erhöhung der Menge des ausgeworfenen Blutes ist. Vielmehr kann es sich bei dieser Art um eine der Positionierung bzw. der Stabilisierung des Implantats gegenüber dem Herz (900) dienende expandierbare Einheit (21) handeln. In beiden genannten Fällen ist eine Energiezufuhr notwendig, um den Expansionsgrad der zumindest einen expandierbaren Einheit (21) einstellen zu können. Es kann sich um aktive expandierbare Einheit (21)en handeln. Alternativ kann die zumindest eine expandierbare Einheit (21) auch ohne Energiezufuhr auskommen. Hierbei kann die zumindest eine expandierbare Einheit (21) einen zuvor einstellbaren Expansionsgrad aufweisen und diesen unabhängig von der Zeit beibehalten. Eine solche expandierbare Einheit (21) kann als passive expandierbare Einheit (21) angesehen werden. In einem folgenden Abschnitt wird diese Art der expandierbaren Einheit (21) näher beschrieben.

Die expandierbare Einheit (21) kann eine mechanische Einheit sein, welche eine expandierte und eine nicht expandierte Konfiguration einnehmen kann. Die zumindest eine expandierbare Einheit (21) kann auch Konfigurationen zwischen der expandierten und der nicht expandierten Konfiguration einnehmen. Die zumindest eine expandierbare Einheit (21) kann der Aufbringung eines Druckes auf die Herzoberfläche dienen. Die zumindest eine expandierbare Einheit (21) kann der Positionierung einer Substanz (212) oder eines Substanzträgers (211) dienen. Die zumindest eine expandierbare Einheit (21) kann auch der Sicherstellung des Kontakts zwischen der Herzoberfläche und einem Substanzträger (211) oder einer Substanz (212), welche sich auf der Oberfläche der expandierbaren Einheit (21) befindet, dienen. Die expandierbare Einheit (21) kann auch dazu dienen, in der Substanz (212) und/oder im Substanzträger (211) einen definierten mechanischen Spannungszustand zu erzeugen.

Die zumindest eine expandierbare Einheit (21) kann aus dem gleichen Material wie die Hülle (2) bestehen. Das Material der zumindest einen expandierbaren Einheit (21) kann sich auch vom Material der Hülle (2) unterscheiden. Das Material der zumindest einen expandierbaren Einheit (21) kann ein Polymer sein. Das Material der zumindest einen expandierbaren Einheit (21) kann Polyurethan, Silikon oder Polytetrafluorethylen (PTFE) sein. Auch andere Materialien, die prinzipiell in der Lage sind, den Anforderungen an ein expandierbare Einheit (21) gerecht zu werden, sind denkbar. Die zumindest eine expandierbare Einheit (21) kann als inflatierbare, balgförmige Kammer ausgeführt sein. Die in der dargestellten Ausführungsform vorhandene Kammer hat die Form eines Faltenbalgs. Eine balgförmige Kammer hat zumindest einen Abschnitt in Form eines Balges. Die expandierbare Einheit (21) kann ein Faltenbalg sein, der aus ein, zwei, drei, vier, fünf, sechs, sieben oder mehr Falten besteht. Die zumindest eine expandierbare Einheit (21) kann auch von der Balgform abweichen.
Eine solche expandierbare Einheit (21) kann durch spann- und entspannbare Federelementen, falt- und entfaltbaren Hebelelemente und/oder eine elastische, schwamm-/schaumartige Innenstruktur betrieben werden. Die expandierbare Einheit (21) kann auch elektrisch aktuiert werden, bspw. durch zumindest einen Elektromagneten. Der zumindest eine Elektromagnet kann auf einer Seite der expandierbaren Einheit (21) befestigt sein oder in eine Wand der expandierbaren Einheit (21) eingearbeitet sein. Auf der gegenüber positionierten Fläche einer expandierbaren Einheit (21) kann ein ferromagnetisches, paramagnetisches Material, bspw. in Form von Drähten, einer dünnen Platte oder Folie, oder ein weiterer Elektromagnet platziert sein, so dass eine Bestromung des zumindest einen Elektromagneten eine Änderung des Expansionszustandes der expandierbaren Einheit (21) hervorruft. Alternativ können auch Federelemente, Hebelelemente, Elektromagnete und/oder ferromagnetische Werkstoffe in einer expandierbaren Einheit (21) miteinander kombiniert werden. Beispielsweise kann die Expansion der expandierbaren Einheit (21) durch ein oder mehrere Federelemente erfolgen und die Bestromung des zumindest einen Elektromagneten zur gegenseitigen Anziehung der gegenüberliegenden Seiten und damit zur Rückführung in einen nicht-expandierten Zustand führen. Die zumindest eine expandierbare Einheit (21) kann über eine Zuleitung zur Zuführung von Energie, welche zur Änderung des Expansionszustandes erforderlich ist, verfügen. Wird die expandierbare Einheit (21) elektrisch, bspw. elektromagnetisch, betrieben, so kann die Leitung (213) zur Zuführung von Energie ein elektrisches Kabel sein.

Alternativ ist die zumindest eine expandierbare Einheit (21) eine Kammer, welche mit einem Fluid gefüllt werden kann. Als Fluide, welche zum Befüllen der Kammer geeignet sind, können Flüssigkeiten, Gase, Festkörper (bspw. Nanopartikelgemische) oder Gemische von Flüssigkeiten und/oder Gasen und/oder Festkörpern in Frage. In diesem Fall verfügt die expandierbare Einheit (21) über eine Zuleitung, durch welche das zur Expansion der zumindest einen expandierbaren Einheit (21) genutzte Medium eingeleitet werden kann. Die zumindest eine expandierbare Einheit (21) kann über eine Ableitung verfügen, durch welche das zur Expansion der zumindest einen expandierbaren Einheit (21) genutzte Medium abgeleitet werden kann. Es kann auch eine Leitung (213) zur Zu- und Ableitung des zur Expansion der zumindest einen expandierbaren Einheit (21) genutzten Mediums vorgesehen sein.

Die zumindest eine expandierbare Einheit (21) kann auf der Innenseite der Hülle (2) befestigt werden. Die expandierbare Einheit (21) kann auch auf der Außenseite der Hülle (2) befestigt werden. Die zumindest eine expandierbare Einheit (21) kann in einem beliebigen Bereich der Außen- oder der Innenfläche der Hülle (2) angebracht werden.
Der Bereich der Herzoberfläche, welcher den Einsatz der zumindest einen expandierbaren Einheit (21) erfordern kann, kann individuell je nach Patient an unterschiedlicher Stelle liegen. Die freie Platzierbarkeit der zumindest einen expandierbaren Einheit (21) dient der Anpassung des Implantats an die individuelle, zu behandelnde Herzgeometrie.
Die zumindest eine expandierbare Einheit (21) kann die Form einer Kammer haben. Die Kammer kann balgförmig sein. Eine balgförmige Kammer hat zumindest einen Abschnitt in Form eines Balges. Als eine Falte kann eine nach außen gerichtete Knickkante definiert werden. Als eine Falte kann eine nach innen gerichtete Knickkante definiert werden. Eine, mehrere oder alle Knickkanten können verstärkt sein. Eine Verstärkung einer Knickkante ist vorteilhaft, da die Knickkante durch das Expandieren und Entspannen der Kammer erhöhten Belastungen ausgesetzt sein kann. Eine Verstärkung einer oder mehrerer Knickkanten kann eine Materialermüdung entlang der zumindest einen Knickkante reduzieren oder verhindern. Eine Verstärkung einer Knickkante kann durch eine höhere Wandstärke des Materials an der Knickkante erreicht werden. Eine Verstärkung einer Knickkante kann auch durch Aufbringen von zusätzlichem Material erfolgen, wobei das aufgebrachte Material das gleiche Material wie das darunterliegende Material sein kann, oder wobei das aufgebrachte Material ein anderes Material als das darunterliegende Material sein kann. Eine Kammer kann eine Oberseite, eine Unterseite und eine Seitenfläche aufweisen, wobei die Seitenfläche bevorzugt balgförmig ausgebildet ist. Die Ober- und/oder die Unterseite kann oval, kreisförmig, elliptisch oder polygonal sein. Die Oberseite kann eine andere Form haben als die Unterseite.

Eine balgförmige Kammer kann in eine wie oben beschriebene Rahmenstruktur (1) eingebracht werden. Die Kammer kann direkt in der Rahmenstruktur (1) befestigt oder fixiert werden. Die Kammer kann an strukturellen Elementen der Rahmenstruktur (1), wie zum Beispiel einem Draht eines Drahtgeflechts, einer Strebe eines Gitterwerks, oder einer Struktur auf einem Rahmenstrukturmantel befestigt werden. Die Kammer kann an Kreuzungspunkten eines Geflechts oder Gitterwerks befestigt werden.

Die balgförmige Kammer kann auch auf der Hülle (2) befestigt sein. Mehrere balgförmige Kammern können auf der Hülle (2) befestigt sein. Die Hülle (2) kann zumindest zum Teil die Form eines Herzes (900) haben. Die Hülle (2) kann eine ähnliche Form wie die Rahmenstruktur (1) haben. Die Hülle (2) kann neben einer oder mehreren Augmentationseinheiten, wie z.B. eine oder mehrere balgförmige Kammern, auch eine oder mehrere Positionierungseinheiten aufweisen. Die Unterseite der Kammer kann aus dem gleichen Material gefertigt sein wie die Hülle (2). Die Hülle (2) kann Teil der Kammer sein. Die Hülle (2) kann die Unterseite der Kammer formen. In solchen Fällen werden auf die Hülle (2) nur die Seitenflächen aufgebracht, die balgförmig sein können. Zusätzlich kann eine Oberseite angebracht werden. Die Oberseite kann auch eine Hülle (2) sein, kann aber auch eine als eigenständige Komponente ausgeführte Oberseite sein. Die zumindest eine Leitung (213), die die zumindest eine expandierbare Einheit (21) versorgt, kann ähnlich wie die Kammer zumindest zum Teil auch unter Nutzung der Hülle (2) als Rückseite und eine durch bspw. eine Klebe- oder Schweißverbindung aufgebrachte Vorderseite definiert sein. Die zumindest eine Leitung (213) kann alternativ auch ein gänzlich separates, schlauchartiges Bauteil sein.

Die zumindest eine expandierbare Einheit (21) kann über zumindest einen Sensor und/oder zumindest eine Elektrode (23) verfügen. Dieser Sensor kann bevorzugt auf einer dem Herz (900) zugewandten Fläche der zumindest einen expandierbaren Einheit (21) angebracht sein. Der Sensor kann aber auch an einer anderen Stelle der zumindest einen expandierbaren Einheit (21) angebracht sein. Der Sensor kann ein Temperatursensor, ein Drucksensor, ein pH-Sensor, ein Sauerstoffsensor, ein CO₂-Sensor, ein optischer Sensor oder ein Leitfähigkeitssensor sein. Alternativ kann der zumindest eine Sensor auch ein Impedanzsensor zur Überwachung der Haftung von auf der zumindest einen expandierbaren Einheit (21) aufgebrachten Zellen oder Zellkulturen oder aber zur Überwachung des Kontakts zum Epikard dienen. Die Elektrode (23) kann auch ein Sensor sein. Der Vorteil der Anbringung des zumindest einen Sensors und/oder der zumindest einen Elektrode (23) besteht darin, dass mithilfe der zumindest einen expandierbaren Einheit (21) ein steter Kontakt des zumindest einen Sensors und/oder der zumindest einen Elektrode (23) zur Herzoberfläche gewährleistet werden kann.

Lage und/oder Form der zumindest einen expandierbaren Einheit (21) kann in Form von Koordinaten im dreidimensionalen, euklidischen Raum so beschrieben werden, dass diese Koordinaten Oberflächenpunkten der Hülle (2) entsprechen und somit bspw. von Werkzeugmaschinen wie Fräsen, 3D-Druckern, industriellen Kleberobotern oder Laserschneidemaschinen interpretiert und genutzt werden können. Diese Koordinaten können anhand der dreidimensionalen Bilddaten des Herzes (900), bspw. aus einem CT-Datensatz, ermittelt werden. So können bspw. Lage und/oder Form der zumindest einen expandierbaren Einheit (21) an Lage und/oder Form einer anatomischen Struktur oder Lage und/oder Form einer pathologischen Veränderung des Herzes (900), bspw. durch einen Myokardinfarkt verursacht, angepasst werden. Die zumindest eine expandierbare Einheit (21) kann den Ort des Auftretens eines Myokardinfarkts zumindest teilweise bedecken oder kann in unmittelbarer räumlicher Nähe der Infarktstelle ("border zone") platziert werden.

Die Hülle (2) kann zumindest einen Sensor und/oder zumindest eine Elektrode (23) zur Messung eines Parameters des Herzes (900) oder zur Stimulation des Herzes (900) umfassen. Eine detailliertere Erläuterung folgt in einem nachfolgenden Anschnitt. Die zumindest eine Elektrode (23) kann aus Nitinol gefertigt sein, was ihre Deformierbarkeit erhöht und somit bei der minimalinvasiven Einbringung des Implantats in der nicht expandierten Konfiguration von Vorteil sein kann. Auch andere gängige Elektrodenmaterialien sind denkbar. Beispielsweise kann die Elektrode (23) auch eine Beschichtung aufweisen zur Verbesserung der elektrischen Eigenschaften aufweisen, was insbesondere bei der Messung zumindest eines Parameters des Herzes (900) vorteilhaft sein kann.

In die Hülle (2) kann zumindest ein Substanzträger (211) eingebracht werden. Der zumindest eine Substanzträger (211) dient der Speicherung zumindest einer Substanz (212), welche zumindest eine therapeutische Wirkung haben kann. Die Substanz (212) kann als Flüssigkeit, Gel, in pastöser Form, als Feststoff oder in einer Kombination daraus, bspw. als Emulsion oder Suspension, vorliegen. Die Substanz (212) kann anteilig aus einer Flüssigkeit und/oder einem Feststoff und/oder tierischer oder menschlichen Zellen und/oder Proteinen und/oder einem Gas bestehen. Die zumindest eine therapeutische Wirkung der Substanz (212) kann antithrombotisch, antiproliferativ, antiinflammatorisch, antineoplastischen, antimitotisch, antimikrobiell, antikoagulierend, cholesterinsenkend oder eine Kombination daraus sein. Die Substanz (212) kann einen Biofilm-Syntheseinhibitor, ein Antibiotikum, einen Antikörper, einen beta-Blocker oder Kombinationen davon enthalten. Die Substanz (212) kann lebendige biologische Zellen enthalten. Die Substanz (212) kann Proteine und/oder Wirkstoffe enthalten, welche das Überleben lebendiger biologischer Zellen ermöglichen und/oder die Zellaktivität und somit die therapeutische Effizienz beeinflussen können. Die Proteine und/oder Chemikalien können in Mikro- und/oder Nanopartikel eingekapselt sein, welche die eingekapselten Proteine und/oder Chemikalien mit einer definierten Freisetzungskinetik zur gerichteten Abgabe von Proteinen und/oder Chemikalien ermöglichen. Der zumindest eine Substanzträger (211) kann auf einer expandierbaren Einheit (21) platziert und befestigt werden. Der zumindest eine Substanzträger (211) kann auch direkt auf der Hülle (2) platziert und befestigt werden. Der Bereich der Platzierung des zumindest einen Substanzträgers (211) entweder auf der zumindest einen expandierbaren Einheit (21) oder der Oberfläche der Hülle (2) ist frei wählbar. Die freie Wahl des Platzierungsortes des zumindest einen Substanzträgers (211) ermöglicht die patientenspezifische Anpassung des Implantats. Lage und Form des Substanzträgers (211) können sich bspw. am Ort des Auftretens einer Herzerkrankung, bspw. ein Myokardinfarkt, orientieren. Der Substanzträger (211) kann den Ort des Auftretens eines Myokardinfarkts zumindest teilweise bedecken oder kann in unmittelbarer räumlicher Nähe der Infarktstelle ("Border Zone") platziert werden. Der Vorteil der Platzierung des zumindest einen Substanzträgers (211) in der Border Zone kann darin liegen, dass diese Region über lebendes Gewebe verfügt. Im Falle einer gut auf der Hülle (2) oder den expandierbaren Einheiten (21) haftenden Substanz (212) kann auch eine direkt Aufbringung in Form eines Aufstrichs oder einer Beschichtung in Erwägung gezogen werden, was den Substanzträger (211) in dieser Ausführungsform unnötig macht. Die Border Zone kann bspw. auf der Hülle (2) vormarkiert bzw. umrissen sein (ggf. mit einer gewissen Toleranz), so dass die Substanz (212) gezielt an dieser Stelle aufgetragen werden kann. Dies kann der Applikation an falscher Stelle vorbeugen. Der Substanzträger (211) in der vorliegenden Ausführungsform ist als an der zumindest einen expandierbaren Einheit (21) befestigtes, folienförmiges Bauteil dargestellt. Der Substanzträger (211) kann eine flache Form haben. Der Substanzträger (211) kann eine Dicke von 0,01 mm bis 5 mm, zwischen 0,05 mm bis 2 mm, bevorzugt zwischen 0,2 mm und 1,5 mm haben. Die dem Herz (900) zugewandte Fläche des Substanzträgers (211) - die Stirnfläche des Substanzträgers (211) - kann zumindest teilweise kreisförmig, elliptisch, rechteckig oder polygonal sein. Der zumindest eine Substanzträger (211) kann patientenspezifisch sein (mit Hinblick auf Geometrie und therapeutische Wirkung, bspw. bei der Dosierung des zumindest einen Wirkstoffes) und bspw. in seiner Größe, unter Berücksichtigung anatomischer Merkmale oder der Größe und Gestalt einer pathologischen Veränderung des Herzgewebes oder der Form der zumindest einen expandierbaren Einheit, auf welcher der Substanzträger (211) befestigt sein kann, angepasst sein. Die Befestigung des zumindest einen Substanzträgers (211) an der zumindest einen expandierbaren Einheit (21) oder der Hülle (2) erfolgt durch eine Kopplung durch Formschluss, Kraftschluss und/oder durch Stoffschluss. Im Falle einer formschlüssigen Verbindung kann die Kopplung durch Haken, Ösen, Knöpfe, Schlaufen oder zumindest einen Klett- und/oder Reißverschluss stattfinden. Dabei können zumindest ein Haken und zumindest eine Öse, zumindest ein Haken und zumindest eine Schlaufe, zumindest ein Knopf und zumindest ein komplementäres Formschlusselement oder zumindest ein Haken und zumindest ein weiterer Haken die Verbindungspartner sein. Im Falle einer kraftschlüssigen Verbindung kann der zumindest eine Substanzträger (211) mittels faden- oder lamellenförmigen Fortsätzen mit ebenfalls faden- oder lamellenförmigen Fortsätzen auf der zumindest einen expandierbaren Einheit (21) oder der Hülle (2) reibschlüssig verknüpft werden. Im Falle einer stoffschlüssigen Verbindung kann die Verbindung zwischen dem zumindest einen Substanzträger (211) und dem zumindest einen expandierbaren Element oder der Hülle (2) durch Kleben, Vulkanisieren oder Schweißen zustande kommen. In der vorliegenden Ausführungsform kann die Substanz (212) ein Gel oder eine Paste sein und auf die Stirnfläche des Substanzträgers (211) appliziert werden. Die Applikation der Substanz (212) auf die Stirnfläche des Substanzträgers (211) kann durch Kleben, Verstreichen, Auftragen oder Einspritzen erfolgen. Auch andere Formen der Applikation sind denkbar.

Der Substanzträger (211) kann auch von der flachen Form abweichen. Der Substanzträger (211) kann als Behältnis ausgelegt sein, er kann als Beutel oder Tasche ausgelegt sein. Der Substanzträger (211) in seiner Ausführungsform als Beutel oder Tasche kann die auszubringende Substanz (212) aufnehmen, halten und freigeben. Die Substanz (212) kann in den Substanzträger (211) im implantierten Zustand eingebracht und wieder abgeführt werden. Sie kann auch im nicht implantierten Zustand in den Substanzträger (211) eingebracht werden und kann bis zum Zeitpunkt der Explantation des Implantats im Substanzträger (211) verbleiben oder wieder abgeführt werden. Eine detailliertere Beschreibung findet sich in einem nachfolgenden Abschnitt.

Die Hülle (2) kann des Weiteren zumindest einen Sensor und/oder zumindest eine Elektrode (23) umfassen, mit deren Hilfe sich zumindest ein Parameter des Herzes (900) erfassen lässt, bspw. die Herzfrequenz, der Herzkammerdruck, die Kontaktkraft zwischen der Herzwand und einer expandierbaren Einheit, der systolische Blutdruck oder der diastolische Blutdruck. Der zumindest eine Sensor kann auch zur Erfassung von verschiedenen Parameter im Substanzträger (211) und/oder der Umgebung des Substanzträgers (211) dienen. Der zumindest eine Sensor kann ein Temperatursensor, ein Sensor zur Erfassung von auf dem Substanzträger (211) wirkenden mechanischen Kräften und ein Sensor zur Konzentrationsbestimmung von die Leitfähigkeit beeinflussenden Stoffen, den pH-Wert beeinflussenden Stoffen, sowie den Sauerstoff und CO2-Gehalt beeinflussenden Stoffen im Substanzträger (211) und/oder der Umgebung des Substanzträgers (211) sein. Der zumindest eine Sensor kann auch geeignet sein, den von einer expandierbaren Einheit (21) auf eine Fläche ausgeübten Druck, den pH-Wert, die Sauerstoffsättigung, den elektrischen Widerstand, die Osmolarität einer Lösung, oder den Durchfluss durch ein Gefäß zu messen. Der zumindest eine Sensor kann in, an oder auf der Rahmenstruktur (1) angebracht sein. Vorzugsweise wird der zumindest eine Sensor auf der Hülle (2) befestigt, die in die Rahmenstruktur (1) eingebracht werden kann. Der zumindest eine Sensor kann auch eine Elektrode (23) sein.

Die Kabel (221) können als Leiterbahnen ausgeführt sein. Die Leiterbahnen des zumindest einen Sensors und/oder der zumindest einen Elektrode (23) können zumindest teilweise auf die Hülle (2) aufgebracht sein. Beispielsweise können die Leiterbahnen auf Folien aus Polyimid (PI) aufgetragen werden, etwa durch Aufdampfen oder Sputtern. Auch andere Verfahren der Aufbringung oder der Abtragung, welche die Formung von Leiterbahnen ermöglichen, sind denkbar. Alternativ können leitfähige Sensor- und/oder Elektrodenkabel oder -leiterbahnen auf die Hülle (2) aufgeklebt oder in ihr verschweißt werden. Die Leiterbahnen können hierbei zumindest teilweise aus Nitinol bestehen. Die zumindest eine Elektrode (23) kann aus demselben Material wie die Leiterbahnen bestehen.

Wiederum alternativ können die Leiterbahnen und/oder die zumindest eine Elektrode (23) auch aus leitfähiger Tinte bestehen und auf die Hülle (2) aufgedruckt werden. Alternativ können die Leiterbahnen des zumindest einen Sensor und/oder der zumindest einen Elektrode (23) auch ohne Kopplung zur Hülle (2) innerhalb oder außerhalb der Hülle (2) entlang in Richtung Fixierhülse (41) und Kabelbaum (4) geführt werden.

Die zumindest eine Elektrode (23) kann geeignet sein, Bereiche des Herzes (900) zu stimulieren und/oder das Aktionspotential am Herzmuskel während des Erregungsprozesses zu messen. Insbesondere kann die zumindest eine Elektrode (23) geeignet sein den Herzmuskel mit Hilfe von elektrischen Impulsen zu stimulieren. Eine elektrische Stimulation kann einen Herzmuskel zu einer Kontraktion anregen. Die zumindest eine Elektrode (23) kann eine Herzschrittmacherelektrode sein. Die Elektrode (23) kann eine extrakardiale Stimulationselektrode sein. Der Herzmuskel kann mit einer Elektrode (23) vor, während oder nach einer Unterstützung der Pumpfunktion des Herzes (900) durch eine Rahmenstruktur (1) mit zumindest einer expandierbaren Einheit (21) stimuliert werden. Die zumindest eine Elektrode (23) kann auch eine Defibrillationsfunktion ausüben. Die zumindest eine Elektrode (23) kann hierbei die Form eines flächigen, leitfähigen Kissens haben ("patch"), welches sich an für eine Defibrillation geeigneten Stellen der Herzoberfläche befinden kann. Vorteil eines Patches kann der flächige Aufbau eines elektrischen Feldes sein, dessen Feldlinien geradliniger ausgerichtet sind als bei stabförmigen oder punktförmigen Elektrodengeometrien. Alternativ kann auch die Rahmenstruktur (1) als Elektrode (23) fungieren. In diesem Falle muss zumindest eine weitere Elektrode (23) vorhanden sein. Die Expansion einer expandierbaren Einheit (21) kann vor, während oder nach einer Stimulation mit einer Elektrode (23) ablaufen. Die Vorrichtung zur Unterstützung der Funktion eines Herzes (900) kann nur mit zumindest einer expandierbaren Einheit (21) oder nur durch Stimulation mit zumindest einer Elektrode (23) betrieben werden. Ein gleichzeitiger Betrieb der zumindest einen expandierbaren Einheit (21) und der zumindest einen Elektrode (23) kann synchron oder asynchron sein. Die zumindest eine Elektrode (23) kann auch als Sensor verwendet werden. Der zumindest eine Sensor und/oder die zumindest eine Elektrode (23) können zumindest ein elektrisches Kabel (221) umfassen. Das zumindest eine Kabel (221) kann in die Hülle (2) eingearbeitet sein. Das zumindest eine Kabel (221) kann auch eine gegenüber der Umgebung isolierte Leiterbahn sein. Das elektrisch leitfähige Material kann auf die Hülle (2) aufgetragen werden und zusätzlich isoliert werden. Das zumindest eine Kabel (221) kann mit der Hülle (2) gekoppelt sein, es kann in die Hülle (2) eingeschweißt werden, es kann mit der Hülle (2) verklebt werden. Je nach Lage des zumindest einen Sensors und/oder der zumindest einen Elektrode (23) kann das zumindest eine Kabel (221) auf der Außenseite oder der Innenseite der Hülle (2) verlaufen. Das zumindest eine Kabel (221) kann auch durch eine dafür in der Hülle (2) vorgesehene Öffnung (26) durchgeführt werden und auf der bei auf der Innenseite der Hülle (2) liegendem zumindest einem Sensor und/oder zumindest einen Elektrode (23) auf der Außenseite entlanggeführt werden. Das zumindest eine Kabel kann auch durch eine dafür in der Hülle (2) vorgesehene Öffnung (26) durchgeführt werden und auf der bei auf der Außenseite der Hülle (2) liegendem zumindest einem Sensor und/oder zumindest einen Elektrode (23) auf der Innenseite entlanggeführt werden.

Die Hülle (2) kann am unteren Ende eine Öffnung (26) aufweisen. Diese Öffnung (26) kann dazu dienen, die verlängerten Enden des zumindest einen Drahts oder der zumindest einen Strebe im Falle einer Ausführungsform, bei welcher die Rahmenstruktur (1) innerhalb der Hülle (2) liegt, aus dem Inneren der Hülle (2) nach außen zu führen. Diese Öffnung (26) kann dazu dienen, die zumindest eine Leitung (213) der zumindest einen expandierbaren Einheit (21) im Falle einer Ausführungsform, bei welcher die zumindest eine expandierbare Einheit (21) auf der Innenseite der Hülle (2) befestigt ist, aus dem Inneren der Hülle (2) nach außen zu führen. Diese Öffnung (26) kann dazu dienen, das zumindest eine Kabel (221) des zumindest einen Sensors und/oder der zumindest einen Elektrode (23) von der Innenseite der Hülle (2) in Richtung der Fixierhülse (41) auf die Außenseite der Hülle (2) zu führen. Diese Öffnung (26) kann dazu dienen, den Austausch von Substanzen (212) zwischen der Außenseite und der Innenseite der Hülle (2) zu erleichtern.

Die Rahmenstruktur (1) kann über zumindest eine röntgenopake Markierung (24) verfügen, mithilfe welcher sich die Positionierung und Orientierung der Rahmenstruktur (1) in Bezug auf ein Herz (900) während und nach der Implantation überprüft werden kann. Alternativ kann die zumindest eine röntgenopake Markierung (24) dazu dienen, die gewünschte und für die Implantation günstige Lage der Rahmenstruktur (1) im in ein Zuführsystem eingebrachten Zustand einzustellen und zu überwachen. Detaillierte Erläuterungen bzgl. des Zuführsystems erfolgen in einem anderen Abschnitt.

Figur 2 zeigt weiterhin eine Perikardschleuse (3), welche die Einbringung des Implantats in das Perikard (901) ermöglicht. Die Perikardschleuse (3) erzeugt einen Zuführkanal für das Implantat (100), indem sie ein Lumen gegenüber dem umliegenden Gewebe abgrenzt. Das Perikard (901) ist ein Sack aus Bindegewebe, der das Herz (900) umgibt und dem Herz (900) durch eine schmale Gleitschicht freie Bewegungsmöglichkeit gibt. In der Perikardhöhle (902) befindet sich seröse Flüssigkeit, die auch Liquor pericardii genannt wird. Damit dieser Liquor pericardii nicht durch eine im Zuge der Implantation des Implantats erzeugte chirurgische Öffnung (903) im Perikard (901) abfließen kann und damit keine anderen Flüssigkeiten oder Festkörper (wie z.B. Zellen, Proteine, Fremdkörper etc.) in die Perikardhöhle (902) gelangen können, kann eine Perikardschleuse (3) zumindest teilweise durch die chirurgisch erzeugte Öffnung (903) im Perikard (901) eingebracht werden. Die Perikardschleuse (3) kann die chirurgisch erzeugte Öffnung (903) im Perikard (901) zur Einbringung des Implantats in die Perikardhöhle (902) verschließen und abdichten. Die Perikardschleuse (3) kann ein inneres Lumen aufweisen, durch welches Teile der erfindungsgemäßen Vorrichtung ragen können. Nach der Implantation der Vorrichtung kann sich bspw. eine Fixierhülse (41) oder der ein Teil des Kabelbaums (4) in der Perikardschleuse (3) befinden und dort für die Implantationsdauer verbleiben. Die Perikardschleuse (3) dichtet die chirurgisch erzeugte Öffnung (903) im Perikard (901) zum Kabelbaum (4) ab. Die Perikardschleuse (3) kann einteilig ausgeführt werden. Die Perikardschleuse (3) kann auch zweiteilig oder mehrteilig ausgeführt werden. Die Perikardschleuse (3) kann eine rotationssymmetrische Form haben. Die Perikardschleuse (3) kann auch eine asymmetrische Form haben. Die Perikardschleuse (3) kann sich zumindest teilweise innerhalb der Perikardhöhle (902) befinden. Die Perikardschleuse (3) kann sich zumindest teilweise außerhalb des Perikards (901) befinden.

Die Perikardschleuse (3) kann aus einem Material bestehen. Die Perikardschleuse (3) kann auch aus zumindest zwei Materialien bestehen. Das Material der Perikardschleuse (3) kann zumindest ein Polymer sein. Die Perikardschleuse (3) kann aus Kunststoff, Polymer, Kautschuk, Gummi, Latex, Silikon, Polyurethan (PU) oder Polytetrafluorethylen (PTFE) bestehen. Die Perikardschleuse (3) kann zumindest teilweise aus einem Metall oder einer Metalllegierung bestehen. Die Perikardschleuse (3) kann zumindest teilweise aus einer Formgedächtnislegierung bestehen. Die Perikardschleuse (3) kann zumindest teilweise aus Nitinol bestehen. Die Perikardschleuse (3) kann aus einer Kombination zumindest zweier genannter Materialien bestehen.

Eine Ausführungsform der Perikardschleuse (3) ist in Figur 2 im Schnitt dargestellt. Die Ausführungsform ist zweiteilig. Sie umfasst die Vorrichtung zur Erzeugung des Lumens sowie einen Dichtring außerhalb des Perikards (901). Die Perikardschleuse (3) kann zumindest zwei Bauteile umfassen, sie kann jedoch auch allein aus der Vorrichtung zur Erzeugung des Lumens bestehen. Die Perikardschleuse (3) in dieser Ausführungsform besitzt zwei unterschiedliche Enden. Das eine Ende der Perikardschleuse (3) besitzt eine Dichtlippe (31), welche ringförmig sein kann. Das andere Ende der Perikardschleuse (3) besitzt in dieser Ausführungsform keine Dichtlippe (31). Alternativ kann die Perikardschleuse (3) auch an beiden Enden eine Dichtlippe (31) aufweisen. Das Material der Dichtlippe (31) kann dem Material der übrigen Perikardschleuse (3) gleichen. Das Material der Dichtlippe (31) kann vom Material der übrigen Perikardschleuse (3) abweichen. Das Material der Dichtlippe (31) kann Kunststoff, Polymer, Kautschuk, Gummi, Latex, Silikon, Polyurethan (PU) oder Polytetrafluorethylen (PTFE) sein. Die Dichtlippe (31) der Perikardschleuse (3) kann zumindest teilweise aus einem Metall oder einer Metalllegierung bestehen. Die Dichtlippe (31) der Perikardschleuse (3) kann zumindest teilweise aus einer Formgedächtnislegierung bestehen. Die Dichtlippe (31) kann neben der Dichtwirkung auch eine mechanisch stabilisierende in radialer und in Umfangsrichtung haben. Durch die Dicke der Dichtlippe (31) kann die Steifigkeit in radialer und in Umfangsrichtung eingestellt werden. Die Dicke der Dichtlippe (31) kann zwischen 1 mm und 10 mm liegen. Die Dicke der Dichtlippe (31) kann zwischen 3 mm und 6 mm liegen. Die Dichtlippe (31) kann eine selbsthemmende Wirkung haben. In der implantierten Form kann sich das obere Ende der Perikardschleuse (3) mit der Dichtlippe (31) gegen die Innenwand des Perikards (901) abstützen. Durch die Verjüngung des Perikards (901) zur Herzspitze hin kann einen mechanische Selbsthemmung zwischen der Perikardschleuse (3) und dem Perikard (901) auftreten und die Dichtwirkung sowie die mechanische Stabilität und Positionstreue der Perikardschleuse (3) erhöhen.

Der Kern der Dichtlippe (31) kann aus einem anderen Material sein als die um den Kern der Dichtlippe (31) gelegte Ummantelung. Der Kern der Dichtlippe (31) kann aus zumindest einem Teil aus Federstahl oder einer Formgedächtnislegierung geformt sein, die Ummantelung des Kerns aus einem Polymer. Im Falle einer Ausführungsform einer Perikardschleuse (3) mit einem Kern der Dichtlippe (31) aus Metall oder einer Metalllegierung kann das Material der Ummantelung des Kerns der Dichtlippe (31) vom Material des Kerns der Dichtlippe (31) abweichen. In der vorliegenden Ausführungsform des Implantats besteht die Vorrichtung zur Erzeugung des Lumens aus einem Material, bevorzugt aus einem Polymer. Das Ende mit der Dichtlippe (31) ist in dieser Ausführungsform im Inneren der Perikardhöhle (902) verortet. Dort steht es in Kontakt mit der Innenwand des Perikards (901). Die Dichtlippe (31) gewährleistet die Dichtwirkung und verhindert den Flüssigkeitsaustritt aus dem Perikard (901) oder den Eintritt von Stoffen in die Perikardhöhle (902). Das selbst-expandierende Implantat (100) kann durch die Perikardschleuse (3) in die Perikardhöhle (902) eingeführt werden. Somit befindet sich auch das der Rahmenstruktur (1) nahe Ende des Kabelbaums (4) mit der Fixierhülse (41) in der Perikardschleuse (3). Zwischen der Fixierhülse (41) und der Perikardschleuse (3) kann eine dichtende Verbindung aufgebaut werden. In der vorliegenden Ausführungsform wird die Dichtwirkung durch einen über die Perikardschleuse (3) gestülpten Dichtring erzeugt. Die Dichtung zwischen Perikardschleuse (3) und der Fixierhülse (41) kann auch anderweitig ermöglicht werden. Die Dichtwirkung kann alternativ durch Nähen der Perikardschleuse (3) an die Fixierhülse (41), Festklemmen der Perikardschleuse (3) durch eine Klemme, eine Schelle oder mittels zumindest einem Kabelbinder, welche um die Fixierhülse (41) und die darüber liegende Perikardschleuse (3) gelegt werden können, erzeugt werden. Die Dichtwirkung kann auch durch die Perikardschleuse (3) allein bzw. durch einen Teil einer Ausführungsform der Perikardschleuse (3) erzeugt werden. Die dichtende Wirkung kann in diesem Fall durch Zunähen, Verknoten oder Verschweißen des unteren Endes der Perikardschleuse (3) erzeugt werden. Die komplette Abdichtung der chirurgisch erzeugten Öffnung (903) im Perikard (901) kann an zumindest zwei Grenzflächen erfolgen: zwischen Perikard (901) und Perikardschleuse (3) sowie zwischen Perikardschleuse (3) und Umgebung bzw. Perikardschleuse (3) und Fixierhülse (41).
Ein zusätzlicher Nutzen der Perikardschleuse (3) kann sein, dass sie die Explantation des Implantats erleichtert, da die Perikardschleuse (3) die gemeinsame Oberfläche zwischen Perikard (901) und dem Implantat und somit die Adhäsion zwischen diesen verringert.
Figur 2 zeigt die Perikardschleuse (3) in ihrer Konfiguration nach Einbringung des Implantats in die Perikardhöhle (902). Der Vorgang der Einbringung der Perikardschleuse (3) wird in einem späteren Abschnitt näher erläutert.

Eine alternative Ausführungsform des Implantats kann auch ohne die Rahmenstruktur (1) ausgeführt sein. Dies kann der Fall sein, wenn keine mechanische Augmentation des Herzes (900) vonnöten ist. Alternativ kann das Implantat (100) ohne Hülle (2), aber mit einer Rahmenstruktur (1) ausgeführt sein. Dies kann von Vorteil sein, wenn vornehmlich die mechanische Stützwirkung der Rahmenstruktur (1) genutzt werden soll.

Wiederum alternative Ausführungsformen können ausschließlich aus der Rahmenstruktur (1) und/oder der Hülle (2) sowie zumindest einer expandierbaren Einheit (21) bestehen. Solche Ausführungsformen können daher komplett implantierbar sein. Dies hat den Vorteil, dass die Entzündungsgefahr verringert wird, da kein Kabelbaum (4) vom Körperinnern nach außen tritt. Die zumindest eine expandierbare Einheit (21) kann bspw. mithilfe zumindest eines Federelements einen bestimmten Expansionsgrad einstellen bzw. beibehalten, womit etwa ein permanenter Kontakt eines auf der zumindest einen expandierbaren Einheit (21) angebrachten Substanzträgers (211) mit der Herzoberfläche gewährleistet werden kann. Das Federelement kann aus Polymer oder Kunststoff, Metall oder Metalllegierungen oder einem Formgedächtnismaterial bestehen. Bei Ausführungsformen, welche über kein Federelement verfügen, kann die expandierbare Einheit (21) auch pneumatisch/hydraulisch betätigt werden. So kann etwa ein konstanter oder aber ein zeitlich veränderlicher Expansionsgrad eingestellt werden. Anstelle zumindest einer expandierbaren Einheit (21) kann auch ein passives Silikonkissen verwendet werden. Das Implantat (100) kann autark und ohne Energiezufuhr von außerhalb des Körpers ausgeführt sein. Alternativ kann das Implantat (100) auch verbindungslos, etwa per Induktion, mit Energie versorgt werden.

Eine weitere Ausführungsform kann einzig aus zumindest einer Hülle (2) bestehen, welche mit einer therapeutischen Substanz (212) versehen sein kann. Eine derartige Ausführungsform kann sehr platzsparend implantiert werden. Vorteil einer derartigen Ausführungsform kann sein, dass sie ohne Kabelbaum (4) und Perikardschleuse (3) auskommt.

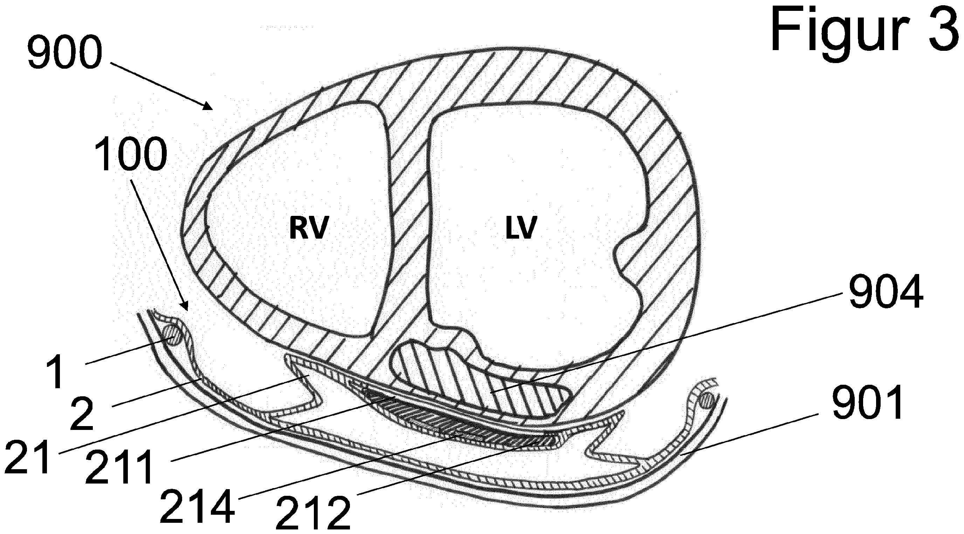

**Figur 3** zeigt einen Schnitt durch ein Herz (900), um welches eine Ausführungsform des Implantats (100) gelegt ist, welches wiederum vom Perikard (901) umgeben ist. In der Herzwand ist Infarktgewebe (904) gegenüber intaktem Gewebe des Herzes (900) zeichnerisch abgesetzt. Eine expandierbare Einheit (21) kann derart positioniert werden, dass sie einen begrenzten Teil der Oberfläche des Herzes (900), welcher direkt über dem Infarktbereich liegt, gänzlich abdeckt. Die expandierbare Einheit (21) ist in diesem Ausführungsbeispiel als inflatierbare, balgförmige Kammer dargestellt. Die expandierbare Einheit (21) kann auch von der Balgform abweichen. Die Unterseite der expandierbaren Einheit (21) wird in dieser Ausführungsform von einem Teil der Hülle (2) gebildet. Die Unterseite der expandierbaren Einheit (21) kann auch separat ausgeführt sein und anschließend an der Hülle (2) befestigt werden, etwa durch Kleben, Schweißen oder Vulkanisieren. Mithilfe der expandierbaren Einheit (21) kann der Substanzträger (211) mit der Herzoberfläche in Kontakt gebracht werden. Der Substanzträger (211) ist in dieser Ausführungsform als Tasche mit Öffnung an der Stirnfläche dargestellt. Die Öffnung an der Stirnfläche ermöglicht den Substanzübertritt vom Substanzträger (211) zur Herzoberfläche. Die Unterseite des Substanzträgers (211) ist in dieser Ausführungsform Teil der Stirnfläche der expandierbaren Einheit (21). Die Unterseite des Substanzträgers (211) kann auch separat ausgeführt sein und an der Stirnfläche der expandierbaren Einheit (21) befestigt werden, etwa durch Kleben, Schweißen, Vulkanisieren, alternativ durch Einhängen oder mithilfe von Klettverschlüssen.
Im Allgemeinen ist der Substanzträger (211) an einem beliebigen Ort auf der Oberfläche der Hülle (2), der zumindest einen expandierbaren Einheit (21) oder der Rahmenstruktur (21) platzierbar. In manchen Ausführungsformen kann der Substanzträger (211) zumindest teilweise auf zumindest einer expandierbaren Einheit (21) platziert werden. Die vom Substanzträger (211) bedeckte Oberfläche kann hierbei identisch und deckungsgleich zu der Stirnfläche der expandierbaren Einheit (21) sein. Die vom Substanzträger (211) bedeckte Oberfläche kann jedoch auch größer oder kleiner als die Stirnfläche der expandierbaren Einheit (21) sein. Die vom Substanzträger (211) abgedeckte Fläche kann durch die Größe des zu therapierenden Gebiets auf der Herzoberfläche beeinflusst sein. Bei Ausführungsformen mit Substanzträgern (211), welche eine größere Fläche als die von ihm bedeckte zumindest eine expandierbare Einheit (21) einnimmt, kann die durch Expansion der expandierbaren Einheit (21) in den Substanzträger (211) eingebrachte mechanische Spannung eine therapeutische Wirkung haben. Zu diesem Punkt erfolgt in einem nachfolgenden Abschnitt eine genauere Beschreibung. Ist der Kontakt zwischen Substanz (212) bzw. Substanzträger (211) und der Herzoberfläche nicht permanent, d.h. in manchen Ausführungsformen bevorzugt steuer- oder zumindest einstellbar, so kann die Platzierung des Substanzträgers (211) auch in Bereichen des Implantats (100) vorgenommen werden, in welchen keine Überdeckung des Substanzträgers (211) und zumindest einer expandierbaren Einheit (21) vorhanden ist.

Alternativ ist auch eine Ausführungsform denkbar, bei welcher der Substanzträger (211) mehrere expandierbare Einheiten (21) und/oder Flächen der Hülle (2) ohne expandierbare Einheiten (21) bedeckt, etwa, wenn die Ausführung des Substanzträgers (211) mehrere separate oder zusammenhängende Teilbereiche mit einer Substanz (212) aufweist, welche an unterschiedlichen Bereichen der Herzoberfläche mit dieser in Kontakt gebracht werden können.

Die Form des Substanzträgers (211) bzw. dessen Grundfläche ist in dieser Ausführungsform rechteckig dargestellt. Alternativ kann die Form des Substanzträgers (211) auch eine beliebige Gestalt annehmen, um derart effizienter beim Substanzeinsatz zu sein und besser auf die Geometrie des zu therapierenden Bereichs des Herzes (900) eingehen zu können. Auch sind Substanzträger (211) denkbar, welche örtlich separiert mehrere Substanzen (212), die unterschiedlicher Art sein können, enthalten können.

Weiterhin kann der Substanzträger zumindest einen Sensor (214) umfassen. Der zumindest eine Sensor (214) kann auch eine Elektrode sein. Der zumindest eine Sensor (214) kann dazu verwendet werden, zumindest einen Parameter im Substanzträger oder der Substanz zu erfassen. Der zumindest eine Sensor (214) kann ein Konzentrationsmesssensor, ein pH-Sensor, ein Sauerstoffsättigungssensor oder ein Drucksensor sein. Auch andere Sensortypen sind denkbar. Ist der zumindest eine Sensor (214) eine Elektrode und sind davon zumindest zwei angebracht, so kann bspw. das elektrische Potential zwischen den beiden Anbringungsorten der Elektroden gemessen werden. Auch können die zumindest zwei Elektroden dazu genutzt werden, eine elektrische Spannung anzulegen.

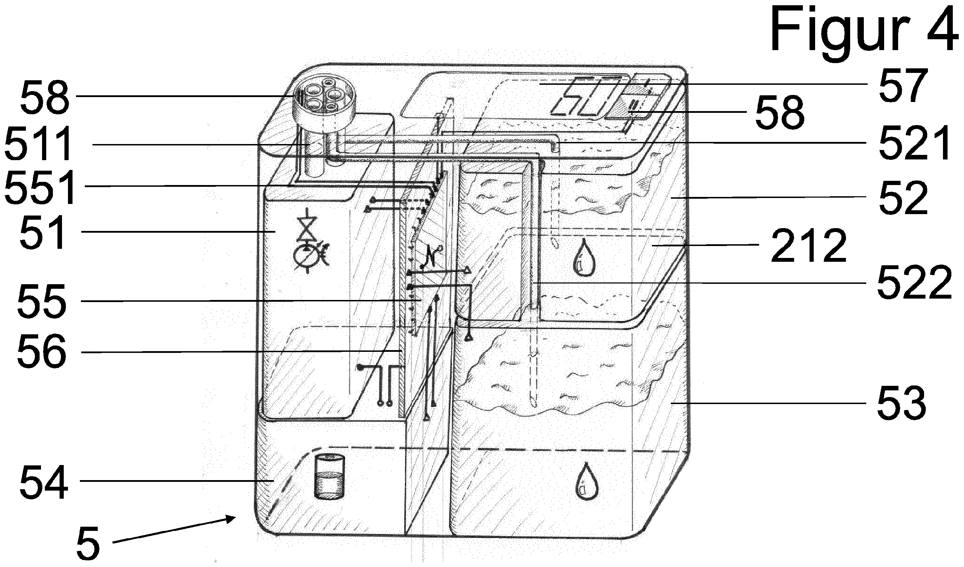

**Figur 4** zeigt eine Ausführungsform der Versorgungseinheit (5). Die Versorgungseinheit (5) kann die Energieversorgung der zumindest einen Aktuatoreinheit (51) der zumindest einen expandierbaren Einheit, insbesondere zur Aktuation einer inflatierbaren, balgförmigen, pneumatischen Kammer, gewährleisten. Die Versorgungseinheit (5) kann den zumindest einen Sensor und/oder die zumindest eine Elektrode mit Energie versorgen. Die Versorgungseinheit (5) kann das zumindest eine Element zur Einstellung eines Parameters, bspw. zumindest eine Heizwendel, im zumindest einen Substanzträger in der Ausführungsform als befüllbare und entleerbare Tasche mit Energie versorgen. Weiterhin kann die Energieversorgung für die zumindest zwei Elektroden zur Anlegung einer elektrischen Spannung am Substanzträger, die Pumpvorrichtung für das zumindest eine Reservoir (52) und die zumindest eine Steuereinheit mittels der Versorgungseinheit (5) gewährleistet werden. Auch kann die Versorgungseinheit (5) zumindest ein auffüll- und nachfüllbares Reservoir (52) für zumindest eine Substanz (212) umfassen. Das zumindest eine Reservoir (52) kann austauschbar sein. Das zumindest eine Reservoir (52) kann in die Versorgungseinheit (5) einbaubar und aus ihr ausbaubar sein. Das zumindest eine Reservoir (52) kann auch getrennt von der Versorgungseinheit (5) ausgeführt werden. Das zumindest eine Reservoir (52) kann als Beutel, Kanister oder Flasche ausgeführt werden. Das zumindest eine Reservoir (52) kann dazu genutzt werden, über zumindest eine dafür vorgesehene Leitung (521) eine Substanz (212) zum Substanzträger hin oder in den Substanzträger hinein zu leiten. Das zumindest eine Reservoir (52) kann über zumindest eine Pumpvorrichtung verfügen, welche die zumindest eine Substanz (212) zum zumindest einen Substanzträger befördern und wieder abpumpen kann. Mithilfe des zumindest einen Reservoirs (52) kann auch eine kontinuierliche Zirkulation einer Substanz (212) durch zumindest einen Substanzträger in einer Ausführungsform als Tasche gewährleistet werden. In einer solchen Ausführungsform kann die Zirkulation durch eine Pumpvorrichtung gewährleistet werden. Das zumindest eine Reservoir (52) kann auch über zumindest eine Filtervorrichtung verfügen. Die zumindest eine Filtervorrichtung kann zumindest eine Membran umfassen. Die zumindest eine Membran kann semipermeabel sein. Die zumindest eine Membran kann dazu dienen, zumindest eine Substanz (212) am Übertritt von einem Teilvolumen des zumindest einen Reservoirs (52) in das zumindest eine andere Teilvolumen des Reservoirs (52) zu verhindern oder exklusiv zu ermöglichen. Die zumindest eine Leitung (521) kann zum zumindest einen Substanzträger hin offen sein. Die zumindest eine Leitung (521) kann zum zumindest einen Substanzträger hin auch abgeschlossen sein. Die zumindest eine Leitung (521) kann zum zumindest einen Substanzträger hin auch für zumindest eine Substanz (212) durchlässig sein und zugleich für zumindest eine Substanz (212) undurchlässig sein. Die zumindest eine Leitung (521) kann im Bereich, in welchem der zumindest eine Substanzträger verortet ist, semipermeabel sein. Eine weitere Ableitung (522) kann vom Substanzträger weg zurück zum Reservoir (52) führen. Die zumindest eine Ableitung (522) kann auch eine Weiterführung der zumindest einen Zuleitung zum Substanzträger sein. Die zumindest eine Ableitung (522) kann auch nicht an das Reservoir (52) angeschlossen werden. Die zumindest eine Ableitung (522) kann in ein weiteres, separates Reservoir (53) führen.

Die Versorgungseinheit (5) hat einen Energiespeicher, mit Hilfe dessen die zumindest eine expandierbare Einheit angetrieben werden kann. Der Energiespeicher kann in Form eines Akkumulators (54) vorliegen, der elektrische Energie bereitstellt, um die expandierbare Einheit expandieren zu können. Der Akkumulator (54) kann gewechselt werden. Die Versorgungseinheit (5) kann auch einen Druckspeicher enthalten, der ein komprimiertes Gas bereitstellt, um eine inflatierbare Kammer expandieren zu können. Geeignete Gase sind unter anderem Druckluft, CO₂, oder Edelgase. Das Gehäuse der Versorgungseinheit (5) selbst kann als Druckspeichergehäuse dienen. Dies hat den Vorteil, dass der ohnehin im Gehäuse vorhandene Hohlraum genutzt werden kann und auf eine Ausführung mit separatem Druckspeicher verzichtet werden kann. Die Versorgungseinheit (5) kann weiterhin Pumpen, Ventile, Sensoren und zumindest ein Display (57) enthalten. Die Versorgungseinheit (5) kann ferner zumindest einen Mikroprozessor (55) auf einer Mikroprozessorplatine (56) umfassen, der geeignet ist, Daten von dem zumindest einen Sensor zu empfangen und zu verarbeiten. Wird die Versorgungseinheit (5) außerhalb des Körpers getragen, kann die zu bereitstellende Energie durch eine direkte Verbindung über ein Kabel transferiert werden, oder kabellos, z.B. durch elektromagnetische Induktion. Der zumindest eine Mikroprozessor (55) kann auch die Aktuation der zumindest einen expandierbaren Einheit steuern und/oder die Zirkulation der zumindest einen Substanz (212) vom Reservoir (52) über zumindest eine Substanzleitung in den Substanzträger und gegebenenfalls wieder zurück in das zumindest eine Reservoir (52) oder zumindest eine anderes Reservoir (53) regeln.

Die Versorgungseinheit (5) kann auch zumindest zum Teil im Körperinnern ausgeführt sein. Beispielsweise kann ein separater Druckspeicher, etwa zum Zwecke des Nachfüllens eines unter Druck stehenden Fluides, im Körperinneren liegen. Alternativ kann auch die zumindest ein Akkumulator (54) für den Betrieb der energieverbrauchenden Komponenten des Implantats im Körperinnern liegen. Die Daten von dem zumindest einen Sensor können ebenfalls direkt über ein Kabel oder kabellos über eine Funktechnik, wie z.B. Bluetooth, übertragen werden.
Das Implantat kann ferner einen Kabelbaum umfassen, der die zumindest eine expandierbare Einheit und/oder den zumindest einen Sensor oder die zumindest eine Elektrode mit der Versorgungseinheit (5) verbindet. Eine detaillierte Beschreibung des Kabelbaums ist in einem vorangegangen Abschnitt zu finden. Im Falle eines mehrteiligen Kabelbaums kann ein Kabel mit Steckerteil an der zumindest einen expandierbaren Einheit und/oder an dem zumindest einen Sensor oder der zumindest einen Elektrode und auch ein Kabel an der Versorgungseinheit (5) angebracht werden, an dessen Ende sich vorzugsweise ebenfalls ein Steckerteil befindet.

Die Versorgungseinheit (5) kann über eine kabellose Kommunikationsschnittstelle verfügen. Über diese können Daten zwecks Auswertung oder Weitergabe, bspw. an die in einem vorangehenden Abschnitt beschriebenen Steuerungs- und Überwachungseinheiten übertragen werden. Diese kabellose Verbindung kann etwa ein WLAN- oder eine Bluetooth-Verbindung sein.
Alle Leitungen, bspw. zumindest eine pneumatische Leitung (511), zumindest eine elektrische Leitung (551) und/oder zumindest eine Substanzleitung (521), können der vereinfachten Handhabbarkeit wegen im weiblichen Teil eines Multikonnektors (58) zusammengefasst sein. Die detaillierte Beschreibung dieses Multikonnektors erfolgt an anderer Stelle.

Weiterhin kann die Versorgungseinheit (5) über zumindest eine Bedienelement (58) verfügen, über welches sich zumindest eine Funktion der Versorgungseinheit (5) zumindest überprüfen lässt. Das zumindest eine Bedienelement (58) kann auch der Einstellung zumindest eines Parameters oder einer Funktion der Versorgungseinheit (5) dienen. Beispielsweise kann so der Massenstrom der Substanz (212) überwacht und/oder eingestellt werden.

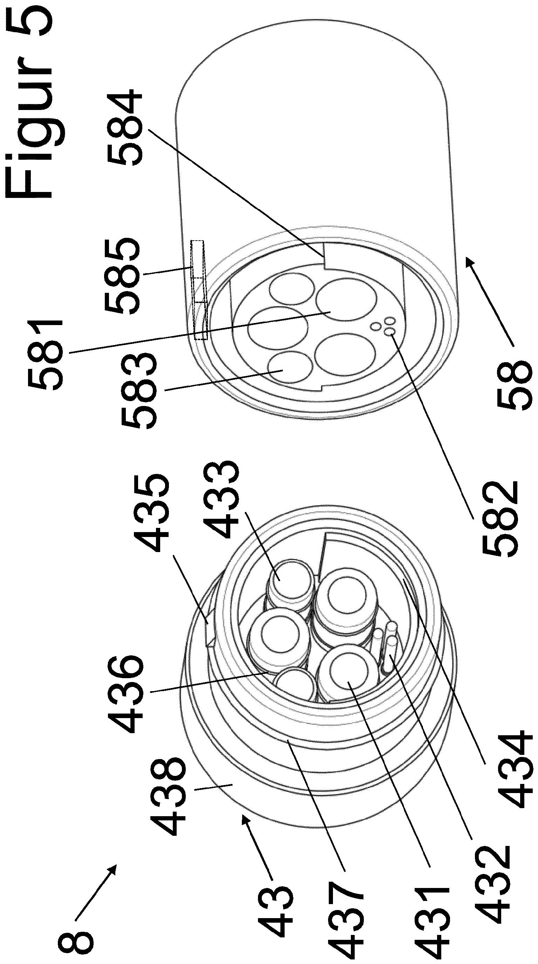

**Figur 5** zeigt eine Ausführung des Multikonnektors (8), welcher zwei komplementäre Steckerteile umfasst, einen männlichen Steckerteil (43) und einen weiblichen Steckerteil (58). Der männliche Steckerteil (43) umfasst zumindest einen Anschluss (431) zumindest einer pneumatischen bzw. hydraulischen Leitung, zumindest einen Anschluss zumindest einer elektrischen Leitung (432) und zumindest einen Anschluss (433) zumindest einer Substanzleitung. Der komplementäre, weibliche Steckerteil (58) umfasst zumindest einen Anschluss (581) zumindest einer pneumatischen bzw. hydraulischen Leitung, zumindest einen Anschluss zumindest einer elektrischen Leitung (582) und zumindest einen Anschluss (583) zumindest einer Substanzleitung. Die in Figur 5 gezeigte Ausführungsform umfasst Anschlüsse für drei pneumatischen Leitungen (431, 581), drei elektrische Leitungen (432, 582) sowie zwei Substanzleitungen (433, 583). Vorteile des Multikonnektors (8) sind die kompakte räumliche Zusammenfassung verschiedenartiger Leitungstypen, die einfache Handhabe beim Koppeln und Entkoppeln sowie die einfache Abdichtbarkeit.

Der Multikonnektor (8) kann des Weiteren Vorrichtungen zur Zentrierung (434, 584) enthalten, bspw. in Form eines Zentrierdorns (434) und der komplementären Form (584), welche die korrekte Orientierung zur problemfreien Zusammenführung der beiden komplementären Steckerteile (43,58) gewährleistet. Der Zusammenhalt der Steckverbindung kann durch einen Kopplungsmechanismus, bspw. einen reversiblen, zerstörungsfreien Schnappverschluss (435, 585), sichergestellt werden. In der dargestellten Ausführungsform ist ein Schnapper (435) auf der männlichen Steckerseite (43) dargestellt, welcher in das dafür vorgesehene komplementäre Schloss (585) auf der weiblichen Steckerseite (58) einrasten kann. Der Schnapper (435) kann in manchen Ausführungen des Multikonnektors (8) auch als Zentriervorrichtung dienen.

Der Multikonnektor (8) kann abgedichtet sein gegen den unerwünschten Ein- und/oder Austritt von bspw. Fluiden, Fluid-Festkörper-Gemischen oder Granulaten. Auch die Abdichtung gegen andere Medien ist denkbar. Die Dichtigkeit wird durch Dichtungen, z.B. in Form von Dichtringen (436) wie in der gezeigten Ausführung, erzielt. Jede Leitung, welche über ein dicht zu haltendes Lumen verfügt, kann mit einer individuellen Dichtung mit Dichtringen (436) versehen sein. Zudem kann der Multikonnektor (8) im gesamten zusätzlich abgedichtet sein. In der dargestellten Ausführungsform kann dies ein an der Steckerhülse (438) des männlichen Teils (43) angebrachter Dichtring (437) sein.

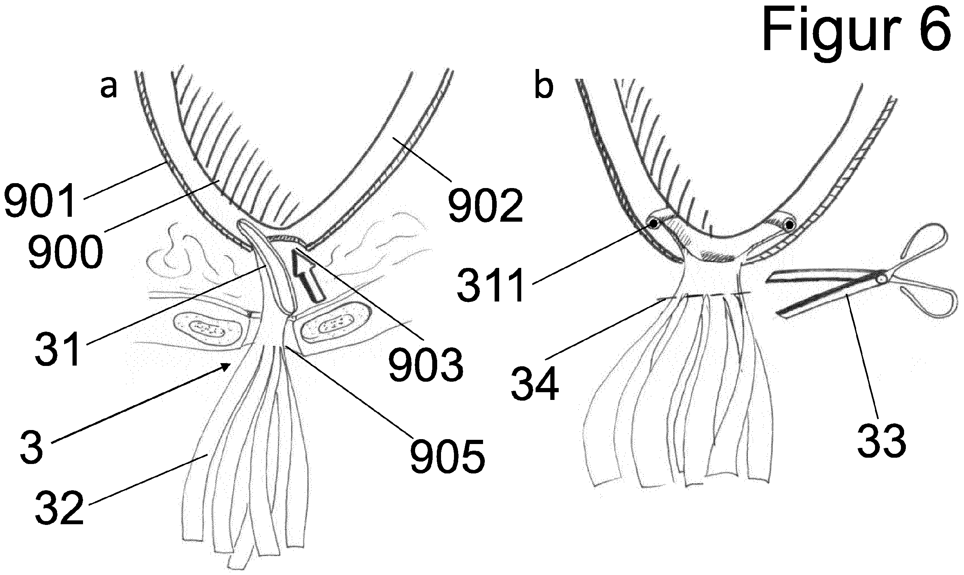

**Figur 6a und 6b** zeigen den Vorgang der Einbringung einer Ausführungsform der Perikardschleuse (3) durch einen chirurgisch erzeugten Zugang (905) und eine chirurgisch erzeugten Öffnung (903) in die Perikardhöhle (902). Die Perikardschleuse (3) hat ein proximales Ende und ein distales Ende. Das proximale Ende kann zumindest eine Lamelle (32) besitzen. Das distale Ende besitzt eine Dichtlippe (31). Das proximale Ende der Perikardschleuse (3) ist während der Implantation dem Operateur zugewandt, das distale Ende ist dem Patienten zugewandt. Das für die Perikardschleuse (3) verwendete Material ist in einem vorangehenden Abschnitt näher erläutert. Das Material der Perikardschleuse (3) kann derart gewählt werden, dass die Perikardschleuse (3) großen elastischen Deformationen unterzogen werden kann. Diese Eigenschaft ist beim Einbringungsvorgang der Perikardschleuse (3) in die Perikardhöhle (902) von Vorteil. Das Material zumindest eines Teils der Perikardschleuse (3) kann ein Polymer sein. Das Material zumindest eines Teils der Perikardschleuse (3) kann Silikon, Polytetrafluoräthylen (PTFE) oder Polyurethan (PU) sein. Das Material zumindest eines weiteren Teils der Perikardschleuse (3) kann Stahl, eine Formgedächtnislegierung oder ein von den obigen Nennungen abweichendes Polymer oder ein Kunststoff sein. Die Dichtlippe (31) kann bspw. einen Kern (311) aus einem steiferen Stoff enthalten, welcher die Formstabilität der Perikardschleuse (3) erhöhen kann. Die Perikardschleuse (3) kann zusätzlich auf der Innenseite und/oder auf der Außenseite beschichtet sein, so dass die Reibung zwischen der Perikardschleuse (3) und in sie eingeführte Bauteile eingestellt werden kann. In manchen Bereichen kann eine erhöhte Reibung von Vorteil sein (bspw. an der Außenseite in Kontakt zu Körpergewebe), in anderen Bereichen kann, bspw. zur vereinfachten Einführung eines Zuführsystems oder des Implantats, die Reibung durch die Beschichtung herabgesetzt werden.

Die Perikardschleuse (3) kann aus zumindest einem Teil gefertigt sein. Die Perikardschleuse (3) ist bevorzugt rotationssymmetrisch ausgeführt. Sie umfasst Lamellen (32) am proximalen Ende, eine Halsregion sowie eine Dichtlippe (31), welche einen von der Halsregion abweichenden Durchmesser haben kann. Bevorzugt hat die Dichtlippe (31) einen größeren Durchmesser als die Halsregion der Perikardschleuse (3), so dass nach der Einbringung der Perikardschleuse (3) in die Perikardhöhle (902) Selbsthemmung auftreten kann. Der Durchmesser der Dichtlippe (31) kann um den Faktor 1.0 bis 2.0 größer sein als der Durchmesser der Halsregion, er kann um den Faktor 1.05 bis 1.2 größer sein als der Durchmesser der Halsregion.

Die Perikardschleuse (3) kann unter anderem dazu dienen, einen Einführkanal für eine minimalinvasive Implantation des Implantats zu erzeugen und aufrecht zu erhalten. Die Perikardschleuse (3) kann mit ihrer äußeren Oberfläche und/oder mit einer Dichtlippe (31) eine Öffnung (903) im Perikard (901) verschließen und/oder abdichten und ein inneres Lumen als Zuführkanal für die Implantation des Implantats erzeugen.

Das Lumen der Halsregion kann einen Durchmesser von 5 mm bis 100 mm, bevorzugt 40 mm bis 80 mm, haben. Die Perikardschleuse (3) kann aus einem Schlauch gefertigt sein. Alternativ kann die Perikardschleuse (3) aus separaten Teilen für die Aufweitung zur Dichtlippe (31) hin, die Halsregion sowie die Lamellen (32) am proximalen Ende bestehen. Der sich aufweitende Bereich und die Halsregion der Perikardschleuse (3) können auch aus einem Teil gefertigt sein, während die Lamellen (32) ebenfalls ein eigenes Teil darstellen, welches mit dem anderen Teil bspw. durch Kleben oder Verschweißen oder eine andere Fügetechnik, gekoppelt werden kann. Die Materialien der unterschiedlichen Teile können gleich oder unterschiedlich sein. Es kann vorteilhaft sein, einen Teil der Perikardschleuse (3) steifer auszulegen als den anderen. Die Wandstärke der Perikardschleuse (3) kann 0.2 mm bis 4 mm sein. Die Wandstärke der Perikardschleuse (3) kann 0.4 mm bis 2.5 mm betragen. Über den Durchmesser kann die Größe des durch die Perikardschleuse (3) erzeugten Lumens eingestellt werden. Durch die Wandstärke kann die Steifigkeit der Perikardschleuse (3) eingestellt werden. Die Wandstärke kann lokal variieren. An Orten, bspw. im Bereich der Aufweitung zur Dichtlippe (31) hin oder in der Halsregion, kann eine höhere Wandstärke vorliegen, damit die Einführung des Implantats vereinfacht wird, indem die Perikardschleuse (3) definierter verformbar wird. Am proximalen Ende kann die Perikardschleuse (3) Lamellen (32) aufweisen, die in Richtung der Schleusenlängsachse ragen. Wird die Perikardschleuse (3) bspw. aus einem durchgängigen Stück Schlauch thermogeformt oder wird die Perikardschleuse (3) durch Gießen hergestellt, so können die Lamellen (32) auch durch Einschnitte entlang der Axialrichtung der Perikardschleuse (3) hergestellt werden oder nachträglich an die Perikardschleuse (3) gekoppelt werden, bspw. durch Kleben. Entlang des Umfangs der Perikardschleuse (3) können sich 1 bis 16 Lamellen (32), bevorzugt 2 bis 6 Lamellen (32), befinden. Die Lamellen (32) können entlang des Umfangs äquidistant angeordnet sein, was zu gleich breiten Lamellen (32) führt. Sie können untereinander auch unterschiedliche Abstände aufweisen, was zu unterschiedlich breiten Lamellen (32) führt. Während der Implantation können die Lamellen (32) der Perikardschleuse (3) zumindest teilweise außerhalb des Körpers positioniert sein und zum Aufspannen der Perikardschleuse (3) dienen. Die Lamellen (32) können eine spreizende Wirkung haben, indem sie in radialer Richtung gezogen werden können. Die Lamellen (32) können dazu dienen, die Perikardschleuse (3) radial möglichst gleichmäßig zu spreizen. Die Lamellen (32) können dadurch ein möglichst großes, sich an den Haut- und Körperhüllenschnitt anschmiegendes Lumen zu erzeugen. Werden mehr axiale Schnitte gesetzt, d.h. gibt es mehr Lamellen (32), kann der Spreizvorgang und das Anschmiegen der Perikardschleuse (3) an die Form des Haut- und Körperhüllenschnittes feiner justiert werden. Unterschiedlich breite Lamellen (32) entlang des Umfangs können dazu dienen, die Spreizung und das Anschmiegen der Perikardschleuse (3) in unterschiedlichen Umfangssegmenten der Perikardschleuse (3) unterschiedlich fein zu justieren, um etwa verschiedenen Geweben mit unterschiedlichen Materialeigenschaften oder der individuellen Körperform des Patienten Rechnung zu tragen. Die Lamellen (32) können dazu dienen, die Eintrittstiefe der Perikardschleuse (3) zu justieren, indem man sie mehr oder weniger stark anzieht. Zieht man sie stärker an, kann sich die Eintrittstiefe der Perikardschleuse (3) verringern, zieht man schwächer an, kann sich die Eintrittstiefe der Perikardschleuse (3) vergrößern. Die Lamellen (32) können dazu dienen, eine konstante Eintrittstiefe der Perikardschleuse (3) einzustellen.

Die Perikardschleuse (3) kann auch durch Gießen gefertigt werden. Der Perikardschleuse (3) kann entlang der axialen Richtung auch einen veränderlichen Durchmesser aufweisen. In der vorliegenden Ausführungsform kann sich die Perikardschleuse (3) vom oberen Ende her bis zu einem bestimmten Punkt unterhalb des oberen Endes verjüngen und kann sich dann unterhalb dieses Punktes wieder weiten.

Zusätzlich kann im Bereich der Aufweitung der Perikardschleuse (3) zur Dichtlippe (31) hin und/oder in der Halsregion eine die entsprechenden Bereiche zumindest teilweise bedeckende poröse Struktur oder ein Gewebe vorgesehen werden, bspw. ein Filzgewebe, expandiertes PTFE, Dacron oder ein anderes geeignetes biokompatibles synthetisches Stoffgewebe. Dies hat den Vorteil, dass die Perikardschleuse (3) an besagten Stellen besser mit dem umgebenden Körpergewebe verwächst und somit sowohl die mechanische Belastbarkeit als auch die Dichtung verbessert werden.

Eine alternative Ausführungsform kann ein weiteres Bauteil umfassen, eine Einführhilfe, welches rotationssymmetrisch sein kann und vorzugsweise aus einem steiferen und/oder glatteren Material, d.h. potentiell mit einer geringeren Reibung in Bezug zu einzuführenden Bauteilen, als die Perikardschleuse (3) besteht. Oben genannten Maßnahmen zur Einstellung der Reibung können auch für dieses Bauteil angewendet werden. Dieses Bauteil kann zur temporären Aufweitung und Stützung des Implantationskanals dienen sowie die Neigung der Perikardschleuse (3) hinsichtlich Zerknautschen und Knicken bei Berührung mit einzuführenden Ausführungsformen des Implantats vermindern. Nach der Implantation kann dieses Bauteil in der Perikardschleuse (3) verbleiben oder entfernt werden.

Figur 6a zeigt eine Ausführungsform der Perikardschleuse (3) während der Einführung in die Perikardhöhle (902). Die Implantation kann zwischen zwei Rippen hindurch erfolgen. Durch die hohe elastische Deformierbarkeit kann die Perikardschleuse (3) durch eine Öffnung (903) im Perikard (901) geführt werden, welche in ihrer größten Ausdehnung kleiner ist als der Durchmesser der Perikardschleuse (3) im unverformten Zustand.

Figur 6b zeigt die Lage der Perikardschleuse (3) nach der Einführung in die Perikardhöhle (902). Aufgrund des oben beschriebenen Größenverhältnisses zwischen Öffnung (903) im Perikard (901) und dem Schleusendurchmesser kann eine kraftschlüssige Verbindung zwischen Perikard (901) und der Perikardschleuse (3) entstehen. Diese Verbindung kann die Stabilität und die Positionstreue der Perikardschleuse (3) im Innern der Perikardhöhle (902) erhöhen. Der sich zur Dichtlippe (31) hin erweiternde Hals der Perikardschleuse (3) kann sich gegen das Perikard (901) abstützen und eine selbsthemmende Wirkung entfalten, wodurch die Stabilität und die Positionstreue der Perikardschleuse (3) im Innern der Perikardhöhle (902) erhöht werden kann. Die Dichtlippe (31) kann ebenfalls die Stabilität und Positionstreue der Perikardschleuse (3) im Innern der Perikardhöhle (902) erhöhen, da sie ein Bereich erhöhter Materialsteifigkeit sein und somit schwerer deformierbar sein kann.

Die Perikardschleuse kann nach Einbringung des Implantats zumindest teilweise im Körper verbleiben. Die Lamellen (32) der Perikardschleuse (3) können nach der Implantation des Implantats einer anderen Funktion dienen. Sie können der zusätzlichen Befestigung der Perikardschleuse (3) an der Fixierhülse dienen, etwa durch Umwickeln, Festbinden, Vernähen, Verkleben oder Verschweißen. Auch andere Befestigungsformen sind denkbar. Sie können nach der Implantation des Implantats auch funktionslos werden. Um die im Körper verbleibende exogene Materialmenge zu minimieren, können diese Lamellen (32) abgetrennt werden. Die Abtrennung der Lamellen (32) kann mittels einer Trennvorrichtung (33), bspw. einer Schere oder einer Zange, in einer für den Anwendungsfall adäquaten Trennebene (34) erfolgen. Die Abtrennung kann auch ohne Trennvorrichtung geschehen, wenn an der Perikardschleuse (3) am Ort der vorgesehenen Abtrennung in der Trennebene (34) bspw. Sollbruchstellen vorgesehen sind.

Alternative Ausführungsformen können eine mehrteilige Perikardschleuse (3) aufweisen. Solche Ausführungsformen können bspw. über ein Dichtelement (etwa in Form einer weiteren Dichtlippe) verfügen, welche an ein weiteres Teil gekoppelt werden kann, so dass beide Teile zusammen die Dichtwirkung erzielen. Der eine Teil der Dichtung kann sich hierbei im Innern der Perikardhöhle (902) befinden, das andere außerhalb. Die Kopplung beider Teile bewirkt, dass die Perikardöffhung (903) verschlossen werden kann und somit abgedichtet ist.

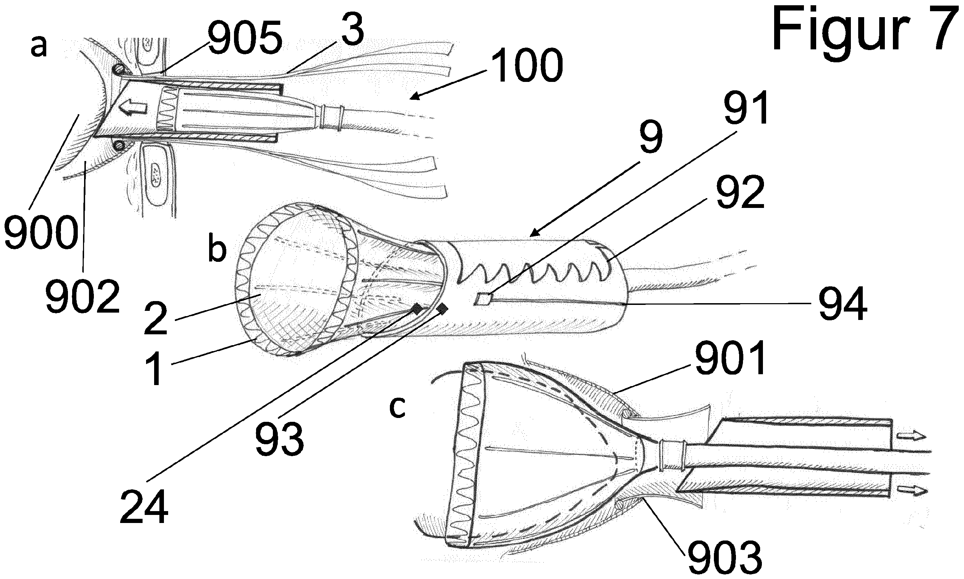

**Figur 7a, b und c** zeigen die Einbringung des Implantats (100) über ein Zuführsystem (9) in die Perikardhöhle (902). Figur 7a zeigt eine Lage des Zuführsystems (9) im Inneren der Perikardschleuse (3) knapp unterhalb der Herzspitze und teilweise innerhalb der Perikardhöhle (902).

Das Zuführsystem (9) kann ein im Wesentlichen rohrförmiges Zuführsystem (9) sein. Das Zuführsystem (9) hat eine proximale und eine distale Seite. Die proximale Seite des Zuführsystems (9) ist während der Implantation dem Operateur zugewandt, die distale Seite ist die dem Patienten zugewandt Seite.
In dieser Ausführungsform ist das Zuführsystem (9) als gerades, zylindrisches Rohr mit einem abgeschrägten distalen Ende dargestellt. Das Zuführsystem (9) kann auch von der geraden, zylindrischen Rohrform abweichen. Das Zuführsystem (9) kann rotationssymmetrisch bezüglich einer Bezugsgerade sein. Das Zuführsystem (9) kann auch von der Rotationssymmetrie bezüglich einer Bezugsgerade abweichen. Die Bezugsgerade kann auch die Vereinigungsmenge mehrerer Symmetrieebenen sein (Rohr mit polygonalem Querschnitt). Die Bezugsgerade kann auch die Menge der Schwerpunkte aller Querschnitte des Rohres sein (Rohr mit beliebigem Querschnitt). Die Bezugsachse kann auch eine Bezugslinie sein. Eine Bezugslinie kann gerade sein. Eine Bezugslinie kann auch krummlinig sein. Die Definition der Bezugslinie gibt Rückschluss auf die Rohrführung. In seiner Ausführungsform als gerades, zylindrisches Rohr kann das Zuführsystem (9) eine konstante Wanddicke aufweisen. Die Wanddicke des rohrförmigen Zuführsystems (9) kann auch veränderlich sein. Die Wanddicke kann in axialer Richtung und in Umfangsrichtung veränderlich sein. Die veränderliche Wanddicke kann dazu dienen, die Einbringen des Implantats (100) in das Zuführsystem (9), das Durchführen des Implantats (100) durch das Zuführsystem (9) oder das Ausbringen des Implantats (100) aus dem Zuführsystem (9) zu beeinflussen. Die veränderliche Wanddicke kann das Einbringen des Implantats (100) in das Zuführsystem (9), das Durchführen durch das Zuführsystem (9) oder das Ausbringen aus dem Zuführsystem (9) erleichtern. Eine veränderliche Wanddicke im Falle einer Ausführungsform in Form eines zylindrischen Rohres kann zur Folge haben, dass sich der Innendurchmesser des Rohres verändern kann. Dies kann das Einbringen des Implantats (100) in das Zuführsystem (9), das Durchführen durch das Zuführsystem (9) oder das Ausbringen aus dem Zuführsystem (9) erleichtern. Eine veränderliche Wanddicke im Falle einer Ausführungsform in Form eines zylindrischen Rohres kann auch zur Folge haben, dass sich der Außendurchmesser des Rohres verändern kann. Eine veränderliche Wanddicke im Falle einer Ausführungsform in Form eines zylindrischen Rohres kann auch zur Folge haben, dass sich Innen- und Außendurchmesser des Rohres verändern können. Dadurch lassen sich die Folgen beider Durchmesserveränderungen kombinieren. In der vorliegenden Ausführungsform verringert sich die Wanddicke linear zu dem Ende hin, an welchem der Austritt des Implantats (100) vorgesehen ist. Dabei bleibt der Außendurchmesser der dargestellten Ausführungsform konstant, der Innendurchmesser vergrößert sich zum Austritt hin und weist eine trichterförmige Geometrie auf.

Das Implantat (100) kann in ein Zuführsystem (9) eingebracht werden. Die Einbringung des Implantats (100) in das Zuführsystem (9) kann durch eine radiale Komprimierung des Implantats (100) geschehen. Der Komplex aus Zuführsystem (9) und erfindungsgemäßer Vorrichtung kann durch eine Perikardschleuse (3), welche sich teils innerhalb der Perikardhöhle (902), teils außerhalb des Perikards, aber innerhalb des Köpers und teils außerhalb des Körpers befindet, an das Herz (900) herangeführt werden.

In einer Ausführungsform, in der die Rahmenstruktur (1) aus einem elastisch komprimierbaren oder selbst-expandierendem Material besteht, kann sich das Implantat (100) aufgrund der beim Einbringen in das Zuführsystem (9), im Falle eines elastisch komprimierten Materials, gespeicherten elastischen Energie oder alternativ, im Fall einer Formgedächtnislegierung, aufgrund der veränderten Temperatur im Körperinneren beim Ausbringen aus dem Zuführsystem (9) expandieren und das Herz (900) zumindest teilweise umschließen.

Figur 7b zeigt das Zuführsystem (9) und eine Ausführungsform des Implantats (100), welche teilweise aus dem Zuführsystem (9) ausgetreten und in einer Expansion begriffen ist. An der Oberfläche des Zuführsystems (9) können zumindest ein Sensor (91) und zumindest ein Element zur Beeinflussung zumindest eines Parameters in oder am Zuführsystem (9), bspw. eine Heizwendel (92), angebracht sein.

Der zumindest eine Sensor (91) kann an der Innenfläche oder an der Außenfläche des Zuführsystems (9) angebracht sein. Alternativ kann der zumindest eine Sensor (91) in die Wand des Zuführsystems (9) eingearbeitet sein. Der zumindest eine Sensor (91) kann der Erfassung verschiedener Parameter im Zuführsystem (9) oder der Umgebung dienen. Der Sensor (91) kann ein Temperatursensor, ein Drucksensor, ein pH-Sensor ein Sauerstoffsensor, ein CO₂-Sensor, ein optischer Sensor oder ein Leitfähigkeitssensor sein. Die elektrische Leitung (94) kann den Sensor mit einer Auswertevorrichtung verbinden. Der zumindest eine Sensor kann alternativ auch kabellos betrieben werden.

In der vorliegenden Ausführungsform ist das zumindest eine Element zur Beeinflussung zumindest eines Parameters in oder am Zuführsystem (9) als Heizwendel (92) ausgeführt. Durch die Heizwendel (92) kann bspw. das Expansionsverhalten der selbst-expandierenden Rahmenstruktur (1) eingestellt werden. Das zumindest eine Element zur Beeinflussung zumindest eines Parameters kann an der Innenfläche des Zuführsystems (9) angebracht sein. Das zumindest eine Element zur Beeinflussung zumindest eines Parameters kann auch an der Außenfläche des Zuführsystems (9) angebracht oder in die Wand des Zuführsystems (9) eingearbeitet sein. Beeinflussbare Parameter können die Temperatur im Inneren und/oder der Umgebung des Zuführsystems (9), die Konzentration von Leitfähigkeits-beeinflussenden Stoffen, den pH-Wert beeinflussenden Stoffen, sowie den Sauerstoff- und CO₂-Gehalt beeinflussenden Stoffen im Inneren und/oder der Umgebung des Zuführsystems (9) steuern. Ein weiterer Parameter kann die Haft- und Gleitreibung zwischen Implantat (100) und Zuführsystem (9) sein, welche durch geeignete Zusätze wie etwa Gleitmittel eingestellt werden kann. Ist die Zuführung bspw. elektrischer Energie für den Betrieb des zumindest einen Elements (94) zur Beeinflussung zumindest eines Parameters in oder am Zuführsystem (9) vonnöten, so kann diese mittels der elektrischen Leitung (94) geschehen.

Die Stirnflächen eines Zuführsystems (9) können an zumindest einem Ende des Rohres von einer planaren Form abweichen. Die Stirnflächen können ein räumlich verlaufendes Flächenband beschreiben. Die Stirnflächen können auch planar sein, aber gegen die Mittelachse des Zuführsystems (9) verkippt sein, d.h. die Stirnflächennormale und die Tangente der Bezugslinie des Zuführsystems (9) im Stirnflächenschwerpunkt schließen einen Winkel ein, der betragsmäßig größer oder gleich Null ist. Vorzugsweise ist die distale Stirnfläche des Zuführsystems (9) abgeschrägt, was das Einsetzen des Implantats (100) erleichtert. Die Veränderung der Form und der Orientierung zumindest einer Stirnfläche des Zuführsystems (9) gegenüber einer Ausführungsform mit planen Stirnflächen, deren Normalen parallel oder antiparallel zur Tangente der Bezugslinie des Zuführsystems (9) im Stirnflächenschwerpunkt der Stirnfläche sind, kann die Einbringung des Zuführsystems (9) in die Perikardhöhle (902) durch die Perikardschleuse (3), die Expansion der Rahmenstruktur (1) und die Umschließung eines Herzes (900) durch die Rahmenstruktur (1) und die Hülle (2) erleichtern und/oder verbessern, da anatomische Gegebenheiten wie die Lage der Rippen und des Herzes (900) den operativen Zugang zum Herz (900) unter einem bestimmten Winkel zur Herzlängsachse erfordern können. Die Abschrägung gegenüber der Flächennormalen einer Ausführungsform mit planer Stirnfläche kann zwischen 30° und 90° liegen, sie kann zwischen 45° und 80° liegen.

Das Zuführsystem (9) kann zumindest teilweise transparent ausgeführt sein. Der Vorteil liegt darin, dass die in das Zuführsystem (9) eingebrachte Ausführungsform des Implantats (100) mithilfe entsprechender Markierungen (24, 93) in Bezug auf das Zuführsystem (9) ausgerichtet werden und somit eine zielgenaue Zuführung in den Körper und die Einbringung in die Perikardhöhle (902) gewährleistet werden kann. Diese Markierungen (24, 93) können röntgenopak sein.

Figur 7c zeigt eine Ausführungsform des Implantats (100), welche nach der Ausbringung aus dem Zuführsystem (9) durch die Perikardschleuse (3) zumindest teilweise ein Herz (900) umschließt. Die Perikardschleuse (3) kann, wie in einem vorangegangenen Abschnitt beschrieben, zum Zwecke der Abdichtung und Versiegelung der Öffnung (903) im Perikard (901) im Körper verbleiben. Das Zuführsystem (9) kann nach der Ausbringung des Implantats (100) durch die Perikardschleuse (3) hindurch wieder aus dem Körperinneren entfernt werden.

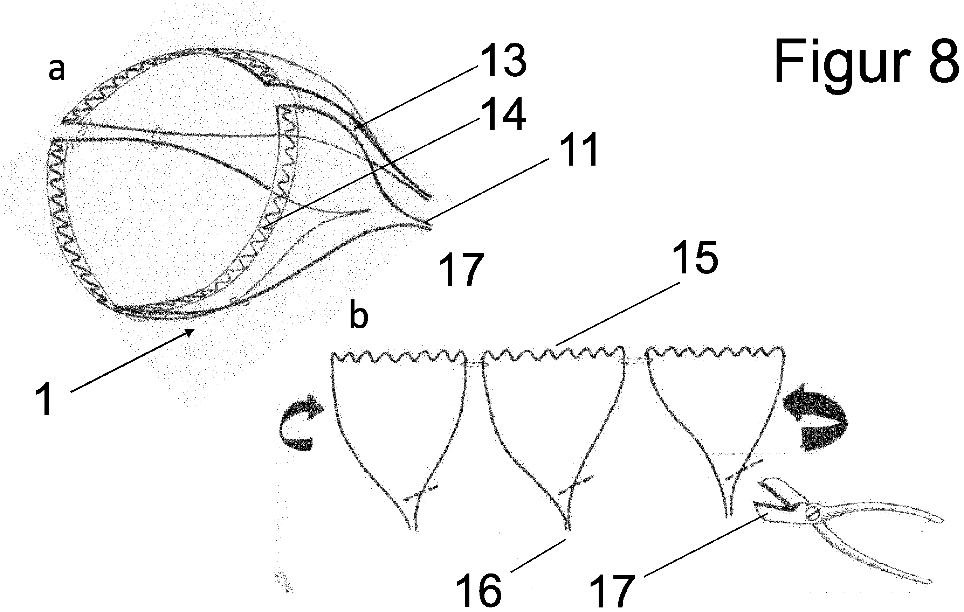

**Figur 8a** **und b** zeigen eine Ausführungsform der Rahmenstruktur (1), welche dem Implantat im allgemeinen und einer in die Rahmenstruktur (1) eingebrachten Hülle Form und Stabilität verleihen kann und welche die Expansion des Implantats nach der Ausbringung aus einem Zuführsystem in die Perikardhöhle gewährleisten kann.

Figur 8a zeigt die räumliche Ansicht einer Ausführungsform der Rahmenstruktur (1) des Implantats. Die Rahmenstruktur (1) in dieser Ausführungsform ist als Drahtgestell aus drei Drähten (14) dargestellt.

Die Rahmenstruktur (1) kann in Form eines Gitterwerks ausgeführt sein. Das Gitterwerk kann aus einem durchgängigen Material bestehen aus dem Teile entfernt wurden oder in welches die Öffnungen hineingeformt wurden. Beispielsweise kann die Rahmenstruktur (1) aus einem Rohr oder einem individuell geformten Rahmenstrukturmantel gefertigt werden, in welche die Öffnungen geschnitten werden. Bevorzugt wird dabei ein Verfahren in dem kleine Schlitze, bspw. mittels Laserschneiden, in ein Rohr geschnitten werden ("slotted tube"). Das geschlitzte Rohr kann einen Durchmesser von 4 mm bis 30 mm haben, es kann einen Durchmesser von 6 mm bis 20 mm haben. Das geschlitzte Rohr kann zwischen 5 cm und 20 cm lang sein, es kann zwischen 8 cm und 15 cm lang sein.

Ein geschlitztes Rohr, welches aus einer Formgedächtnislegierung besteht, wird dann über formgebende Dorne aufgespannt und mittels einer Wärmebehandlung normalgeglüht, so dass die aufgespannte Form die neue natürliche Konfiguration des Rohres darstellt. Diese Aufspann- und Wärmebehandlungsvorgänge können so oft mit immer größer werdenden Dornen wiederholt werden, bis zuletzt das geschlitzte Rohr über eine Form gespannt werden kann, die einem Abbild des Herzens entspricht und womit in der Wärmebehandlung die Herzform auf das geschlitzte Rohr übertragen wird. Die Herstellung der Dorne wird in einem nachfolgenden Abschnitt näher beschrieben. Das geschlitzte Rohr wird somit in eine aus einem Gitterwerk bestehende Rahmenstruktur (1) überführt, deren Form einem Abbild des Herzens entspricht. Die Schlitze im Rohr gehen dabei über in die Öffnungen des Gitterwerks. Die Öffnungen werden dabei durch Streben (14) begrenzt. Mehrere Streben (14) einer Öffnung bilden dabei einzelne Zellen des Gitterwerks. Das Gitterwerk kann dabei bevorzugt rautenförmige Zellen aufweisen. Die Form der Zellen kann durch geeignete Schnittführung beim Schlitzen des Rohres modifiziert werden. Alternativ können so Zellen mit einer hexagonalen Wabenstruktur oder Zellen einer polygonalen Struktur erzeugt werden.

Durch geeignete Schnittführung können längere oder kürzere Zellen hergestellt werden. Durch die Länge und Anzahl der Schlitze wird auch die Anzahl der Zellreihen entlang der Herzlängsachse der Rahmenstruktur (1) definiert. Längere Schnitte führen zu längeren Streben (14), kürzere Schnitte zu kürzeren Streben (14) in der Rahmenstruktur (1). Längere Streben (14) sind dabei biegsamer als kurze Streben (14). Die Länge der Zellen beträgt 5 mm bis 50 mm, insbesondere 10 mm bis 30 mm. Die Länge der Zellen kann von Zellreihe zu Zellreihe variieren und auch innerhalb einer Zellreihe gleich sein oder variieren.

Über die Anzahl der Schlitze entlang des Umfangs des Rohres wird die Anzahl der Zellen entlang des Umfangs der Rahmenstruktur (1) bestimmt. Weniger Schlitze entlang des Umfangs führen zu einer größeren Breite der resultierenden Streben (14), eine Erhöhung der Schlitzanzahl entlang des Umfangs führt zu dünneren Streben (14). Breitere Streben (14) sind dabei weniger flexibel als dünnere Streben (14). In Längsrichtung können zwischen 4 und 15 Schnitte in einer Reihe hintereinander angebracht werden, es können zwischen 5 und 10 Schnitte in einer Reihe hintereinander angebracht werden. In Umfangsrichtung können 14 bis 80 solcher Schnittreihen über den Rohrumfang verteilt werden, es können 20 bis 45 solche Schnittreihen entlang des Umfangs verteilt werden, es können 26 bis 36 solcher Schnittreihen entlang des Umfangs verteilt werden. Bei der Herstellung können die Schnitte gleichmäßig über den Rohrumfang (äquidistant) verteilt werden, womit eine homogene Struktursteifigkeit im Gitterwerk erreicht wird. Alternativ können die Schnitte ungleichmäßig (nicht äquidistant) zueinander verteilt werden, so dass Bereiche mit Zellen mit größerer Strebenbreite (höhere Struktursteifigkeit) und Bereiche mit Zellen geringerer Strebenbreite (niedrigere Struktursteifigkeit) erzeugt werden können. Bereiche in denen eine höhere Struktursteifigkeit erwünscht ist, können beispielweise Bereiche sein die den linken Ventrikel des Herzens abdecken oder Bereiche die das Widerlager für die expandierbaren Einheiten bilden. Bereiche in denen eine geringere Struktursteifigkeit erwünscht ist, sind beispielweise Bereiche am rechten Ventrikel.

Die Wandstärke des Rohres in welches die Schlitze geschnitten werden resultiert in die Strebenhöhe der Rahmenstruktur (1). Eine größere Strebenhöhe führt zu einem steiferen Verhalten des Gitterwerks. Eine geringere Strebenhöhe führt zu einem flexibleren Verhalten des Gitterwerks. Durch die Verwendung eines Rohres mit regional unterschiedlicher Wandstärke können somit Bereiche geringerer oder höherer Struktursteifigkeit erzeugt werden. Die Wandstärke des Rohres kann zwischen 0,2 mm und 2 mm betragen, bevorzugt zwischen 0,4 mm und 1,5 mm, insbesondere zwischen 0,6 mm und 1 mm. Die Wandstärke der Rahmenstruktur (1) ist gleich der Wandstärke des Rohres.

Bei gleicher Schlitzanzahl resultiert ein größerer Rohrdurchmesser in einer größeren Strebenbreite. Das Herstellungsverfahren garantiert, dass das Gitterwerk der expandierten Rahmenstruktur (1) auch wieder auf die Größe des Rohrs komprimiert werden kann aus dem es geschnitten wurde. Dies kann insbesondere dann hilfreich sein, wenn die Rahmenstruktur (1) in ein zuvor beschriebenes Zuführsystem eingebracht werden soll. Die Rahmenstruktur (1) kann aber auch auf jede Zwischengröße zwischen dem vollständig expandierten, herzförmigen Gitterwerk und dem ursprünglichen Rohrdurchmesser komprimiert werden. Durch die Verwendung z.B. einer Formgedächtnislegierung kann die Rahmenstruktur (1) beim Implantieren/beim Ausbringen aus dem Zuführsystem von selbst in die Herzform expandieren. Das Aufbringen von externen Kräften durch weitere Vorrichtungen ist bei der Verwendung einer Formgedächtnislegierung nicht erforderlich. Insbesondere die Länge der Zellen und/oder Streben (14) des Gitterwerks bestimmt dabei den Öffhungswinkel bei der Expansion der Rahmenstruktur (1), insbesondere bei der selbst-expandierbaren Rahmenstruktur (1).

Die Rahmenstruktur (1) kann anstelle eines Gitterwerkes auch aus einem Geflecht von Drähten (14) bestehen. Die Drähte (14) formen Kreuzungspunkte, die miteinander fest verbunden werden können. Beispielsweise können die Drähte (14) an den Kreuzungspunkten miteinander verschweißt werden. Das Verbinden der Drähte (14) an Kreuzungspunkten erhöht die Stabilität der Rahmenstruktur (1). Die Kreuzungspunkte können auch nicht miteinander verbunden werden. Dies kann die Flexibilität der Rahmenstruktur (1) erhöhen und damit zu einer leichteren Komprimierbarkeit der Rahmenstruktur (1) führen. Dies kann insbesondere dann hilfreich sein, wenn die Rahmenstruktur (1) in ein Zuführsystem mit einem Katheter kleineren Durchmessers eingebracht werden soll. Die Rahmenstruktur (1) kann auch an einigen der Kreuzungspunkte fest miteinander verbunden sein und an anderen Kreuzungspunkten nicht fest miteinander verbunden sein. Durch eine geeignete Wahl von Kreuzungspunkten, die fest miteinander verbunden werden und Kreuzungspunkten, die nicht fest miteinander verbunden werden, kann die Stabilität und Flexibilität der Rahmenstruktur (1) angepasst werden. Bereiche, die im implantierten Zustand eine erhöhte Stabilität erfordern, können durch das Verbinden der Drähte (14) an Kreuzungspunkten stabilisiert werden. Dies können bspw. Bereiche sein, die als Widerlager für expandierbare Einheiten dienen. Solche Widerlager können sich direkt unter einer expandierbaren Einheit befinden oder neben Bereichen mit expandierbaren Einheiten. Bereiche, die eine erhöhte Flexibilität erfordern, können Bereiche sein, die beim Einbringen in ein Zuführsystem stärker komprimiert werden müssen als andere Bereiche. Bereiche, die eine erhöhte Flexibilität erfordern können auch Bereiche sein, in denen eine erhöhte Flexibilität die natürliche Bewegung des Herzens unterstützt.

Anpassungen können auch herbeigeführt werden durch die Wahl des verwendeten Materials, durch Veränderungen des Materials an bestimmten Bereichen durch Einwirkung von z.B. energetischer Strahlung, wie etwa Wärme. Vorzugsweise weist die Rahmenstruktur (1) Öffnungen auf, die von den Drähten (14) eines Drahtgeflechtes, den Streben (14) eines Gitterwerkes oder den Löchern in einem Rahmenstrukturmantel geformt werden. Diese Öffnungen ermöglichen das Komprimieren der Rahmenstruktur (1), sie erlauben den Stoffaustausch vom Inneren der Rahmenstruktur (1) mit Bereichen außerhalb der Rahmenstruktur (1) und umgekehrt, sie verringern die Menge des einzusetzenden Materials für die Rahmenstruktur (1) und sie erlauben eine erhöhte Flexibilität der Rahmenstruktur (1). Formen, die mit durchgehenden Materialien nur schwer zu verwirklichen sind, sind mit geflechtartigen oder gitterartigen Strukturen leichter zu formen. Die Öffnungen können viereckig, rautenförmig, rund oder oval sein. Die durch die Drähte (14), die Streben (14) oder den Löchern in einem Rahmenstrukturmantel definierten Öffnungen haben einen Durchmesser von ungefähr 1 mm bis 50 mm, vorzugsweise von 3 mm bis 30 mm, vorzugsweise von 5 mm bis 20 mm. Der Durchmesser einer Öffnung ist als Pin-Öffnung definiert, das heißt der Durchmesser der Öffnung stellt den größten Durchmesser eines zylindrischen Stiftes dar, der durch eine Öffnung (eine Zelle, ein Loch) hindurchgeführt werden kann.

Die Rahmenstruktur (1) besteht vorzugsweise aus einem Material, das eine Expansion ermöglicht. Vorzugsweise ist die Rahmenstruktur (1) aus einem Material geformt, das aus der Gruppe bestehend aus Nitinol, Titan und Titanlegierungen, Tantal und Tantallegierungen, Edelstahl, Polyamid, Polymerfaser-Werkstoffe, Karbonfaserwerkstoffe, Aramidfaserwerkstoffe, Glasfaserwerkstoffe und Kombinationen daraus ausgewählt ist. Für eine selbstexpandierende Rahmenstruktur (1) eignet sich ein Material, das zumindest zum Teil aus einer Formgedächtnislegierung gefertigt ist. Werkstoffe für Formgedächtnislegierungen sind bspw. NiTi (Nickel-Titan; Nitinol), NiTiCu (Nickel-Titan-Kupfer), CuZn (Kupfer-Zink), CuZnAl (Kupfer-Zink-Aluminium), CuAINi (Kupfer-Aluminium-Nickel), FeNiAl (Eisen-Nickel-Aluminium) und FeMnSi (Eisen-Mangan-Silizium).

Die Rahmenstruktur (1) kann auch aus einem Kunststoff, einem Verbundmaterialien, oder einem Polymer bestehen, welche die für eine Expansion unter den beschriebenen Bedingungen ausreichende Steifigkeit aufweisen. Beispielsweise kann die Rahmenstruktur (1) aus Polyethylen gefertigt sein und wird vorzugsweise durch ein Gießverfahren, bspw. Spritzguss, hergestellt. Auch andere Kunststoffe, Faserverwerkstoffe oder Verbundmaterialien sind denkbar.

Die Rahmenstruktur (1) weist vorzugsweise eine an die individuelle Herzform des Patienten angepasste Form auf. Die individuelle Herzform der Patienten kann dabei aus CT- oder MRT-Bilddaten rekonstruiert werden. Die Rahmenstruktur (1) ist am vom Operateur aus gesehen distalen, oberen Ende (15) offen. Der obere, distale Rand der Rahmenstruktur (1) weist vorzugsweise Schlaufen eines Drahtes oder Bügel, die von Streben (14) geformt sind, auf. Die Schlaufen oder Bügel können als Verankerungsstellen für eine Hülle mit zumindest einer expandierbaren Einheit dienen. Am vom Operateur aus gesehen proximalen, unteren Ende der Rahmenstruktur (1) befindet sich vorzugsweise eine Öffnung (16), durch welche eine oder mehrere elektrische Leitungen des zumindest einen Sensors oder der zumindest einen Elektrode und/oder Leitungen der zumindest einen expandierbaren Einheit durchgeführt werden können. Die Form der Rahmenstruktur (1) stellt zumindest zum Teil die Form eines natürlichen Herzens dar, vorzugsweise dem unteren Teil eines Herzens. Einzelheiten zur Form der Rahmenstruktur (1) werden in einem späteren Abschnitt der Beschreibung näher erläutert.

Bei zwei oder mehr Streben (14) können die zumindest zwei Streben (14) von gleicher Länge sein, können jedoch auch von unterschiedlicher Länge sein. Der Querschnitt der zumindest einen Strebe (14) kann rechteckig sein. Der Querschnitt der Strebe (14) kann auch von der Rechteckform abweichen, bspw. kann er auch rund, elliptisch oder polygonal sein. Das zumindest eine Teil der Rahmenstruktur (1) kann aus mehreren Drähten (14) bestehen, die jeweils paarweise gekoppelt sein können. Es kann auch zumindest ein koppelbares Paar ungekoppelt ausgeführt werden. Alternativ kann der zumindest eine Teil der Rahmenstruktur (1) aus zumindest einer Strebe (14) bestehen. Der zumindest eine Teil der Rahmenstruktur (1) kann auch aus mehreren Streben (14) bestehen, die jeweils paarweise gekoppelt sein können. Es kann auch zumindest ein koppelbares Paar ungekoppelt ausgeführt werden. Der zumindest eine Draht (14) oder die zumindest eine Strebe (14) können so gekoppelt werden, dass unterschiedliche Segmente entlang des zumindest einen Drahtes oder der zumindest einen Strebe (14) miteinander verbunden werden.

Im vorliegenden Ausführungsbeispiel sind die drei geformten Drähte (14) mit dem jeweiligen Nachbardraht durch jeweils zwei Kopplungen (13) verbunden. Eine Kopplung (13) kann durch Stoffschluss, durch Formschluss und/oder durch Kraftschluss erfolgen. Wird die Kopplung (13) als Stoffschluss ausgeführt, können die Koppelpartner untereinander bspw. verschweißt oder verklebt werden. Wird die Kopplung (13) als Formschluss ausgeführt, kann dies bspw. durch komplementäre Koppelelemente (13) an den zumindest zwei zu verbindenden Draht- oder Strebensegmenten geschehen. Dabei können die Koppelelemente (13) Teil der zumindest zwei Draht- oder Strebensegmente sein, bspw. in Form von Haken, Ösen, Knöpfen, Schlaufen bzw. deren komplementärer Kombination für das jeweilige zu koppelnde Drahtsegment- oder Strebensegmentpaar. Alternativ lassen sich die Verbindungspartner, wenn als Drähte (14) ausgeführt, auch verflechten oder verdrillen. Die Koppelelemente (13) können auch zumindest ein individuelles Bauteil sein, bspw. in Form von Ringen, Hülsen, Schellen, Drähten oder Klammern. Die formschlüssige Kopplung (13) kann auch durch zumindest einen Klett- und/oder Reißverschluss erfolgen. Wird die Kopplung (13) durch Kraftschluss erreicht, können die Enden der zumindest zwei Draht- oder Strebensegmente durch Fäden, Fasern oder Seile verknotet werden. Die Enden des zumindest einen Drahtes oder der zumindest einen Strebe (14) können am unteren Ende des Implantats zusammengeführt werden. Diese Enden können miteinander gekoppelt werden. Die Enden können aber auch ungekoppelt verbleiben. Es können die beiden Enden des zumindest einen Drahtes gekoppelt werden. Bei zumindest drei Drähten (14) kann auch ein Ende des einen mit dem einen Ende des benachbarten Drahtes (14) verbunden werden. Die Enden des zumindest einen Drahtes (14) oder der zumindest einen Strebe (14) können der axialen Versteifung (parallel zur Herzlängsachse) und der axial ortsfesten Positionierung des Implantats dienen. Die axial ortsfeste Positionierung wird dadurch erreicht, dass die Enden des zumindest einen Drahtes oder der zumindest einen Strebe (14) am oder in der Fixierhülse befestigt werden, welcher von der vorliegenden Ausführungsform des Implantats weg zu der Versorgungseinheit führt. Diese Verbindung kann durch Form-, Kraft- oder Stoffschluss zustande kommen. Im Falle einer formschlüssigen Verbindung kann die Verbindung als Kopplung (13) zweier komplementärer Verbindungspartner ausgeführt werden, in Form einer Verbindung mit Passfeder, in Form einer Haken-Öse-Verbindung, einer Knopf-Schlaufe-Verbindung oder einer Haken-Schlaufe-Verbindung. Im Falle einer kraftschlüssigen Verbindung kann diese Verbindung in Form einer Klemmverbindung oder mithilfe eines Gewindestifts ausgeführt werden. Im Falle einer stoffschlüssigen Verbindung kann diese Verbindung in Form einer Klebe- oder Schweißverbindung ausgeführt sein.

Durch die Anzahl an Drähten oder Streben (14) lässt sich die Steifigkeit der Rahmenstruktur (1) in axialer, radialer und Umfangsrichtung einstellen. Mit steigender Anzahl eingesetzter Drähte (14) oder Streben (14) steigt die Steifigkeit der Rahmenstruktur (1). Alternativ lässt sich die Steifigkeit der Rahmenstruktur (1) durch die Anzahl der paarweisen Kopplungen von zumindest zwei Drähten (14) oder Streben (14) einstellen. Mit steigender Anzahl an Kopplungen (13) steigt die Steifigkeit der Rahmenstruktur (1). Die Steifigkeit der Rahmenstruktur (1) kann auch über den Querschnitt des zumindest einen Drahtes oder den Querschnitt der zumindest einen Strebe (14) eingestellt werden. Eine größere Querschnittsfläche kann zu einer erhöhten Steifigkeit der Rahmenstruktur (1) führen. Die Stärke des zumindest einen Drahts oder der zumindest einen Strebe (14) kann zwischen 0.2 mm und 2 mm liegen. Die Stärke des zumindest einen Drahts oder der zumindest einen Strebe (14) kann zwischen 0.4 mm und 1 mm liegen. In Umfangsrichtung kann die Steifigkeit der Rahmenstruktur (1) über eine alternierende Führung des zumindest einen Drahtes oder der zumindest einen Strebe (14) eingestellt werden. Die alternierende Führung kann periodisch sein. Sie kann aber auch von einer periodischen Form abweichen. Die für die Einstellung der Steifigkeit eine Rolle spielenden Parameter sind die Amplitude der alternierenden Führung in transversaler Richtung sowie die Anzahl der Maxima und Minima der Draht- oder Strebenführung. Im Falle einer periodischen Draht- oder Strebenführung spiegelt sich die Anzahl der Perioden der Draht- oder Strebenführung in der Periodenlänge wider, wobei eine Periode ein Maximum und ein Minimum aufweist. Im Falle einer periodischen Draht- oder Strebenführung kann eine Ausführung in Sinusform, in Dreiecksform, in Rechteckform, in polygonaler Form, in abschnittsweise Halbkreisform oder in einer Kombination dieser Formen realisiert werden. Die Kombination der oben genannten Formen kann auch zu einer über den Gesamtumfang gesehen aperiodischen Draht- oder Strebenführung führen. Die Draht- oder Strebenführung kann bei einer aus zumindest zwei Teilen bestehenden Ausführungsform der Rahmenstruktur (1) je Teil gleich sein oder zwischen den Teilen unterschiedlich sein. Die Amplitude der Draht- oder Strebenführung beeinflusst die Steifigkeit der Rahmenstruktur (1) vornehmlich, aber nicht ausschließlich in Umfangs- und in radialer Richtung. Ein die axiale Steifigkeit beeinflussender Effekt kann auch erzielt werden. Anhand der Amplitude der Draht- oder Strebenführung kann Einfluss auf das Expansions- und Kompressionsverhalten der expandierbaren Rahmenstruktur (1) genommen werden. Die Amplitude der Draht- oder Strebenführung kann zwischen 0 mm und der halben Gesamthöhe der Rahmenstruktur (1) (Abstand zwischen oberem und unterem Ende der Rahmenstruktur (1)) liegen. Die Amplitude kann zwischen 4 mm und 10 mm liegen. Die Anzahl der Perioden der Draht- oder Strebenführung entlang des Umfangs kann ebenfalls zur Einflussnahme auf die Steifigkeit der Rahmenstruktur (1) vornehmlich, aber nicht ausschließlich in radialer und Umfangsrichtung genutzt werden. Ein die axiale Steifigkeit beeinflussender Effekt kann auch erzielt werden. Die Anzahl der Perioden der Draht- oder Strebenführung entlang des Umfangs kann zwischen 0 und 72 Perioden liegen, sie kann auch zwischen 12 und 36 Perioden liegen. Alle im Rahmen der Beschreibung der Rahmenstruktur (1) genannten Möglichkeiten zur Beeinflussung der axialen, radialen und der Umfangssteifigkeit können auch in Kombination verwendet werden.

Bei Ausführungsformen der Rahmenstruktur (1) aus zumindest einem Draht kann zusätzlich zumindest eine Strebe (14) eingesetzt und mit dem zumindest einen Draht (14) gekoppelt werden, was die Stabilität und Steifigkeit der Rahmenstruktur (1) zusätzlich erhöhen kann. Bei alternativen Ausführungsformen der Rahmenstruktur (1) aus zumindest einer Strebe (14) kann zusätzlich zumindest ein Draht (14) eingesetzt und mit der zumindest einen Strebe (14) gekoppelt werden, was die Stabilität und Steifigkeit der Rahmenstruktur (1) zusätzlich erhöhen kann. Die axiale, radiale und die Umfangssteifigkeit der Rahmenstruktur (1) kann auch durch die gezielte Kombination von Drähten (14) und Streben (14) eingestellt werden.

Das Material des zumindest einen Drahts oder der zumindest einen Strebe (14) kann der Rahmenstruktur (1) den Übergang von einem nicht expandierten Zustand in einen expandierten Zustand ermöglichen. Das Material des zumindest einen Drahts oder der zumindest einen Strebe (14) kann eine Formgedächtnislegierung sein. Das Material des zumindest einen Drahts oder der zumindest einen Strebe (14) der Rahmenstruktur (1) kann auch von einer Formgedächtnislegierung abweichen. Das Material kann auch ein Federstahl oder ein Polymer sein. Das Polymer kann Polyurethan (PU), Polypropylen (PP), Polyethylen (PE), Polycarbonat (PC), Polyamid (PA), ein superelastisches Polymer oder ein Formgedächtnispolymer sein. Die Rahmenstruktur (1) kann auch aus einem bioabbaubaren Material, bspw. Polylactid, Cellulose oder Polyglycolid, bestehen, da dies den Vorteil haben kann, dass eine Ausführungsform des Implantats ohne Kabelbaum und Versorgungseinheit, welche insgesamt aus zumindest einem bioabbaubaren Material besteht, nach Funktionserfüllung nicht mehr explantiert werden muss. Im Falle von Ausführungsformen des Implantats, bei welchen nur die Rahmenstruktur (1) aus zumindest einem bioabbaubaren Material besteht, kann dies vorteilhaft sein, da bspw. das Verletzungsrisiko des umliegenden Gewebes durch die Rahmenstruktur (1) verringert wird und auch der Explantationsvorgang ohne Rahmenstruktur (1) erheblich einfacher sein kann. Eine solche Rahmenstruktur (1) wird vom Körper mit der Zeit abgebaut. Eine Rahmenstruktur (1) aus einem Polymer kann aus Streben (14) bestehen, welche sich vom expandierten Zustand ausgehend elastisch verformen lassen, um die Rahmenstruktur (1) zu komprimieren und in ein Zuführsystem einzubringen. Bei der Ausbringung in die Perikardhöhle gelangt die Rahmenstruktur (1) wieder in den expandierten Zustand zurück. Die Expansion und Kompression der Ausführung einer aus einem Polymer bestehenden Rahmenstruktur (1) kann mithilfe eines Formgedächtniseffekts oder ohne die Ausnutzung eines Formgedächtniseffekts durchgeführt werden. Mit steigender Steifigkeit kann die Eignung der Rahmenstruktur (1) als Widerlager, etwa für die zumindest eine expandierbare Einheit, steigen. Bei niedriger Steifigkeit kann wenig Material verwendet werden für die Rahmenstruktur (1). Die Steifigkeit kann ausreichend sein, um der Hülle Position und Form zu geben. Mit einer niedrigen, aber ausreichenden Steifigkeit der Rahmenstruktur (1) kann sich das Gewicht des Implantats verringern lassen. Mit einer niedrigen, aber ausreichenden Steifigkeit der Rahmenstruktur (1) kann die Einbringung in ein Zuführsystem erleichtert werden. Die Rahmenstruktur (1) kann auch vorwiegend der Positionierung und Formgebung einer Hülle und weniger als Widerlager dienen.

Die Rahmenstruktur (1) kann zumindest eine Aussparung aufweisen, welche sicherstellt, dass anatomische Strukturen nicht räumlich beeinträchtigt werden, bspw. die Vena cava inferior. Die Aussparung kann derart erzeugt werden, dass die umfängliche Draht- oder Strebenführung eine Aussparung im Bereich der anatomischen Struktur vorsieht, welche nicht zusammengedrückt werden soll.

Es kann auch zumindest ein Draht (14) oder zumindest eine Strebe (14) in Umfangsrichtung verlaufen. Der zumindest eine Draht (14) oder die zumindest eine Strebe (14) in Umfangsrichtung kann die Stabilität der Rahmenstruktur (1) erhöhen. Der zumindest eine Draht (14) oder die zumindest eine Strebe in Umfangsrichtung kann die Steifigkeit in Umfangsrichtung erhöhen. Die Anzahl der Drähte (14) oder Streben in Umfangsrichtung kann zwischen 0 und 10 liegen. Sie kann zwischen 0 und 3 liegen. Es kann auch zumindest ein Draht (14) oder zumindest eine Strebe eine teilweise Axialausrichtung und eine teilweise Ausrichtung in Umfangsrichtung haben. Der zumindest eine Draht (14) oder die zumindest eine Strebe (14) in Umfangsrichtung kann die Stabilität der Rahmenstruktur (1) erhöhen. Der zumindest eine Draht (14) oder die zumindest eine Strebe (14) in Umfangsrichtung kann die Steifigkeit sowohl in axialer Richtung als auch in Umfangsrichtung erhöhen.

Figur 8b zeigt die zweidimensionale Abrollung der Rahmenstruktur (1). In der vorliegenden Ausführungsform besteht die abgerollte Rahmenstruktur (1) aus drei Drähten (14), wobei jeweils deren zwei Enden zum unteren Ende der Rahmenstruktur (1) zusammengeführt sind. Dort können sie mithilfe einer Kopplung (13) verbunden werden. Mögliche Verbindungen können eine Klebeverbindung, eine Schweißverbindung, eine kraftschlüssige Verbindung wie z.B. Verschnüren, oder eine formschlüssige Verbindung mit Haken, Ösen, Knöpfen oder Schlaufen sein. Zumindest ein Ende des zumindest einen Drahtes (14) oder der zumindest einen Strebe (14) der Rahmenstruktur (1) kann nach- oder vorträglich gekappt werden. Die Kappung der Rahmenstruktur durch eine Trennvorrichtung (17) oder durch eine an geeigneten Stellen vorgesehenen Sollbruchstellen kann die in den Körper eingebrachte, exogene Materialmenge reduzieren. Die Kappung kann als weitere Justiermöglichkeit der axialen sowie der rotatorischen Steifigkeit aufgefasst werden.

Der zumindest eine Draht (14) kann eine Aussparung formen, welche dem Zwecke dienen kann, anatomische Strukturen wie die Vena cava inferior nicht räumlich zu beeinträchtigen.

Mithilfe eines geeigneten Fertigungsverfahrens, bspw. durch Laserschneiden, können alternativ aus einem Blech oder einem Halbrohr Segmente der Rahmenstruktur (1) herausgeschnitten werden. Durch anschließende Formgebung (bspw. Biegen, Krümmen des nach dem ersten Fertigungsschnitts ebenen Rahmensegments) und Kopplung der Segmente untereinander kann die Rahmenstruktur (1) entstehen.

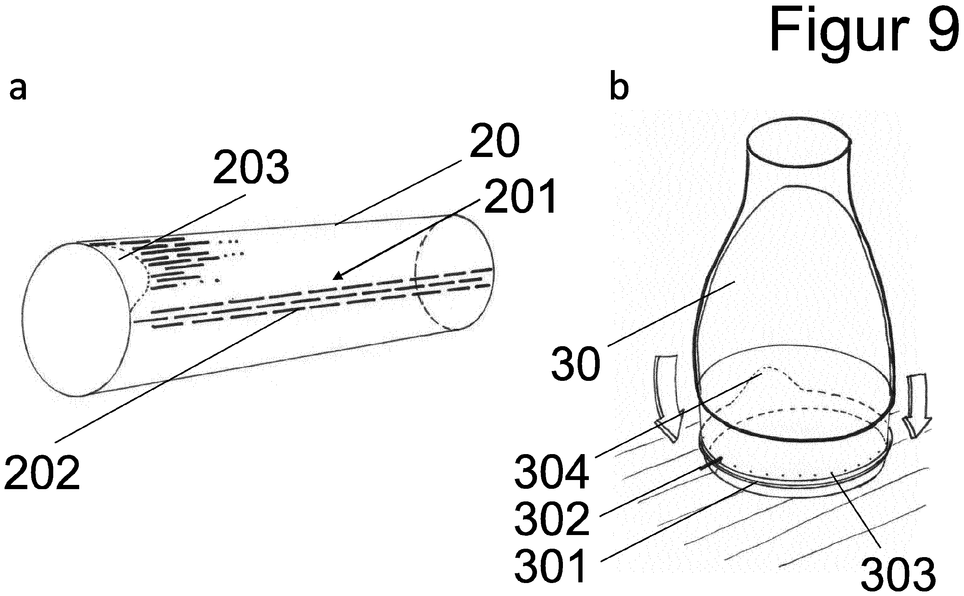

**Figur 9a** **und b** zeigen ein geschlitztes Rohr (20) ("slotted tube") mit angedeutetem Schnittmuster (201) sowie den Vorgang des Aufziehens eines geschlitzten Rohrs (20) auf einen Dorn (30), welcher von einem dreidimensionalen Abbild eines Herzens abgeleitet sein kann. Ziel des Vorgangs ist die Erzeugung einer patientenspezifischen Geometrie der Rahmenstruktur, welche aus einem anfänglich kontinuierlichen, rohrförmiges Halbzeug gefertigt werden kann.

Figur 9a zeigt in Andeutung mehrere in Längsrichtung des Rohres orientierte Reihen aus Schnitten (202), wobei benachbarte Reihen zueinander versetzt sein können. Durch den Versatz kann die Form der Zellen, welche bei der Aufweitung des geschlitzten Rohres entstehen, beeinflusst werden. Des Weiteren kann durch ein entsprechendes Schnittmuster (201) bereits die Aussparung (203) vorgesehen sein, welcher nach der Aufweitung des Rohres dazu führt, dass ein Ausschnitt entlang des Umfangs entsteht, welcher die räumliche Beeinträchtigung der Vena cava oder sonstiger größerer anatomischer Strukturen verhindert. Auch kann durch ein geeignetes Schnittmuster (201) bereits die Form und Länge der Verlängerungsstreben festgelegt werden.

Figur 9b zeigt ein teilweise aufgezogenes geschlitztes Rohr (20) sowie eine Ausführungsform des formgebenden Dorns (30). Der Dorn (30) umfasst eine umlaufende Rippe (301) in der Nähe seiner Basis und einen umlaufenden Kranz aus Bohrungen zur Einführung von Fixierbolzen (302). Die umlaufende Rippe (301) kann der angestrebten umfänglichen Form des Implantats entsprechen. Eine Ausführungsform kann bspw. die Aussparung (304) für die Vena cava oder sonstige größere anatomische Strukturen mithilfe der Führung der umlaufenden Rippe (301) berücksichtigen. Die Rippe (301) kann dazu genutzt werden, die Umfangsform des Implantats genau einzustellen. In dem Zustand, in welchem das Rohr (20) bis zum Anschlag an die Rippe (301) über den Dorn (30) gezogen wurde, kann das Rohr (20) mithilfe von Fixierbolzen (302), welche in die dafür vorgesehenen Bohrungen (303) durch die sich aus der Schlitzung des Rohres ergebenden Öffnungen hindurch eingeführt werden, befestigt werden, bis die aufgeweitete Form in die Vorstufe der Rahmenstruktur eingeprägt wurde. Besteht das geschlitzte Rohr (20) bspw. aus einer Formgedächtnislegierung, so kann der Einprägung der aufgeweiteten Geometrie durch Normalglühen durchgeführt werden. Auch metallische Rahmenstrukturen, die nicht aus Formgedächtnislegierungen bestehen, sind denkbar.

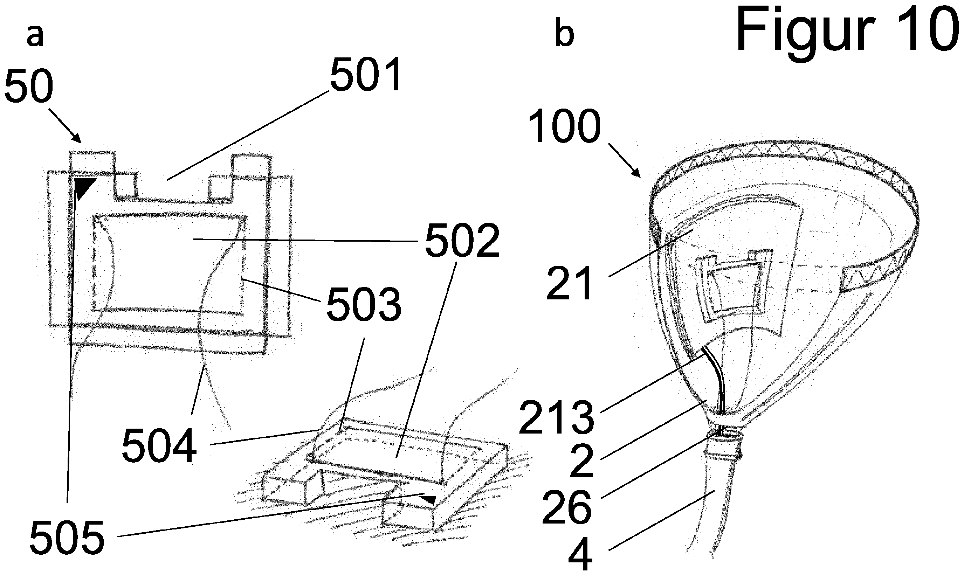

**Figur 10a** **und b** zeigen eine Ausführungsform einer Tasche (50), die einen Substanzträger aufnehmen kann. Die Tasche (50) kann über eine Öffnung (501) an der dem oberen Rand der Hülle (2) zugewandten Seitenfläche mit einer Substanz befüllt werden. Die Tasche (50) verfügt über einen entfernbaren Bereich (502) an der herzzugewandten Fläche. Dessen Entfernung hat die zeitlich und räumlich wohldefinierte Freisetzung der Substanz zur Folge.

Figur 10a zeigt die Abwicklung einer Tasche (50), die einen Substanzträger aufnehmen kann und eine räumliche Darstellung der Tasche (50). Die Tasche (50) kann in der Hülle (2) befestigt werden. Ausführungsformen eines Substanzträgers werden in einem nachfolgenden Abschnitt näher beschrieben. Weist die Vorrichtung in einer Ausführungsform weiter eine expandierbare Einheit (21) auf, welche in die Hülle (2) eingebracht werden kann, so kann die zumindest eine Tasche (50) an der Stirnfläche einer expandierbaren Einheit (21) befestigt werden. Die Tasche (50) kann aber auch an einer Stelle in der Hülle (2) befestigt werden, an der sich keine expandierbare Einheit (21) befindet. Die vorliegende Ausführungsform ist derart ausgelegt, dass die Stirnfläche der expandierbaren Einheit (21) oder die Hülle (2) die Rückseite der zumindest einen Tasche (50) bildet. Alternativ kann die Tasche (50) auch ein vor der Kopplung mit der Hülle (2) und/oder zumindest einer expandierbaren Einheit (21) ein geschlossenes Volumen aufweisen. In diesem Fall kann ein separater Kopplungsmechanismus vonnöten sein. Der Kopplungsmechanismus kann so gestaltet sein, dass die Kopplung auch erst unter OP-Bedingungen erfolgen kann und von einer Person im OP-Saal vorgenommen werden kann.

Die Breite, Länge und Tiefe einer Tasche (50) oder eines Substanzträgers seien jeweils definiert als die Dimension der Tasche (50) oder des Substanzträgers entlang der Umfangsrichtung des Herzes, der Herzlängsachse und entlang einer radialen Richtung senkrecht zur Herzlängsachse. Die zumindest eine Tasche (50) kann zwischen 10 mm und 80 mm breit sein, bevorzugt zwischen 20 mm und 50 mm. Die Länge einer Tasche (50) kann zwischen 10mm und 80mm betragen, bevorzugt zwischen 20 mm und 50 mm. Die Tasche (50) kann zwischen 0.05 mm und 8 mm tief sein. Die zumindest eine Tasche (50) kann zwischen 1 mm und 3 mm tief sein. Das Material der zumindest einen Tasche (50) kann identisch sein mit dem Material der Hülle (2) oder dem Material der zumindest einen expandierbaren Einheit (21). Das Material der zumindest einen Tasche (50) kann sich auch von dem Material der Hülle (2) oder dem Material der zumindest einen expandierbaren Einheit (21) unterscheiden. Das Material der zumindest einen Tasche (50) kann ein Polymer sein. Das Material der zumindest einen Tasche (50) kann Polyurethan, Silikon oder Polytetrafluoräthylen (PTFE) sein. Die Tasche (50) kann aus dem gleichen Material wie die Hülle (2) und/oder eine expandierbare Einheit (21) sein. Die Tasche (50) kann direkt in eine Hülle (2) und/oder eine expandierbare Einheit (21) eingearbeitet sein, womit die Tasche (50) Teil der Hülle (2) oder einer expandierbaren Einheit (21) ist.
Die Tasche (50) kann bspw. zunächst als zweidimensionale Abwicklung aus einer Folie aus zumindest einem der oben genannten Materialien geschnitten werden und anschließend zu ihrer dreidimensionalen Form zusammengefügt werden.

Die zumindest eine Tasche (50) kann eine Öffnung (501) an einer Fläche aufweisen, welche dazu genutzt werden kann, eine Substanz in die Tasche (50) in der vorliegenden Ausführungsform einzubringen. Die zumindest eine Tasche (50) kann über einen entfernbaren Bereich (502) auf der herzzugewandten Fläche verfügen. Der entfernbare Bereich (502) kann rechteckig sein. Der entfernbare Bereich (502) kann eine runde, elliptische oder polygonale Form oder eine Kombination daraus besitzen. Der entfernbare Bereich (502) kann im implantierten Zustand von außerhalb des Körpers durch einen Mechanismus, welcher Zugelemente (404) nutzt, von der Tasche (50) entfernbar sein. Der Mechanismus und dessen Zugelemente (404) können zumindest teilweise aus bspw. Fäden, Schnüre, Stangen, Streifen (bspw. aus Kunststoff) oder ähnlich auf Zug beanspruchbare Bauteile bestehen. Wird dieser Bereich (502) abgezogen, so entsteht eine Öffnung in der zumindest einen Tasche (50), durch welchen die im Innern der zumindest einen Tasche (50) befindliche Substanz nach außen, d.h. ins Innere der Hülle (2) und an dem vorgesehenen Ort der Freisetzung der Substanz mit dem Herz in Kontakt treten kann. Der entfernbare Bereich (502) der zumindest einen Tasche (50) kann durch an diesem Bereich (502) angebrachte Fäden abgezogen werden. Der entfernbare Bereich (502) kann an seinen Rändern zumindest eine Perforierung (403) des Materials der Tasche (50) aufweisen, um den Abziehvorgang ermöglichen zu können. Der entfernbare Bereich (502) kann an seinen Rändern auch zumindest eine Scheinfuge/Sollrissstelle (403) aufweisen, welche den Abziehvorgang ermöglichen kann. Der entfernbare Bereich (502) kann der zeitlich verzögerten Freisetzung einer Substanz im Innern der Perikardhöhle dienen. Die Tasche (50) an sich kann dem Schutz der Substanz, z.B. vor Reibung oder während der Kompression im Zuge des Implantationsvorgangs, dienen. Im implantierten Zustand ist dieser Schutz nicht mehr vonnöten und kann daher entfernt werden.

Alternativ zum entfernbaren Bereich (502) an der herzzugewandten Seite einer Tasche (50) kann ein Bereich (502) vorgesehen sein, welcher zumindest teilweise aus einem bioabbaubaren und oder permeablen Material gefertigt ist. Dies hat den Vorteil, dass eine Substanz während des Implantationsvorganges mechanisch geschützt ist und nachträglich freigesetzt werden kann. Im Gegensatz zum entfernbaren Bereich (502) muss damit kein Teil mehr aus dem Körper entfernt werden, was den Implantationsvorgang erleichtert. Die Freisetzung der Substanz kann auch zeitlich gesteuert erfolgen, indem Materialien mit unterschiedlicher Lösungs- und/oder Abbaugeschwindigkeit, mit definierter Wandstärke oder mit einer bestimmten Permeabilität verwendet werden. Beispiele für solche abbaubaren oder löslichen Materialien sind natürliche Polymere wie Kollagen, Alginat, Polysaccharide wie Hyaluronsäure und Gelatine und/oder synthetische aliphatische Poly(Hydroxycarbonsäure)-Polyester wie Poly-E-Caprolacton, Polylactid, Polyglykolsäure, Poly(4-Hydroxybuttersäure), Poly(3-Hydroxybuttersäure), Poly(2-Hydroxybuttersäure) sowie deren Co-Polymere und/oder selbstassemblierende Peptide wie auf den Aminosäuren Arginin-Glycin-Aspartat basierende Peptide. Beispiele für permeable Stoffe sind Methyl-Cellulose, Polyurethane sowie deren Co-Polymere, wobei die Porosität vom Herstellungsverfahren abhängig ist. Die zumindest eine Tasche (50) kann auch vollständig aus einem bioabbaubaren und/oder einem permeablen Polymer bestehen.

Die Tasche (50) kann weiterhin eine röntgenopake Markierung (505) umfassen, mithilfe welcher sich bspw. bei einer separat an der Hülle (2) und/oder der zumindest anbringbaren Tasche (50)

Figur 10b zeigt eine Ausführungsform des Implantats (100), welche teilweise aufgeschnitten ist, um den Blick auf das Innere der Hülle (2) freizugeben. Auf der vorhandenen expandierbaren Einheit (21) ist eine Tasche (50) befestigt, welche einen Substanzträger aufnehmen kann. Der entfernbare Bereich (502) an der Stirnfläche der Tasche (50) in Form eines Rechtecks ist an den zwei oberen Ecken mit Fäden verbunden, welche durch die an der unten liegenden Öffnung (26) der Hülle (2) durch den ummantelten Kabelbaum (4) nach außen, d.h. aus dem Körper heraus, geführt werden.

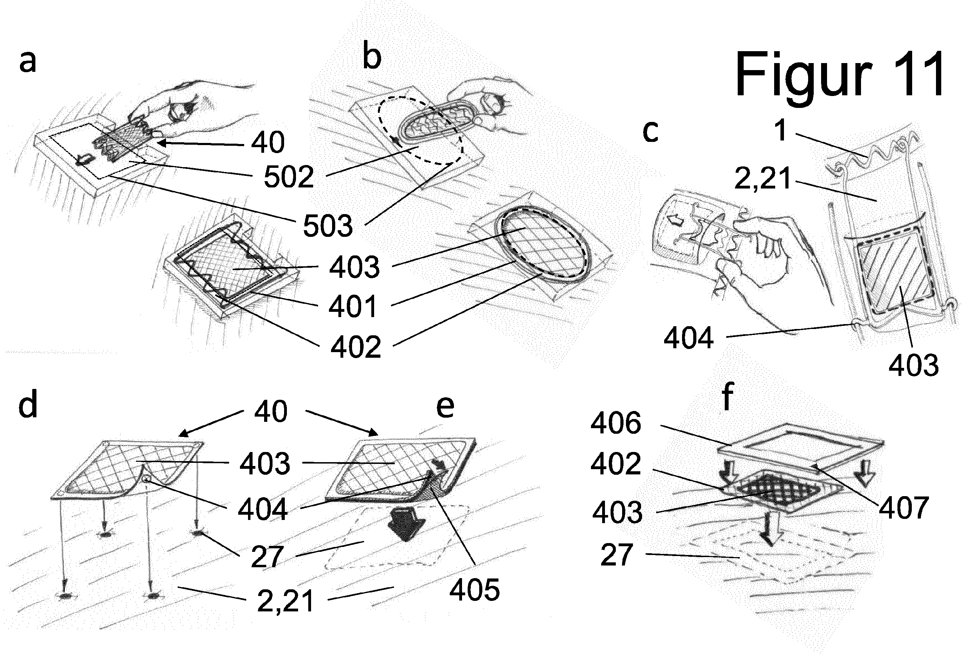

**Figur 11** zeigt verschiedene Ausführungsformen des Substanzträgers (40).

Figur 11a zeigt die Herstellung und Einbringung eines Substanzträgers (40) in eine Tasche (50), die dafür vorgesehen sein kann, einen Substanzträger (40) aufzunehmen während der Einbringung des Substanzträgers (40) in die Tasche (50) und im verbauten Zustand. Auf den Substanzträger (40) kann eine Substanz (403) aufgebracht werden, welche eine therapeutische Wirkung haben kann. Die zumindest eine Substanz (403) kann auf den Substanzträger (40) aufgebracht werden, bspw. durch Auftragen, Verstreichen, Aufkleben, Aufdampfen, Besiedeln mit Zellen, Aufgießen oder Thermobonden.

In Figur 11a wird der Substanzträger (40) mehrteilig dargestellt, bestehend aus einem Rahmen (401) und einer Membran (402), welche durch den Rahmen (401) aufgespannt wird und auf welche zumindest eine Substanz (403) aufgebracht werden kann. Die Membran (402) kann mit dem Rahmen (401) verbunden sein, bspw. durch eine Verklebung. Der Rahmen (401) kann auch in die Membran (402) eingearbeitet, bspw. eingegossen, sein. Der Rahmen (401) kann zur Erleichterung des Einführvorgangs des Substanzträgers (40) in die zumindest eine Tasche (50) dienen. Die Tasche hat einen entfernbaren Bereich (502) haben, welcher eine Perforation (503) aufweisen kann. Der Rahmen (401) und der Substanzträger (40) können zum Zwecke des Einführens in eine Tasche (50) komprimiert werden. Im nicht-komprimierten Zustand kann der Rahmen (401) die korrekte Positionierung des Substanzträgers (40) und der darauf aufgebrachten Substanz (403) gewährleisten. Der Rahmen (401) kann derart dimensioniert sein, dass er im komprimierten Zustand durch eine Öffnung der Tasche (50) in das Innere der Tasche (50) eingeführt werden kann und im nicht-komprimierten Zustand breiter als die Öffnung ist. Dadurch kann vor der Implantation des Implantats ein einfaches Einführen des Substanzträgers (40) gewährleistet werden und eine Dislokation des Substanzträgers (40) nach Implantation des Implantats verhindert werden. Der Rahmen (401) des Substanzträgers (40) kann aus Polyurethan (PU), Silikon, Polytetrafluorethylen (PTFE), Metall oder einer Metalllegierung, insbesondere einer Formgedächtnislegierung, oder einer Kombination daraus sein. Das zumindest eine Material kann eine Formgedächtnislegierung sein. Die Membran (402) kann aus Polyurethan, Silikon oder Polytetrafluorethylen (PTFE) sein. Die Membran (402) kann auch aus dem gleichen Material wie der Rahmen (401) bestehen. Bestehen Membran (402) und Rahmen (401) aus dem gleichen Material, kann der Substanzträger (40) auch einteilig gestaltet sein. Der Rahmen (401) kann dann aus einer Anhäufung des Materials bestehen, bspw. aus einer Verdickung des Materials am Rand des Substanzträgers (40). Alternativ kann ein einteiliger Substanzträger (40) dadurch gekennzeichnet sein, dass im Membranbereich Material abgetragen wurde oder nachträglich geschwächt wurde, bspw. durch Einwirken von Strahlung oder Chemikalien.

Die Membran (402) kann aus einem anderen Material als der Rahmen (401) sein. Die Membran (402) kann über einen eingearbeiteten Rahmen (401) verfügen. Der Rahmen (401) kann aus einem selbst-expandierenden Material sein. Der Rahmen (401) kann aus einer Formgedächtnislegierung und/oder aus einem superelastischen Material sein, so dass der Substanzträger (40) zum Zwecke der Einbringung in eine Tasche (50) von einer nicht-komprimierten Form in eine komprimierte Form überführt werden kann. Der Rahmen (401) kann aus Nitinol sein. Der Substanzträger (40) kann stark verformbar sein. Der Substanzträger (40) kann zum Zwecke der Einbringung in eine Tasche (50) derart verformbar sein, dass seine Breite in einem komprimierten Zustand kleiner oder gleich der Größe einer Öffnung in einer Tasche (50) ist. Der zumindest eine Substanzträger (40) kann zwischen 10 mm und 80 mm breit sein, bevorzugt zwischen 20 mm und 50 mm. Der zumindest eine Substanzträger (40) kann zwischen 10 mm und 80 mm lang sein, bevorzugt zwischen 20 mm und 50 mm. Der zumindest eine Substanzträger (40) kann zwischen 0.5 mm und 5 mm dick sein, bevorzugt zwischen 1 mm und 3 mm. Der Rahmen (401) und/oder der Substanzträger (40) können auch von der flachen Form abweichen und dreidimensional an die Krümmung eines Zylinders, einer Kugel oder eines Abbildes eines Herzes angepasst sein.

In der vorliegenden Ausführungsform kann der Substanzträger (40) durch eine Öffnung der Tasche (50) in das Innere der Tasche (50) eingebracht werden. In der vorliegenden Figur ist ein Substanzträger (40) mit einem rechteckigen Rahmen (401) dargestellt. Die Öffnung der Tasche (50) kann kleiner sein als die Kantenlängen des Substanzträgers (40) und/oder des Rahmens (401). Zumindest eine Kante des Rahmens (401) kann in einer gewellten, mäandernden, krummen oder geknickten Form vorliegen. Die Abweichung der Form zumindest einer Kante des Rahmens (401) von der geraden Form kann zu einer besseren Komprimierbarkeit des Rahmens (401) führen. In einer Ausführungsform, bei der zumindest ein Teil des Substanzträgers (40) aus einem elastischem und/oder superelastischem Material besteht, kann sich der Substanzträger (40) nach der Deformation bei der Einbringung durch die Öffnung der Tasche (50) in das Innere der Tasche (50) wieder in seine Ausgangsgeometrie vor der Deformation zurückformen. Dadurch kann ein besserer Sitz des Substanzträgers (40) in der Tasche (50) erzielt werden. Der Rahmen (401) des Substanzträgers (40) kann zumindest aus einem Draht bestehen. Der Rahmen (401) des Substanzträgers (40) kann durch Heraustrennen eines Profils aus einem zuvor kontinuierlichen Halbzeug hergestellt werden, bspw. durch Stanzen aus einem Blech oder Laserschneiden aus einem Rohr, einem Blech oder einer Folie. Alternativ kann der Rahmen (401) auch durch ein generatives Fertigungsverfahren, wie Gießen, Sintern oder ein Rapid-Prototyping Verfahren, hergestellt werden. Der Rahmen (401) kann aus einem in sich geschlossenen Profil bestehen. Besteht der Rahmen (401) aus zumindest einem Draht, so können die Enden des zumindest einen Drahtes verbunden sein, bspw. durch Kleben, Schweißen, Nieten, Haken und/oder Ösen oder eine Hülse, wodurch ein geschlossener Rahmen (401) entsteht. Das Profil oder ein Draht des Rahmens (401) kann auch an zumindest einer Stelle nicht verbunden sein und es entsteht ein offener und/oder unterbrochener Rahmen (401). Ein geschlossener Rahmen (401) weist eine höhere Struktursteifigkeit auf und kann auch bei Einwirkung von Kräften durch Bewegung des Herzes oder bei Belastung durch eine expandierbare Einheit, der Hülle (2) oder eines anderen Teils des Implantats eine gewisse Formstabilität des Substanzträgers (40) sicherstellen. Ein offener Rahmen (401) hat den Vorteil, dass der Substanzträger (40) leichter zu komprimieren ist als ein vergleichbarer Substanzträger (40) mit geschlossenem Rahmen (401). Die Form der Drahtwicklung oder des Profils kann zumindest teilweise eine von der geraden Linie abweichende Form aufweisen. Der zumindest eine Draht oder das Profil des Rahmens (401) kann eine periodisch alternierende Leitkurve aufweisen. Die Leitkurve kann auch aperiodisch alternierend sein. Ist das Material des Rahmens (401) eine Formgedächtnislegierung, kann der Rahmen (401) mit Hilfe von zumindest einer Wärmebehandlung in eine definierte Form gebracht werden.

Figur 11b zeigt eine weitere Ausführungsform des Substanzträgers (40) während der Einbringung des Substanzträgers (40) in die Tasche (50) und im verbauten Zustand. Die gezeigte Tasche (50) entspricht hierbei jener in Figur 11a. Die vorliegende Ausführungsform des Substanzträgers (40) ist elliptisch und umfasst eine Membran (402), welche zumindest einen in die Membran (402) eingearbeiteten Draht zur Aufspannung der Membran (402) enthält. In der vorliegenden Ausführungsform kann zumindest eine der zwei Hauptachsen länger sein als die zu dieser Hauptachse parallele Kante der in dieser Ausführungsform rechteckigen Tasche (50), was dazu führt, dass der elliptische Substanzträger (40) in der rechteckigen Tasche (50) durch die resultierende Klemmwirkung besser fixiert ist. Die Tasche (50) kann auch von der Rechteckform abweichen. In diesem Fall kann es vorteilhaft sein, auch die Form des Substanzträgers (40) abzuwandeln und die der Taschenform anzupassen. Vorteile der elliptischen Ausführung sind, unter anderem, die simple Geometrie und die einfache Herstellbarkeit des Substanzträgers (40).

Figur 11c zeigt eine weitere Ausführungsform des Substanzträgers (40) und der Tasche (50) während der Einbringung des Substanzträgers (40) in die Tasche (50) und im verbauten Zustand. Verwendbare Materialien für den Substanzträger (40) und die Tasche (50) sind in einem vorangehenden Abschnitt genannt. Die gezeigte Ausführungsform des Substanzträgers (40) ist zweiteilig. Sie umfasst einen Rahmen (401) und eine Membran (402), auf die zumindest eine Substanz (403) aufgebracht werden kann. Für den Substanzträger (40), den Rahmen (401) und die Membran (402) gelten die vorangegangenen Beschreibungen entsprechend. Figur 11c zeigt weiter eine Möglichkeit zur Verbindung des Substanzträgers (40) an die Rahmenstruktur (1), die Hülle (2), die Stirnfläche der zumindest einen expandierbaren Einheit (21) und/oder an die Tasche (50). Zu diesem Zweck kann der Substanzträger (40) über zumindest ein Kopplungselement (404) verfügen. Das zumindest eine Kopplungselement (404) kann Teil des Rahmens (401), bspw. eine längliche Strebe oder ein Drahtelement, sein und vom Rahmen (401) wegragen. Das Kopplungselement (404) kann am Ende derart ausgeführt sein, dass eine Kopplung an ein anderes Bauteil der Vorrichtung ermöglicht wird. Das Kopplungselement (404) kann bis zu einem strukturell tragenden Bauteil der Rahmenstruktur (1) reichen oder bis zu einem anderen Kopplungselement (404) der Hülle (2), der Tasche (50) und/oder einer expandierbaren Einheit (21). In Figur 11c sind vier Kopplungselemente dargestellt, zwei der Kopplungselemente ragen seitlich vom Rahmen (401) des Substanzträgers (40) weg und verbinden diesen mittels Haken an zwei Drähten oder Streben der Rahmenstruktur (1). Die zwei anderen Kopplungselemente aus Figur 11c ragen vom Rahmen (401) nach oben weg und verbinden diesen durch eine Öffnung in Hülle (2) und am "oberen Kranz" der Rahmenstruktur (1) mit dem Implantat. Die Kopplung kann über Formschluss, Kraftschluss und/oder Stoffschluss erfolgen. Im Falle einer formschlüssigen Kopplung kann die zumindest eine Kopplungsmöglichkeit eine Schlaufe, einen Haken, eine Öse, ein Knopf oder zumindest einen Klett- und/oder Reißverschluss enthalten. Im Falle einer kraftschlüssigen Kopplung kann eine Klebeverbindung oder eine Verbindung durch Verknoten mit Fäden, Lamellen oder Seilen eingesetzt werden. Im Falle einer stoffschlüssigen Kopplung kann die Verbindung eine Schweißverbindung oder eine Klebeverbindung sein. In der vorliegenden Ausführungsform ist die Kopplung mit vier Kopplungselementen dargestellt. Ein Substanzträger (40) kann auch eins, zwei, drei, vier, fünf, sechs oder mehr Kopplungselemente aufweisen.

Die Tasche (50) in der vorliegenden Ausführungsform kann zwei Öffnungen an gegenüberliegenden Enden haben. Der Substanzträger (40) kann durch die erste Öffnung in die Tasche (50) eingebracht werden und durch die zweite Öffnung wieder austreten. Die Tasche (50) kann über einen entfernbaren Bereich (502) auf der Stirnfläche verfügen, welche durch eine Perforation (503) und/oder eine Scheinfuge abgegrenzt sein kann. Nach Einführung und Befestigung des Substanzträgers (40) an der Hülle (2) und/oder an der Stirnfläche der zumindest einen expandierbaren Einheit (21) und/oder der Rahmenstruktur (1) kann der entfernbare Bereich (502) entfernt werden. Alternativ ist der entfernbare Bereich (502) wie oben beschrieben zumindest teilweise aus einem bioabbaubaren und oder permeablen Material gefertigt. Der entfernbare Bereich (502) kann der Beschreibung zu Figur 10 folgend geformt sein. In Folge der Entfernung des entfernbaren Bereichs (502) kann die Substanz (403), welche sich auf dem Substanzträger (40) befindet, direkt mit der Herzoberfläche in Kontakt gebracht werden. Die Tasche (50) kann die Funktion eines Schutzes des Substanzträgers (40) im nicht-expandierten Zustand des Implantats haben. Die Tasche (50) kann die Positionstreue des Substanzträgers (40) erhöhen. Ein Vorteil der in Figur 11c gezeigten Ausführungsform ist die zusätzliche Fixierung des Substanzträgers (40) in der Rahmenstruktur (1) bei gleichzeitigem Schutz des Substanzträgers (40) durch die Tasche (50), welche hier in Form einer Schutzhülle gezeigt ist.

Figur 11d zeigt eine weitere Ausführungsform eines Substanzträgers (40) bestehend aus einer Membran (402), welche direkt mit der Innenseite der Hülle (2) und/oder an der Stirnfläche der zumindest einen expandierbaren Einheit (21) verbunden werden kann. In dieser Ausführungsform kann auf eine Tasche (50) und/oder einen Rahmen (401) verzichtet werden. Die Verbindung zwischen Substanzträger (40) und der Oberfläche der Hülle (2) und/oder der Stirnfläche der zumindest einen expandierbaren Einheit (21) kann auf die in einem vorangehenden Abschnitt der Beschreibung erwähnten Arten ausgeführt sein. Insbesondere kann die zumindest eine Kopplung (27, 404) als Steckverbindung oder als Klebeverbindung ausgeführt sein. Die zumindest eine Kopplung (27, 404) kann auch durch zumindest eine Knopf-Öse-Verbindung oder zumindest eine Klettverschlussverbindung stattfinden. In der vorliegenden Ausführungsform kann der Substanzträger (40) vier punktuelle Kopplungsmöglichkeiten aufweisen. Die Kopplungsmöglichkeit (27, 404) kann auch als flächige Kopplung ausgeführt sein. Die Kopplung (27, 404) mit der Oberfläche der Hülle (2) und/oder der Stirnfläche der zumindest einen expandierbaren Einheit (21) kann zumindest eines Kopplungspaars (27, 404) bedürfen, wobei ein Kopplungspartner am Substanzträger (40) befestigt sein kann und der andere Kopplungspartner auf der Oberfläche der Hülle (2) oder der Stirnfläche der zumindest einen expandierbaren Einheit (21). Die Aufbringung der Substanz (403) auf den Substanzträger (40) kann einer vorangehenden Beschreibung entsprechen. Vorteile einer derartigen Ausführungsform sind die vereinfachte Anbringung des Substanzträgers (40) in der Hülle (2) oder auf einer expandierbaren Einheit (21) und dass nicht zusätzlich eine Tasche (50) benötigt wird, um den Substanzträger (40) in Position zu halten.

Figur 11e zeigt eine weitere Ausführungsform des Substanzträgers (40). Sie ist ähnlich der Ausführungsform, welche in Figur 11d beschrieben wird. Der Substanzträger (40) kann nur aus einer Membran (402) auf welche eine Substanz (403) aufgebracht werden kann bestehen und keinen Rahmen (401) enthalten. Die Kopplung (27, 404) zwischen der Unterseite des Substanzträgers (40) und der Innenseite der Hülle (2) und/oder der Stirnseite einer expandierbaren Einheit (21) ist in dieser Ausführungsform flächig ausgeführt. Die zumindest eine flächige Kopplungsmöglichkeit (404) kann die Unterseite des Substanzträgers (40) zumindest teilweise bedecken. Die Unterseite des Substanzträgers (40) kann auch zwei, drei, vier, fünf, sechs oder mehr flächige Kopplungselemente (404) haben, welche die Unterseite zumindest jeweils teilweise bedecken. Die Unterseite des Substanzträgers (40) kann eine, zwei, drei, vier, fünf, sechs, sieben oder mehr flächige Kopplungselemente (404) haben. Der Kopplungspartner des zumindest einen flächigen Kopplungselements (404) kann die Oberfläche der Hülle (2) und/oder die Stirnfläche der zumindest einen expandierbaren Einheit (21) sein. Die Oberfläche der Hülle (2) und/oder die Stirnfläche der zumindest einen expandierbaren Einheit (21) kann auch zumindest ein Kopplungselement (404) aufweisen, welches dem zumindest einen Kopplungselement (404) an der Unterseite des Substanzträgers (40) als Kopplungspartner dient. Das zumindest eine flächige Kopplungselement (404) an der Unterseite des Substanzträgers (40) kann zumindest eine flächige Abdeckung (405) aufweisen, welche entfernt werden kann, um das zumindest eine flächige Kopplungselement (404) an der Unterseite des Substanzträgers (40) freizugeben. Das komplementäre Kopplungsgebiet (27) ist auf der Hülle (2) und/oder der zumindest einen expandierbaren Einheit (21) verortet.

Alternativ kann die flächige Abdeckung (405) auch am Substanzträger (40) verbleiben, wenn sie selbst eine Klebeverbindung ermöglicht und darüber hinaus zumindest eine Zusatzfunktion aufweist. Beispielsweise kann diese Zusatzschicht (405) zumindest teilweise elektrisch leitfähig sein, wodurch die Möglichkeit bestehen kann, ein Potential an den Substanzträger (40) oder die Substanz (403) anzulegen. Dies kann das Wachstum von bspw. Stammzellkulturen beeinflussen.

In der vorliegenden Ausführungsform kann die Abdeckung (405) des zumindest einen flächigen Kopplungselements entfernt werden. Der Substanzträger (40) in der vorliegenden Ausführungsform kann direkt auf die Oberfläche der Hülle (2) und/oder die Stirnfläche der zumindest einen expandierbaren Einheit (21) aufgebracht und befestigt werden. Die Vorteile der vorliegenden Ausführungsform entsprechen jenen der in Figur 11d gelisteten. Zusätzlich ermöglicht die flächige Kopplung (27, 404) einen potentiell besseren Halt als bei der Ausführungsform in Figur 11d. Zudem ist sie, da keine Tasche (50) vorhanden ist, frei in ihrer Platzierung.

Figur 11f zeigt eine weitere Ausführungsform, in welcher der Substanzträger (40) aus zumindest zwei Teilen besteht. Ein Teil kann eine Membran (402) sein, auf welche eine Substanz (403) aufgebracht werden kann. Weiter kann Substanzträger (40) aus zumindest einer Befestigungskomponente (406) bestehen. Die weitere zumindest eine Befestigungskomponente (406) des Substanzträgers (40) kann zum Positionieren und Fixieren der Membran (402) auf der Innenseite der Hülle (2) und/oder der Stirnfläche der zumindest einen expandierbaren Einheit (21) dienen. In der dargestellten Ausführungsform liegt die zumindest eine Befestigungskomponente (406) als flaches, rahmenartiges Bauteil vor. Die zumindest eine Befestigungskomponente (406) kann wie ein Rahmen (401) aus der vorangegangenen Beschreibung gestaltet sein, mit der Besonderheit, dass die Membran (402) auf welche die Substanz (403) aufgebracht werden und der Rahmen (401) erst beim Einbringen in die Hülle (2)/ auf die expandierbare Einheit (21) miteinander verbunden werden. Die Unterseite der zumindest einen Befestigungskomponente (406) kann mit der Oberseite der Membran (402) zumindest teilweise überlappen. Durch diese zumindest teilweise Überlappung kann die Membran (402) auf der Oberfläche der Hülle (2) und/oder der Stirnfläche der zumindest einen expandierbaren Einheit (21) (21) befestigt werden. Die Unterseite der zumindest einen Befestigungskomponente (406) kann mit der Innenseite der Hülle (2) und/oder der Stirnfläche der zumindest einen expandierbaren Einheit (21) mittels zumindest einer Kopplung (27, 406) verbunden werden. Die zumindest eine Kopplung (27, 406) kann als Steckverbindung oder als Klebeverbindung ausgeführt sein. Die zumindest eine Klebeverbindung (27, 406) kann in Form einer selbstklebenden Folie realisiert werden, welche mit der Oberfläche der Hülle (2) und/oder der Stirnfläche der zumindest einen expandierbaren Einheit (21) verbunden werden kann. Die selbstklebende Folie kann aus einer Stützschicht, einer Klebeschicht und einer Schutzfolie, welche die Klebeschicht bspw. vor Wasserkontakt schützt, bestehen. Die Stützschicht kann aus Polyurethan, Silikon oder Polytetrafluorethylen (PTFE) bestehen. Als Klebeschicht bei einer selbstklebenden Folie ist eine Silanschicht als Ein-KomponentenKlebstoff möglich. Auch anders geartete Klebeverbindungen sind denkbar. Die Aushärtung erfolgt nach Entfernen der entfernbaren Schutzschicht in feuchter Umgebung durch Wasser. Die zumindest eine Kopplung kann auch durch zumindest eine Knopf-Öse-Verbindung oder zumindest eine Klettverschlussverbindung bewirkt werden.

Für jede der vorgenannten Ausführungsformen gilt, dass der Substanzträger (40) erst unmittelbar vor einer Implantation des Implantats unter OP-Bedingungen von einer Person im OP-Saal mit der Substanz (403) versehen werden kann und anschließend in die Vorrichtung, bspw. in eine Tasche (50), auf die Innenseite einer Hülle (2) und/oder auf die Stirnseite einer expandierbaren Einheit (21), eingebracht werden kann. Alternativ kann ein bereits mit einer Substanz (403) versehener Substanzträger (40) verwendet werden und dieser unter OP-Bedingungen in die Vorrichtung eingeführt werden. Alternativ kann der Substanzträger (40) bereits während der Fertigung der Vorrichtung in eine Tasche (50), in die Hülle (2) und/oder auf die Stirnseite einer expandierbaren Einheit (21) eingebracht werden kann und während der Fertigung der Vorrichtung oder zu einem späteren Zeitpunkt, bspw. vor der Implantation der Vorrichtung, mit einer Substanz (403) versehen werden kann. Unabhängig vom Vorbereitungsgrad des Substanzträgers (40)/der Vorrichtung, kann vor der Implantation der Vorrichtung eine Vorbehandlung des Substanzträgers (40) und/oder der Substanz (403) erforderlich sein. Vorbehandlungen der Substanz (403) und/oder des Substanzträgers (40) können das Spülen mit einem Puffer (bspw. Phosphate Buffered Saline, PBS) zum Einstellen eines bestimmten pH-Wertes, das Spülen mit einer Nährlösung, das Einstellen einer Temperatur oder eine Kombination daraus sein. Alternativ kann auch eine Vorrichtung mit einem Substanzträger (40) implantiert werden, auf welchem sich noch keine Substanz (403) befindet. Die Substanz (403) kann dann nach erfolgter Implantation hinzugefügt werden. Details hierzu werden in einem späteren Abschnitt erläutert. Vorteil dieser Ausführungsform ist die Funktionstrennung in einen reinen Substanzträger (40) und ein Befestigungsrahmen (406), wodurch die Fertigung der einzelnen Teile weniger komplex ausfallen und zudem die Handhabe im Anwendungsfall vereinfacht werden kann.

Eine weitere Ausführungsform des Substanzträgers (40) kann porös sein. Der Substanzträger (40) kann somit die Substanz (403) schwammartig in sich speichern und bspw. auf Druck hin freigeben. Durch die vergrößerte Oberfläche und durch den Einsatz biokompatibler Materialien oder einer biokompatiblen Beschichtung kann der Substanzträger (40) in einer derartigen Ausführungsform auch mit lebenden Zellen bestückt werden, welche von ihm ausgehend bspw. bei stetem Kontakt zur Herzoberfläche auf diese übertreten können.

Der Substanzträger (40) kann zumindest einen Sensor und/oder zumindest eine Elektrode umfassen. Der Sensor kann hierbei ein Temperatursensor, ein Drucksensor, ein pH-Sensor ein Sauerstoffsensor, ein CO₂-Sensor, ein optischer Sensor, ein Leitfähigkeitssensor oder ein Impedanzsensor sein. Mögliche Funktionen der Sensoren sind in einem vorangehenden Abschnitt erwähnt. Mithilfe eines Sensors können die Bedingungen am und in der Umgebung des Substanzträgers (40) erfasst werden, um bspw. Aufschluss darüber zu geben, ob Kontakt mit der Herzoberfläche besteht.

Der Substanzträger (40) kann ferner zumindest eine röntgenopake Markierung (407) umfassen, mit Hilfe derer die Position des Substanzträgers (40) während und nach der Implantation überprüft werden kann. Alternativ oder zusätzlich kann die Tasche (50) eine röntgenopake Markierung (505) umfassen.

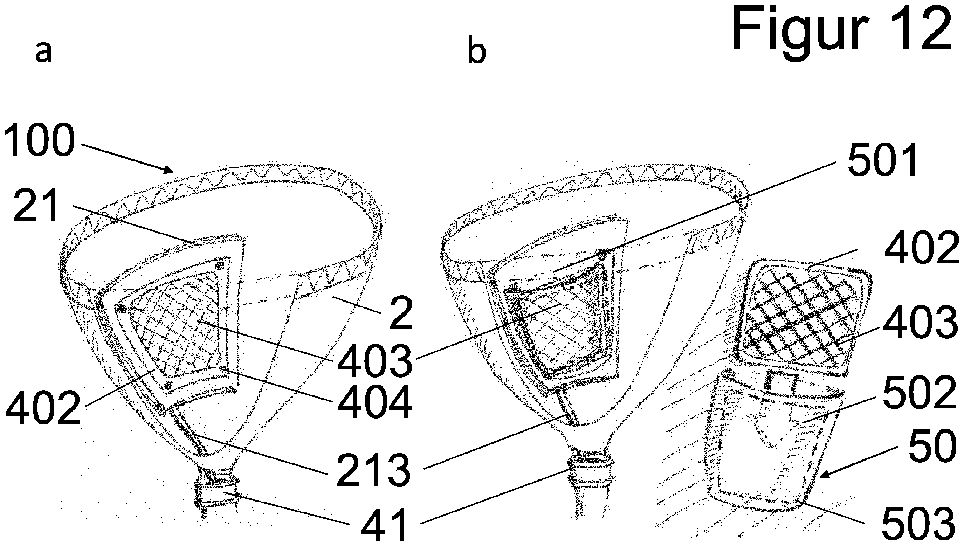

**Figur 12a** **und b** zeigen zwei Ausführungsformen des Implantats (100). Für eine bessere Sichtbarkeit des Substanzträgers (40), welcher auf der zumindest einen expandierbaren Einheit (21) befestigt ist, sorgt ein Teilschnitt durch die Hülle (2). Die zumindest eine expandierbare Einheit (21) kann mit einer Zu- und Ableitung (213) versehen sein.
Figur 12a zeigt eine Ausführungsform des Implantats (100), bei welcher der Substanzträger (40) ohne Tasche (50) direkt auf der Stirnfläche der zumindest einen expandierbaren Einheit (21) befestigt werden kann. Die Befestigung kann mithilfe von in diesem Fall punktförmigen Kopplungen (27, 404) erfolgen. Die punktförmigen Kopplungen (27, 404) können Steckverbindungen, Knopf-Öse-Verbindungen oder Klebeverbindungen, alternativ Klettverschlussverbindungen sein. Der Substanzträger (40) kann aus einer Membran (402) bestehen und eine darauf aufgebrachte Substanz (403) umfassen. Alternativ kann der Substanzträger (40) einen Rahmen (401) haben, über welchen eine Membran (402) gespannt ist, auf welche wiederum die Substanz (403) aufgebracht werden kann. Auch hiervon abweichende Ausführungsformen eines Substanzträgers (40) sind denkbar.

Figur 12b zeigt eine Ausführungsform des Implantats (100), bei welcher der Substanzträger (40) in eine Tasche (50) über eine Öffnung (501) eingebracht und direkt auf der Stirnfläche der zumindest einen expandierbaren Einheit (21) befestigt werden kann. Die Tasche (50) kann einen entfernbaren Bereich (502) aufweisen, welcher nach seiner Entfernung ein Fenster in der Tasche (50) freigeben kann. Der entfernbare Bereich (502) kann von einer Perforation (503) definiert werden, welche die Entfernung des entfernbaren Bereichs (502) erleichtern kann. Dadurch kann die auf dem Substanzträger (40) angebrachte Substanz durch das Fenster hindurch mit der Herzoberfläche in Kontakt gebracht werden. Die Kopplung des Substanzträgers (40) mit der Oberfläche der zumindest einen expandierbaren Einheit (21) kann über eine flächige Kopplungsmöglichkeit (27, 404) stattfinden, etwa einer flächigen Klebeverbindung auf der Unterseite des Substanzträgers (40), welche die Unterseite des Substanzträgers (40) zumindest teilweise bedeckt. In der dargestellten Ausführungsform ist die zumindest eine expandierbare Einheit (21) als inflatierbare Kammer aufgebaut. Hierbei kann es sich um eine inflatierbare Kammer handeln, welche mit einem Fluid gefüllt wird. Als Fluide können Flüssigkeiten oder Gase verwendet werden. Alternativ kann die inflatierbare Kammer auch mit Festkörpern (z.B. Nanopartikelgemische) befüllt werden. Alternativ kann die inflatierbare Kammer auch mit Flüssigkeiten und/oder Gasen und/oder Festkörpern befüllt werden. Die vorliegende Ausführungsform des Implantats (100) weist eine mit einem Gasgemisch (Luft) befüllbare, inflatierbare Einheit auf, welche über eine pneumatische Leitung (213) mit einem Gasgemisch versorgt wird. Die pneumatische Leitung (213) kann hierbei durch die Fixierhülse (41) und den Kabelbaum (4) hindurchgeführt werden.

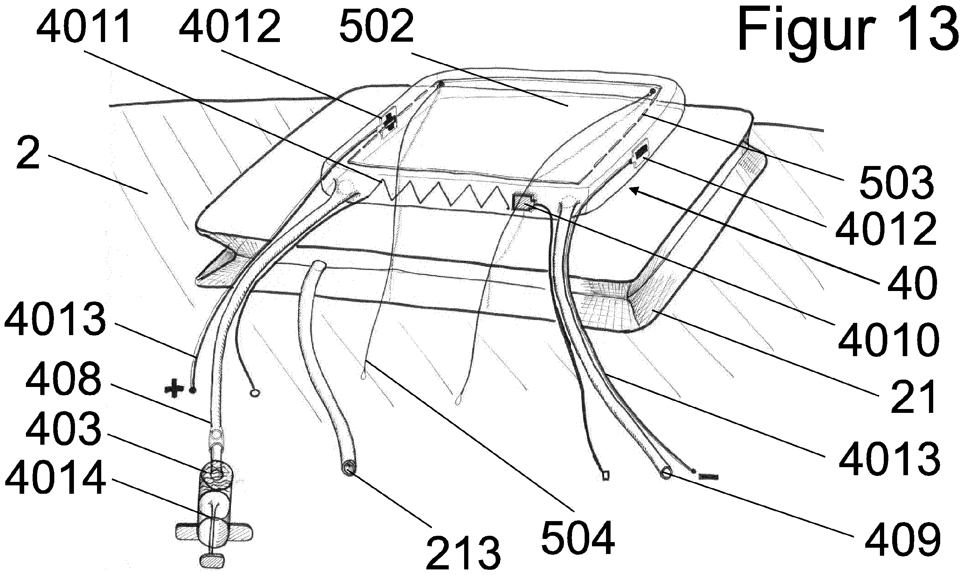

**Figur 13** zeigt eine Ausführungsform des zumindest einen Substanzträgers (40) des Implantats in Form eines Behältnisses (40), eine Substanzzuleitung (408), eine Substanzableitung (409), einen Sensor (4010) und eine Nachstelleinrichtung (4011).

Das Material des Substanzträgers (40) kann gleich dem der zumindest einen expandierbaren Einheit (21) sein. Es kann auch ein davon abweichendes Material gewählt werden. Mögliche Materialien sind an anderer Stelle genannt. Zum Zwecke des Zuführens, Austauschens und/oder Nachfüllen von Substanz (403) in der Vorrichtung im implantierten Zustand, kann die Vorrichtung weiter zumindest eine Substanzzuleitung (408) enthalten. Zum Ableiten alter, verbrauchter oder therapeutisch nicht mehr wirksamer Substanz (403) aus der implantierten Vorrichtung oder auch zum Zwecke des Spülens des Substanzträgers (40) und/oder der Membran kann die Vorrichtung weiter zumindest eine Substanzableitung (409) beinhalten. Die jeweils zumindest eine Substanzzuleitung (408) und/oder - ableitung (409) kann außerhalb des Körpers enden. In dieser Ausführungsform kann der Substanzträger (40) nach der Implantierung von außerhalb des Körpers mit der Substanz (403) selbst oder mit einer Flüssigkeit, welche die zumindest eine Substanz (403) enthält, befüllt werden. In dem dargestellten Ausführungsbeispiel kann die Befüllung des Substanzträgers (40) über eine manuelle Zuführvorrichtung (4014), bspw. eine Spritze, welche die Substanz (403) enthält, stattfinden. Auch eine maschinell gesteuerte Zuführung ist denkbar. Der Substanzträger (40) kann über zumindest eine Ableitung (409) verfügen, über welche eine Flüssigkeit aus dem Inneren des Substanzträgers (40) abgezogen werden kann. Die Entleerung des Substanzträgers (40) kann von außerhalb des Körpers vorgenommen werden. Die Möglichkeit der zumindest teilweisen Befüllung und Entleerung von außerhalb des Körpers kann zur Einstellung der Menge der zumindest einen Substanz (403), zur Einstellung der Konzentration für den Fall, dass die Substanz (403) ein Wirkstoff ist, oder zur Erneuerung der die Substanz (403) beinhaltenden Flüssigkeit dienen. Der zumindest eine Substanzträger (40) kann auch nur über eine gemeinsame Substanzzuleitung und -ableitung verfügen. Die zumindest eine Substanzzuleitung (408) und/oder die zumindest eine Substanzableitung (409) können auch im Kabelbaum aus dem Körper hinaus geführt werden. Die Steckerteile am Kabelbaum können Ports für die Verbindung von Substanzleitungen beinhalten und die Substanzleitungen können an diese Ports verbunden werden. Die Steckerteile können mit einer Versorgungseinheit verbunden werden. Das Zuführen oder Ableiten von Substanz (403) aus einem Substanzträger (40) kann auch durch die Versorgungseinheit geregelt werden. Die Versorgungseinheit kann hierfür entsprechend ein Substanz-Reservoir, eine Förderpumpe und/oder einen Druckspeicher, Ventile und elektrische Komponenten zur Ansteuerung der Komponenten, bspw. einen Mikrokontroller, enthalten.

Der Substanzträger (40) kann über einen entfernbaren Bereich (502) auf seiner Stirnfläche verfügen, welcher entfernt werden kann und welche ein Fenster in das Innere des Substanzträgers (40) freigeben kann. Durch das Fenster kann der Inhalt des Substanzträgers (40) mit der Herzoberfläche oder dem Inneren der Perikardhöhle in Kontakt kommen. Der entfernbare Bereich (502) des Substanzträgers (40) kann über eine dafür vorgesehene Vorrichtung von einem räumlich entfernten Punkt aus vom Substanzträger (40) entfernt werden. Dieser Punkt kann außerhalb des Körpers liegen. Ein Mechanismus kann zur Entfernung des entfernbaren Bereichs auf dem Substanzträger (40) führen.
In Figur 13 ist dieser Mechanismus mithilfe von Schnüren (504) ausgeführt. Beispielhafte Ausführungsformen können zumindest eine zur Entfernung des entfernbaren Bereichs vorgesehene Schnur, eine Faden oder ein Seil, bspw. als Teil eines Seilzugs, umfassen. Auch alternative Mechanismen sind denkbar. In dem vorliegenden Ausführungsbeispiel sind zwei Schnüre (504) mit den oben liegenden Ecken des entfernbaren Bereichs (502) verbunden. Durch Zug an den Schnüren (504) kann der entfernbare Bereich des Substanzträgers (40) entfernt werden. Die Schnüre (504) können als Teil des Kabelbaums ausgeführt sein und der Zug von außerhalb des Körpers stattfinden. Die Lage des entfernbaren Bereichs nach Entfernung vom Substanzträger (40) kann in einer Kavität der innerhalb des Epikards liegenden Fixierhülse sein. Alternativ ist der entfernbare Bereich (502) wie oben beschrieben zumindest teilweise aus einem bioabbaubaren und/oder permeablen Material gefertigt. Weitere Eigenschaften des entfernbaren Bereichs sind in vorangehenden Abschnitten beschrieben.

Der Substanzträger (40) kann weiter als zumindest ein befüllbares und/oder entleerbares Behältnis zum Speichern von Substanz (403) ausgeführt sein. Das zumindest eine Behältnis kann alternativ auch direkt an einen Substanzträger (40) gekoppelt sein, so dass Substanz (403) vom Behältnis (40) auf die Membran übertreten kann. Beispielsweise kann sich ein Behältnis (40) direkt an der Rückseite eines Substanzträgers (40) befinden und die Koppelstelle kann permeabel oder perforiert gestaltet sein. Ist das Behältnis (40) nicht im direkten Kontakt mit einem Substanzträger (40), so kann eine substanzleitende Verbindung vom Behältnis (40) zur Membran durch einen Kanal oder Schlauch hergestellt werden. Das Behältnis (40) kann über zumindest eine Zuleitung (408) verfügen, durch welche eine Substanz (403) oder ein Fluid, welches die einzubringende, zumindest eine Substanz (403) enthält, in das Behältnis (40) eingeleitet werden kann. Das Behältnis (40) kann auch über eine Ableitung (409) verfügen, worüber Substanz (403) aus dem Behältnis (40) aus dem Körper ausgeleitet werden kann.

Der Substanzträger (40) kann weiterhin zumindest einen Sensor (4010) umfassen. Der zumindest eine Sensor (4010) kann einen Parameter des Substanzträgers (40) und/oder des Inhalts des Substanzträgers (40) erfassen. Durch den zumindest einen Sensor (4010) erfassbare Größen können die Druck- oder Zugspannung im Substanzträger (40), der pH-Wert, die Temperatur, eine elektrische Spannung, die Osmolarität, die Sauerstoffkonzentration, die CO₂-Konzentration, die elektrische Leitfähigkeit, die optische Dichte, eine Wirkstoffkonzentration, das Vorhandensein von Flüssigkeit und/oder Substanz (403) oder eine Kombination daraus sein. Die Lage des zumindest einen Sensors (4010) am Substanzträger (40) kann hierbei derart sein, dass sie die optimale Erfassung des zu erfassenden Parameters des Substanzträgers (40) und/oder der darin enthaltenen Substanz (403) ermöglicht.

Der Substanzträger (40) kann weiter zumindest eine Nachstelleinrichtung (4011) zur Beeinflussung zumindest eines Parameters des Substanzträgers (40) und/oder der Substanz (403) umfassen. Im vorliegenden Ausführungsbeispiel ist exemplarisch eine Heizwendel (4011) dargestellt. Die zumindest eine Heizwendel (4011) kann zur Einstellung der Temperatur in der Substanz (403) oder am Substanzträger (40) verwendet werden. Hierdurch können bspw. die Wachstumsbedingungen für lebendige biologische Zellen beeinflusst werden.

Der Substanzträger (40) kann zumindest zwei Elektroden (4012) umfassen, welche über elektrische Leitungen (4013) mit einer Versorgungseinheit verbunden sein könne. Die zwei Elektroden (4012) können eine Nachstelleinrichtung (4011) sein. Über die Elektroden (4012) kann eine elektrische Spannung an den Substanzträger (40), die Membran und/oder die im Substanzträger (40) befindliche Flüssigkeit, welche die Substanz (403) beinhaltet, angelegt werden. Die Anlegung einer elektrischen Spannung kann die Eigenschaften der in dem Substanzträger (40) befindlichen Substanz (403) verändern. Die Anlegung einer elektrischen Spannung kann das Wachstum von im Substanzträger (40) befindlichen lebendigen biologischen Zellen beeinflussen.

In Figur 13 ist der Substanzträger (40) an der Stirnseite einer expandierbaren Einheit (21) befestigt. Mit Hilfe der expandierbaren Einheit (21) kann Kontakt zwischen dem Substanzträger (40) bzw. seines Inhalts und der Herzoberfläche durch Befüllung und Entleerung der expandierbaren Einheit (21) hergestellt werden. Mithilfe der zumindest einen expandierbaren Einheit (21), auf welcher der Substanzträger (40) befestigt ist, kann der Anpressdruck des Substanzträgers (40) gegen die Herzfläche über Zu- und Abfuhr eines Fluides über die pneumatische Leitung (213) eingestellt werden. Mithilfe der zumindest einen expandierbaren Einheit (21), auf welcher der Substanzträger (40) befestigt ist, kann der Anpressdruck des Substanzträgers (40) gegen die Herzfläche bei Bedarf nachgestellt werden. Ist die expandierbare Einheit (21) bspw. als inflatierbare Kammer aufgebaut, so kann der Anpressdruck gegen die Herzfläche durch Befüllen oder Ablassen eines unter Druck stehenden Fluides über die Leitung (213) geregelt werden. Die Befüllung mit Fluid oder das Ablassen des Fluides kann so geregelt werden, dass stets konstanter Anpressdruck des Substanzträgers (40) gegen den Herzmuskel herrscht. Alternativ können auch zeitlich und periodisch alternierende Druckmuster aufgebracht werden. Beispielsweise kann während der Systole ein geringerer Anpressdruck aufgebracht werden als während der Diastole oder entsprechend gegenteilig. Dies hat den Vorteil, dass das Verhalten der körpereigenen Zellen sowie der therapeutischen (Stamm-)Zellen in Abhängigkeit des Anpressdrucks beeinflusst werden kann und somit die therapeutische Effizienz verbessert werden kann.

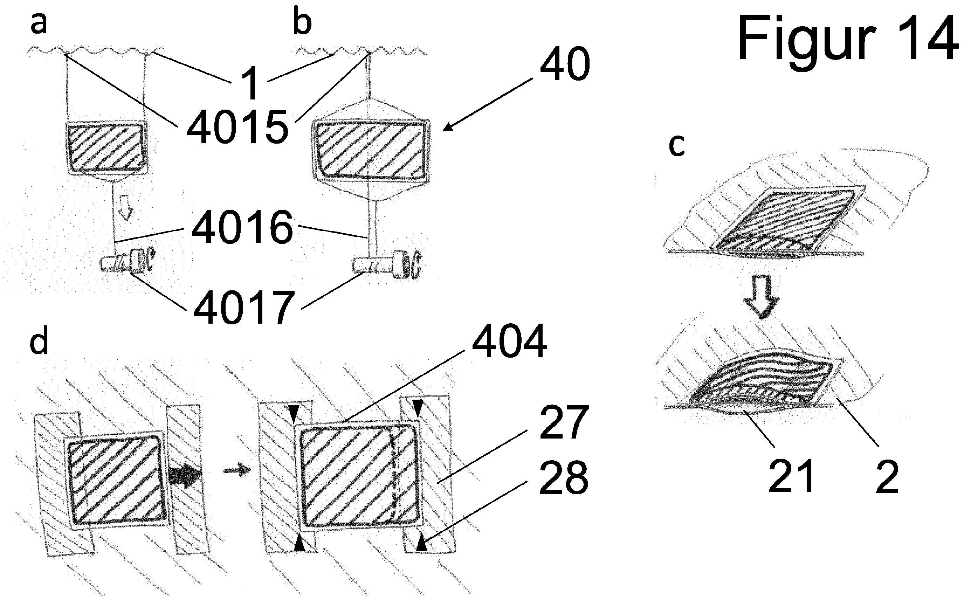

**Figur 14a** **bis d** zeigen verschiedene Ausführungsformen eines Substanzträgers (40), welcher einer mechanischen Belastung ausgesetzt werden kann. Eine mechanische Belastung des Substanzträgers (40) kann zu einer mechanischen Belastung der Membran (402) und des darauf befindlichen Substrats führen. Enthält das Substrat lebendige Zellen können durch die Beeinflussung der Zellphysiologie aufgrund der mechanischen Belastung unterschiedliche sogenannte Zytokine sekretiert werden. In Abhängigkeit der von den Zellen sekretierten Zytokinen kann bspw. die therapeutische Effizienz zur Regeneration von ischämischen Myokardgewebe nach einem Myokardinfarkt reguliert werden.

Figur 14a und b zeigen Ausführungsbeispiele, bei welchem die Zugbelastung des Substanzträgers (40) über einen mechanischen Nachstellmechanismus (4017) in Form von Kopplungselementen (4015), einem Zugelement (4016) und einer Winde (4017) eingestellt wird. Der Substanzträger (40) kann mit der Rahmenstruktur (1) gekoppelt sein. Diese Koppelung kann man in diesem Ausführungsbeispiel durch Kopplungselemente herstellen lassen, bspw. durch zumindest eine Strebe, einen Faden, eine Schnur oder zumindest einen Draht. Das zumindest eine Kopplungselement ist an einem Substanzträger (40) befestigt und kann an dessen Ende an die Rahmenstruktur (1), insbesondere an einem entlang des Umfangs verlaufenden Rahmensegment gekoppelt werden. An dem Substanzträger (40) kann weiter ein Zugelement (4016), bspw. ein Draht, ein Faden oder ein Kunststoffstreifen, befestigt sein, dessen anderes Ende an eine Winde gekoppelt ist. Über die Winde lässt dich der axiale Spannungszustand des Substanzträgers (40) einstellen. Durch die Einstellung eines axialen Spannungszustands kann das Wachstum von auf dem Substanzträger (40) befindlichen lebendigen biologischen Zellen und die Physiologie der lebendigen biologischen Zellen beeinflusst werden. Über die Winde kann an einem Ende des Substanzträgers (40) eine Zugbelastung aufgebracht werden. Je nach Ausführung des Seilzugs kann über die Winde auch an beiden Enden des Substanzträgers (40) eine Zugbelastung aufgebracht werden. Die Zugbelastung kann konstant sein. Die Zugbelastung kann auch transient sein, um bspw. eine pulsatile Belastung ähnlich einer physiologischen Belastung zu erzeugen.

Figur 14c zeigt eine weitere Ausführungsform einer Vorrichtung zur Einstellung einer mechanischen Belastung des Substanzträgers (40) mit Hilfe einer expandierbaren Einheit (21). Der Substanzträger (40) kann über einer expandierbaren Einheit (21), welche in die Hülle (2) eingearbeitet sein kann, angebracht werden. Im vorliegenden Ausführungsbeispiel ist die expandierbare Einheit (21) zur Einstellung der mechanischen Belastung als eine inflatierbare Kammer dargestellt. Eine expandierbare Einheit (21) kann den Substanzträger (40) gegen die Herzoberfläche pressen. Der Substanzträger (40) kann auch größer sein als eine expandierbare Einheit, womit die expandierbare Einheit (21) vom Substanzträger (40) zumindest teilweise überdeckt werden kann. In diesem Fall führt eine Expansion der expandierbaren Einheit zu einer Dehnung des Substanzträgers (40) und/oder der Substanzträgers (402) und der sich darauf befindlichen Substanz (403), wodurch eine mechanische Spannung aufgebaut werden kann. Der Betrag der erzeugten Dehnung und/oder Spannung kann über den Hub der Expansion der expandierbaren Einheit (21) geregelt werden.

Figur 14d zeigt ein Ausführungsbeispiel eines Substanzträgers (40), welcher einer mechanischen Belastung ausgesetzt werden kann. Der Substanzträger (40) kann vorgespannt auf der Hülle und/oder der Stirnfläche der zumindest eine expandierbare Einheit (21) befestigt werden. Der Substanzträger (40) kann auf seiner Unterseite zumindest ein flächiges Kopplungselement aufweisen, mit welchem eine vorzugsweise stoffschlüssige Verbindung zwischen dem Stubstanzträger (40) und der Oberfläche der Hülle (2) und/oder der zumindest einen expandierbaren Einheit (21) aufgebaut werden kann. Die stoffschlüssige Verbindung kann eine Klebeverbindung sein. Durch auf der Oberfläche der Hülle (2) und/oder der Stirnfläche der zumindest einen expandierbaren Einheit (21) angebrachten zumindest einen Markierung (28) kann der Spannungszustand gezielt eingestellt werden. Diese zumindest eine Markierung (28) kann anzeigen, wie weit der Substanzträger (40) gedehnt werden muss, um die einzustellende Spannung aufzubauen. Die zumindest eine Markierung (28) kann einen Abstand anzeigen. Die zumindest eine Markierung (28) kann auch die Umrisse des gedehnten Substanzträgers (40) abbilden, so dass bei der Befestigung der einzustellende Dehnungszustand sichergestellt ist. Alternativ kann zur Befestigung des vorgespannten Substanzträgers (40) auch eine formschlüssige Verbindung eingesetzt werden, um den einzustellenden Spannungszustand zu erzeugen. Die formschlüssige Verbindung kann eine Knopf-Öse-Verbindung, eine Druckknopfverbindung, eine Klettverschlussverbindung oder eine Haken-Öse-Verbindung zwischen den Kopplungselementen (27, 404) sein. Zur Vereinfachung der Dehnung des Substanzträgers (40) können Griffhilfen in Form von Schlaufen oder Flügeln am Substanzträger (40) vorgesehen sein, welche keine Kopplung mit der Hüllenoberfläche eingehen können. So kann an diesen Griffhilfen gezogen werden ohne die Kopplungsfläche oder -punkte durch Hautkontakt zu beeinträchtigen.

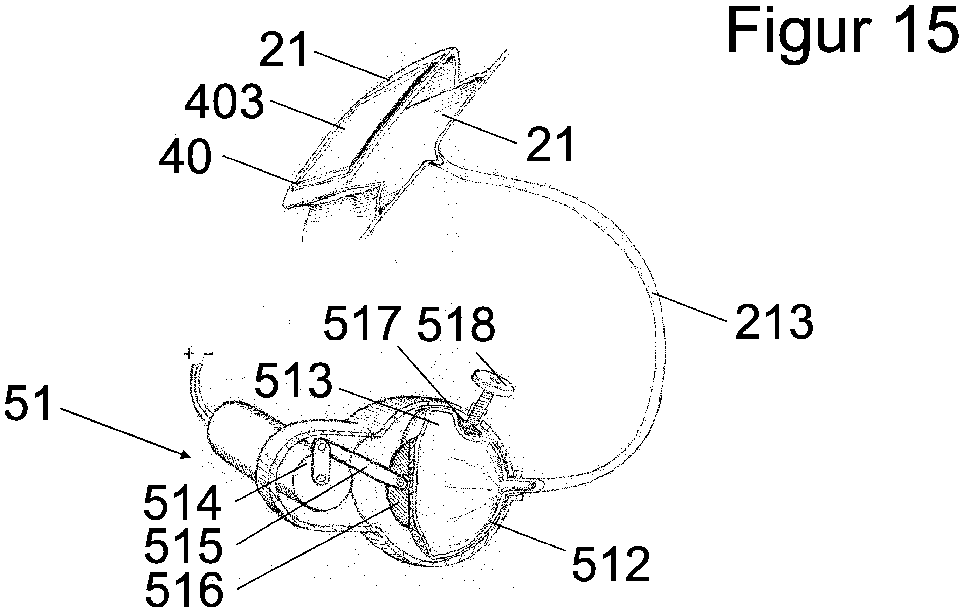

**Figur 15** zeigt eine Ausführungsform der zumindest einen Aktuatoreinheit (51), welche das Volumen der zumindest einen expandierbaren Einheit (21) über die pneumatische Leitung (213) einstellen kann. Ausführungsformen der erfindungsgemäßen Vorrichtung können über zumindest eine Aktuatoreinheit (51) verfügen. Es ist eine Ausführungsform der Aktuatoreinheit (51) dargestellt, welche ein mit einem Fluid gefülltes und von einer Membran (512) umgebenes Reservoirvolumen (513) (kann ein Gas und/oder eine Flüssigkeit sein), eine Antriebseinheit (514) (kann ein Elektromotor oder eine pneumatische Einheit sein), eine Pleuelstange (515) und einen Kolben (516) umfassen kann. Das Reservoirvolumen (513) kann durch eine Membran (512) begrenzt sein. Diese Membran (512) kann elastisch sein. Die Membran (512) kann aus dem gleichen Material sein wie die zumindest eine expandierbare Einheit (21). Die Membran (512) kann auch aus einem anderen Material als die zumindest eine expandierbare Einheit (21) sein. Die Membran (512) kann aus Polyurethan, Silikon oder Polytetrafluorethylen sein. Die Membran (512) kann eine ellipsoide Form haben. Die Membran (512) kann auch eine vom Ellipsoid abweichende Form haben. Der Kolben (516) kann Teil der Membran (512) sein. Der Kolben (516) kann auch als eigenständiges Teil ausgeführt sein. Das Reservoirvolumen (513) ist mit dem Volumen der zumindest einen expandierbaren Einheit (21) über eine Leitung verbunden. Das Reservoirvolumen (513) kann außerhalb des Körpers liegen. Das Reservoirvolumen (513) kann auch innerhalb des Körpers liegen. Das Reservoirvolumen (513) kann in der Versorgungseinheit untergebracht sein. Das Volumen der zumindest einen expandierbaren Einheit (21) des Implantats kann das Kammervolumen (215) einer inflatierbaren, balgförmigen Kammer sein. Das Reservoirvolumen (513) kann größer sein als das Kammervolumen (215). Das Gesamtvolumen, bestehend aus dem Reservoirvolumen (513), dem Kammervolumen (215) und dem Volumen der Leitung, ist abgeschlossen und mit einem Fluid befüllt. Das Fluid kann ein Gas und/oder eine Flüssigkeit sein.

Der Kolben (516) kann das Reservoirvolumen (513) verringern. Der Kolben (516) kann das Reservoirvolumen (513) durch Eindrücken der Membran (512) verringern. Der Kolben (516) kann das Eindrücken oder das Entlasten der Membran (512) durch eine translatorische Bewegung erreichen. Das Eindrücken oder das Entlasten der Membran (512) durch den Kolben (516) kann auch durch eine von einer translatorischen Bewegung abweichende Bewegung des Kolbens (516) erreicht werden. Das Eindrücken oder das Entlasten der Membran (512) kann durch eine Kombination translatorischer und rotatorischer Bewegungen erreicht werden. Durch die Veränderung des Reservoirvolumens (513) kann aufgrund der Abgeschlossenheit des Gesamtvolumens das Kammervolumen (215) verändert werden. Dadurch kann der Druck der zumindest einen expandierbaren Einheit (21)auf die Herzoberfläche eingestellt werden.

Der Kolben (516) kann durch eine dafür vorgesehene Vorrichtung bewegt und dazu gebracht werden, die Membran (512) des Reservoirvolumens (513) einzudrücken oder zu entlasten. Die Vorrichtung kann eine Exzenterscheibe sein. Diese Vorrichtung kann in seiner Form auch von einer Exzenterscheibe abweichen. Diese Vorrichtung kann eine Kurbelwelle sein. Die Vorrichtung kann zum Zwecke der Bewegung des Kolbens (516) eine rotatorische Bewegung ausführen. Die Vorrichtung kann zum Zwecke der Bewegung des Kolbens (516) auch eine translatorische Bewegung oder eine Kombination aus translatorischer und rotatorischer Bewegung ausführen. Durch Bewegung der Vorrichtung kann der Kolben (516) bewegt werden, welcher das Reservoirvolumen verändern kann.

Die Vorrichtung kann auch identisch mit dem Kolben (516) sein. In solch einer Ausführungsform kann die Vorrichtung dazu genutzt werden, das Reservoirvolumen (513) zu verändern.

Eine Aus führungs form der Antriebseinheit (514) der zumindest einen expandierbaren Einheit (21) kann eine Vorrichtung zur Bewegung des Kolbens (516) umfassen. Diese Vorrichtung zur Bewegung des Kolbens (516) kann eine Exzenterscheibe sein, welche durch eine Motoreinheit bewegt werden kann. Diese Motoreinheit kann ein Elektromotor sein. Die Motoreinheit kann auch eine pneumatische Einheit sein. Die Exzenterscheibe kann auf der Antriebswelle der Motoreinheit platziert sein. Die Antriebswelle der Motoreinheit kann axial rotieren. Die Stellgröße der Motoreinheit kann die Drehzahl der Welle sein. Die Stellgröße der Motoreinheit kann auch ein translatorischer Hub sein. Die Motoreinheit kann durch eine translatorische Hubbewegung das Reservoirvolumen (513) verändern. Die Motoreinheit kann einen zeitlich konstanten Hub einstellen. Die Motoreinheit kann auch einen zeitlich veränderlichen Hub einstellen.

Eine Aus führungs form der Aktuatoreinheit (51) kann auch über eine weitere Einstellmöglichkeit (517) des Reservoirvolumens (513) aufweisen. Diese Einstellmöglichkeit (517) kann statisch sein. Die Einstellmöglichkeit kann ein Hubelement sein. In der vorliegenden Ausführungsform kann das Hubelement (517) über einen Drehknopf (518) eingestellt werden. Die Einstellung des Hubelements (517) kann auch über ein von einem Drehknopf abweichenden Bedienelement durchgeführt werden, bspw. durch einen Schieberegler, welcher die Einstellung des Hubelements steuern kann.

Figur 15 zeigt eine Ausführungsform der Aktuatoreinheit (51), bei welcher die Exzenterscheibe durch eine exzentrisch an der Motorwelle angebrachte Pleuelstange (513) ausgeführt ist. Die Lage der Pleuelstange (513) wird durch eine Motoreinheit (514) eingestellt, bei welcher es sich um einen Elektromotor handeln kann. Das nicht exzentrisch an der Motorwelle angebrachte Ende der Pleuelstange (513) kann über ein Zwischenstück mit der Membran (512) des Reservoirvolumens (513) gekoppelt sein. Die Kopplung kann durch Kleben, Schweißen oder Vulkanisieren oder eine andere Fügetechnik oder Kopplungsform erfolgen. Eine Veränderung der Lage der Pleuelstange (513) kann eine Veränderung des Reservoirvolumens (513) bewirken, womit sich das Kammervolumen (215) der zumindest einen expandierbaren Einheit (21) verändern kann. Das Reservoirvolumen (513) kann größer sein als das Kammervolumen (215). Die Motoreinheit kann mittels des zur Energieversorgung des Implantats in der Versorgungseinheit enthaltenen Energiespeichers betrieben werden.

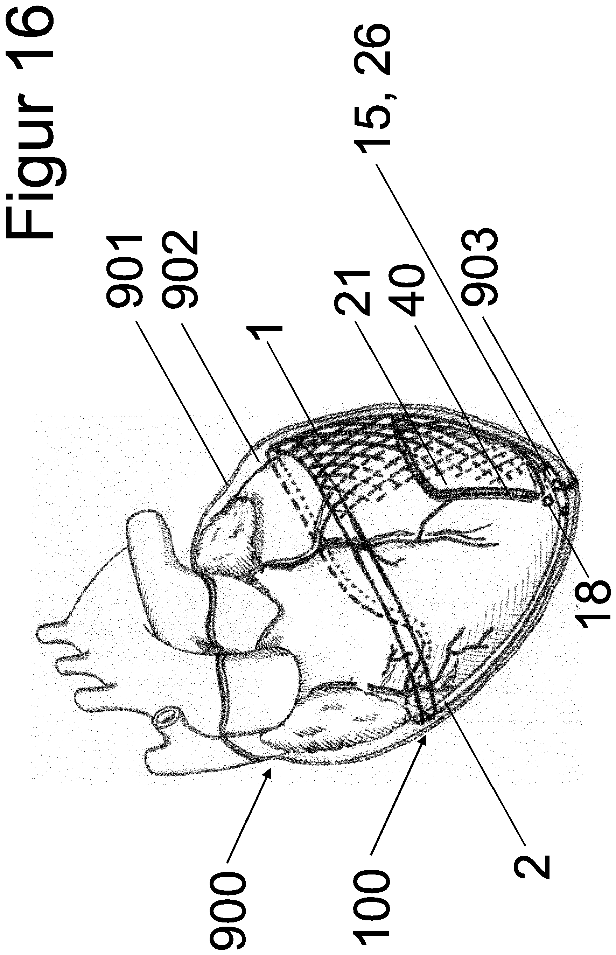

**Figur 16** zeigt eine Ausführungsform des Implantats (100), welches nur aus der Rahmenstruktur (1), zumindest einer Hülle (2), der zumindest einen expandierbaren Einheit (21) sowie zumindest einem Substanzträger (40) mit zumindest einer Substanz bestehen kann. Somit kann das Implantat vollständig in den Körper bzw. durch eine chirurgisch erzeugte Öffnung (903) im Perikard (901) in die Perikardhöhle (902) implantiert werden, ohne dass ein Austritt durch eine künstliche Körperöffnung, eine Perikardschleuse oder einen Kabelbaum realisiert werden muss. Vorteil dieser Ausführungsform ist die geringere Infektionsgefahr durch Vermeidung einer künstlichen Körperöffnung, ihre einfache Implantierbarkeit, da das Perikard wieder zugenäht werden kann, sowie die einfache Bauform, welche material- und kostensparend sein kann.

Die zumindest eine expandierbare Einheit (21) kann bspw. eine pneumatische Einheit in Form eines mit einem Fluid befüllbaren Kissens sein, welche mit einem statischen Druck beaufschlagt sein kann. Dadurch kann der stete Kontakt zwischen Herzoberfläche und dem auf der zumindest einen expandierbaren Einheit (21) angebrachten zumindest einen Substanzträger (40) gewährleistet werden. Alternativ kann die expandierbare Einheit (21) auch als Schwamm oder mithilfe einer Feder, bspw. aus Nitinol, ausgeführt sein und durch diese Feder ein bestimmter Expansionsgrad eingestellt werden.

Die zumindest eine Hülle (2) kann zumindest eine Öffnung (26) aufweisen, wodurch der Flüssigkeitsaustausch innerhalb der Perikardhöhle (902) gewährleistet werden kann. Auch die Rahmenstruktur (1) kann mit der Hülle (2) deckungsgleich eine Öffnung (15) aufweisen.

Die Rahmenstruktur (1) kann über zumindest einen Kopplungspunkt (18) an dem der Herzspitze nahen Ende aufweisen, welche mit einem dafür geeigneten Explantationsbesteck oder einer Explantationsvorrichtung ähnlich der in einem vorangegangenen Abschnitt beschriebenen minimal-invasiv aus dem Körper entfernt werden kann.

Weitere vollständig in den Körper implantierbare Ausführungsformen können nur aus einer Rahmenstruktur (1) mit zumindest einer expandierbaren Einheit (21) und/oder nur zumindest einem Substanzträger (40) mit zumindest einer Substanz bestehen, ohne dass das Implantat eine Hülle (2) umfasst. Noch weitere Ausführungsformen können aus einer Hülle (2) mit zumindest einer expandierbaren Einheit (21) und/oder nur zumindest einem Substanzträger (40) mit zumindest einer Substanz bestehen, ohne dass das Implantat eine Rahmenstruktur (1) umfasst. In diesem Fall kann die Wandstärke der Hülle (2) so dick gestaltet sein, dass allein die Hülle (2) ohne Rahmenstruktur (1) ausreichend steif ist, um im Körper in der richtigen Passform zu bleiben. Beispielsweise kann eine solche Hülle (2) aus Silikon mit einer Wandstärke von 0.5 mm bis 5 mm, oder einer Wandstärke von 1mm bis 3mm, gefertigt sein.

In manchen Ausführungsformen kann das Implantat (100) zumindest zu einem Teil und aus zumindest einem bioabbaubaren Material gefertigt sein. Vorteil der Ausführung eines Teils des Implantats (100) aus einem bioabbaubaren Material, bspw. im Falle einer bioabbaubaren Rahmenstruktur (1), ist die einfachere Explantation, da die steife Rahmenstruktur (1) nicht mehr aus dem Körper entfernt werden muss. Vorteil eines komplett aus bioabbaubaren Materialien gefertigten Implantats (100) ist, dass keine Notwendigkeit der Explantation mehr besteht.

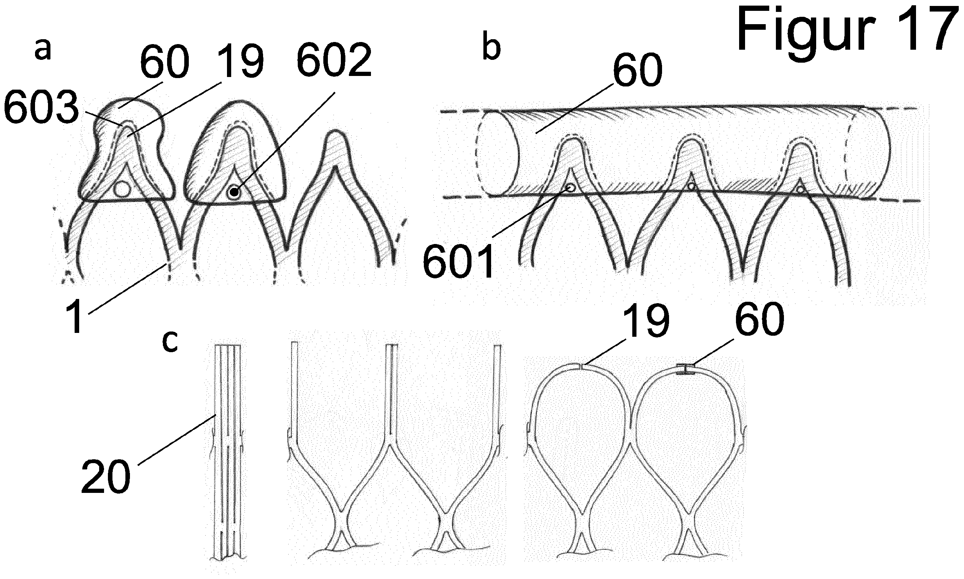

**Figur 17** zeigt Geometrien der Rahmenstruktur (1), die derart ausgeführt sein können, dass spitze und scharfkantige Teile der Rahmenstruktur (1), oftmals an den Enden (19) der Rahmenstruktur (1) verortet, das Risiko eines Durchtritts durch die die Rahmenstruktur (1) zumindest teilweise umhüllende Hülle oder einer Verletzung von Körpergewebe erhöhen.

Figur 17 zeigt verschiedene Ausführungsformen von Endstücken (60), welche der Aufgabe dienen können, spitze oder scharfkantige Teile der Rahmenstruktur (1) abzudecken. Dies kann vorteilhaft sein, da dadurch das Risiko eines Durchtritts der Rahmenstruktur (1) durch die über sie gestülpte Hülle (nicht dargestellt) minimiert werden kann. Des Weiteren kann die Gefahr beseitigt werden, dass Teile einer potentiell spitzen oder scharfkantigen Geometrie der Rahmenstruktur (1) Körpergewebe verletzen.

Figur 17a zeigt die Geometrie einer Rahmenstruktur (1), über welche Endstücke (60) gestülpt werden können. Diese Endstücke (60) können aus einem elastischen, bevorzugt weichen Material bestehen. Die Endstücke (60) können aus einem Kunststoff oder einem Polymer bestehen. Beispielsweise können die Endstücke (60) aus Silikon oder Polurethan bestehen. Die Endstücke (60) können auch aus Materialien abweichend von Silikon oder Polyurethan bestehen. Die Endstücke (60) können alternativ aus einem Kunststoffschaum bestehen. Aufgabe der Endstücke (60) ist es, den Durchtritt der Rahmenstruktur (1) durch die über sie gestülpte Hülle und/oder die Verletzung von Körpergewebe durch die Rahmenstruktur (1) zu vermeiden.

Die Endstücke (60) können mit der Rahmenstruktur (1) gekoppelt werden. Kopplungsmöglichkeiten sind bspw. kraftschlüssige, stoffschlüssige und/oder formschlüssige Verbindungen. Die Endstücke (60) können bspw. auf die Rahmenstruktur (1) geklebt werden. Sie können mit der Rahmenstruktur (1) verklebt werden. Sie können aber auch durch eine Öffnung der Rahmenstruktur (1) hindurch mit sich selbst verklebt werden. Alternativ kann die Befestigung auch mithilfe von Stiften oder Knöpfen (602) ausgeführt sein. Für eine Verbindung mit Knöpfen können die hierfür notwendigen zumindest zwei komplementären Koppelstellen (601) Teil des Endstücks (60) sein. Alternativ kann ein Endstück (60) auch nur auf die entsprechende Stelle der Rahmenstruktur (1) aufgesetzt sein und im Anschluss mit der Hülle gekoppelt, z.B. verklebt werden.

Die Endstücke (60) können zumindest eine Kavität (603) aufweisen, welche dazu geeignet sein kann, einen Teil der Rahmenstruktur (1) hineinzuführen. Dadurch kann der Halt eines Endstücks (60) auf der Rahmenstruktur (1) verbessert werden.

Figur 17b zeigt eine weitere Ausführungsform eines Endstücks (60), welche sich in Form eines zumindest teilweisen Rings über zumindest einen Teil des Umfangs der Rahmenstruktur (1) legen lassen kann. Das Material des Endstücks (60) kann stark elastisch deformierbar und flexibel sein, so dass es im Zuge der Überführung des Implantats von einem expandierten in einen nicht-expandierten Zustand sowie umgekehrt ohne Schaden an sich selbst und die Beschädigung anderer Bauteile sowie des Körpergewebes bewerkstelligen kann.

Figur 17c zeigt eine Geometrie der Rahmenstruktur (1) mitsamt einem dazugehörigen Endstück (60), bei welchem durch eine geeignete Schnittführung an der Ausgangsgeometrie, dem geschlitzten Rohr (20), eine atraumatische Form hergestellt werden kann. Diese Form minimiert das Risiko der Verletzung von Körpergewebe durch Eindringen, Aufspießen, Schneiden oder Kratzen der Rahmenstruktur (1) sowie das Risiko der Durchbruchs der Rahmenstruktur (1) durch die zumindest eine die Rahmenstruktur (1) umgebende Hülle. Die Schnittführung kann bspw. derart ausgeführt sein, dass die jeweils letzten Schlitze an zumindest einem Ende (19) des geschlitzten Rohres soweit ausgeführt sind, dass zwischen den durch die Schlitze entstehenden Streben keine materielle Verbindung mehr vorhanden ist. Es können dadurch freistehende Enden entstehen. Bei der Aufweitung der Struktur, im Zuge derer die expandierte Geometrie (wie in einem vorangegangenen Abschnitt beschrieben) durch Wärmebehandlung eingeprägt wird, können die frei stehenden Enden der Rahmenstruktur (1) derart verformt werden, dass die eingeprägte Geometrie im expandierten Zustand an den Enden derart gewölbt sind, dass diese Enden eine atraumatische Form annehmen. Zusätzlich können diese bereits atraumatisch geformten Enden auch mit Endstücken (60) von passender Gestalt abgedeckt und abgesichert sein. Dadurch lassen sich die oben beschriebenen Risiken weiter verringern.

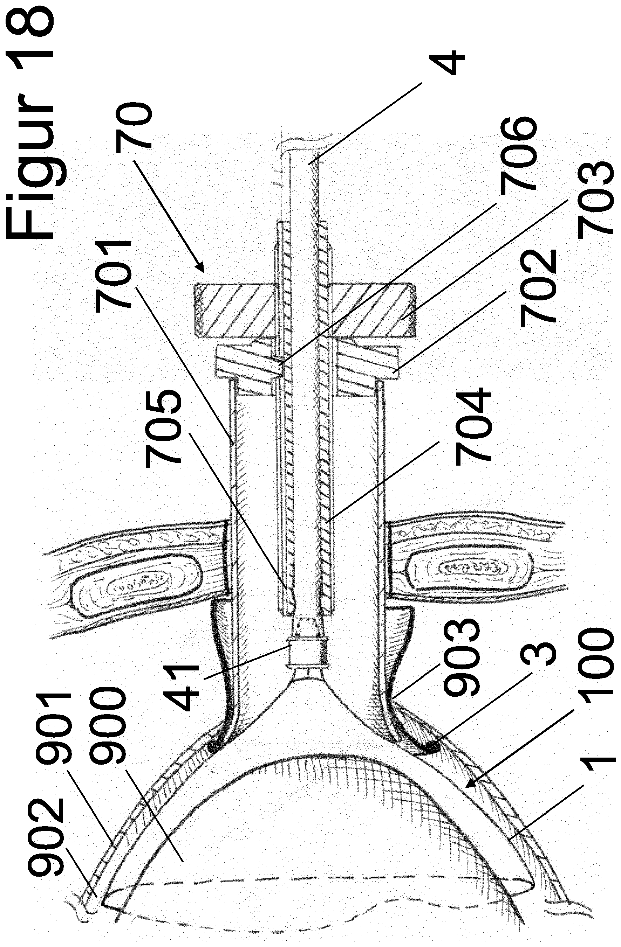

**Figur 18** zeigt einen Schritt während der Explantation des Implantats (100). Eine Ausführungsform der Explantationsvorrichtung (70) kann aus einem zylindrischen Explantationsrohr (701) bestehen. Das Explantationsrohr (701) hat ein proximales, dem Operateur zugewandtes Ende und ein distales Ende, das in den Patienten eingeführt wird. Das zylindrische Explantationsrohr (701) weist am distalen Ende eine radiale Aufweitung auf. Des Weiteren kann die Explantationsvorrichtung (70) ein Zugelement (704) mit Außengewinde mit einer Klemme (705) am distalen Ende des Zugelements (704), einem Endstück (702) mit Axialbohrung sowie einer Mutter (703) bestehen.

Wie in Figur 18 gezeigt, ermöglicht eine Thorakotomie oder eine Mini-Thorakotomie den Zugang zur implantierten Vorrichtung. Eine durch eine Perikardschleuse (3) gewährleistete Versiegelung einer Perikardöffnung (903) kann aufgehoben werden, um die Explantationsvorrichtung zu positionieren. Die Perikardschleuse (3) kann dabei an ihrer Position im Körper verbleiben, da diese den Explantationsvorgang nicht behindert. Alternativ kann die Perikardschleuse (3) entfernt werden, da sie andererseits für die Explantation nicht benötigt wird. Zumindest ein Teil des Kabelbaums (4) kann zur Vereinfachung der Prozedur abgetrennt werden, beispielsweise durch Abschneiden oder Abzwicken. Für die Explantation des Implantats (100) kann zunächst das Explantationsrohr (701) in den Körper eingeführt werden, so dass das distale Ende des Rohrs sich innerhalb der Perikardhöhle und/oder einer ggf. vorhandenen Perikardschleuse (3) befindet. Der vom Implantat (100) wegführende Kabelbaum (4) oder der verbliebene Teil eines abgetrennten Kabelbaums (4) kann durch das Innere des Explantationsrohrs (701) hindurch geführt werden. In das Explantationsrohr (701) kann von außen ein Zugelement (704) in das Explantationsrohr (701) geführt werden. Das Zugelement (704) kann ein inneres Lumen haben, durch das zumindest ein Teil des Kabelbaums (4) geführt werden kann. Das Zugelement (704) kann ein Gewinde umfassen, beispielsweise ein Außengewinde. Am vorderen Ende des Zugelements (704) kann sich eine Klemme (705) befinden, welche den Kabelbaum (4) zumindest teilweise umschließt und das fest an den Kabelbaum (4) gekoppelt werden kann. Alternativ zur Klemme (705) am Kabelbaum (4) kann sich am Zugelement (704) auch eine Klemmvorrichtung befinden, die an die Fixierhülse (41) des Kabelbaums (4) gekoppelt werden kann. Das Zugelement (704) und die Klemme (705) können zwei oder mehrere Teile sein, kann aber auch nur aus einem Teil bestehen. Weiter kann die Explantationsvorrichtung (70) ein Endstück (702) mit Öffnung, beispielsweise eine Bohrung, umfassen, welches auf das proximale Ende des Explantationsrohrs (701) aufgesetzt werden kann. Das Endstück (702) kann derart an das Explantationsrohr (701) gekoppelt werden, bspw. mithilfe eines Zentrierdorns (706), dass das Endstück (702) nicht gegen das Explantationsrohr (701) verdreht werden kann. Das Zugelement (704) kann durch die Öffnung des Endstücks (702) geführt werden. Das Zugelement (704) und das Endstück (702) können gegen Verdrehung gegeneinander gesichert werden. Weiter kann die Explantationsvorrichtung eine Mutter (703) enthalten, beispielsweise eine Rändelmutter, welche ein Innengewinde aufweist, das mit dem Außengewinde des Zugelements (704) in Eingriff gebracht werden kann. Durch Drehen an der Mutter (703) kann dabei eine Zugkraft auf das Zugelement (704) ausgeübt werden, welches wiederrum an den Kabelbaum (4)/das Implantat (100) gekoppelt ist, und die dazu dient das Implantat (100) in das Explantationsrohr (701) hinein zu ziehen. Der Vorteil dieser Explantationsvorrichtung (70) ist, dass die benötigte Zugkraft von der Mutter (703), dem Zugelement (704) und der Klemme (705) auf das Implantat (100) ausgeübt wird und die Gegenkraft als Druckkraft vom Implantat (100) auf das Explantationsrohr, das Endstück (702) und die Mutter (703) aufgebracht wird. Die Explantationsvorrichtung (70) ist damit nach außen kraftfrei, d.h. es werden während der Explantation keine Kräfte (bzw. zum Abstützen oder ähnlich) auf das Herz oder den Körper des Patienten ausgeübt. Befindet sich das Implantat (100) dann weit genug im Inneren des Explantationsrohrs (701), so kann die gesamte Explantationsvorrichtung (70) mitsamt dem Implantat (100) aus dem Körper genommen werden. Das Perikard und die für die Explantation benötigte Thorakotomie können anschließend geeignet verschlossen werden.

Wie in Figur 18 gezeigt, kann das distale Ende des Explantationsrohrs (701) durch die Perikardschleuse (3) hindurch in die Perikardhöhle hineingeführt werden. Das Innere des Explantationsrohrs (701) definiert den Explantationskanal, in welchen das Implantat (100) während der Explantation zumindest teilweise hineingezogen wird. Das Implantat (100) kann zum Zwecke der Explantation in einen komprimierten Zustand überführt werden Das Explantationsrohr (701) kann am distalen Ende eine radiale, trichterförmige Aufweitung aufweisen. Diese Aufweitung ermöglicht eine verletzungsarme Explantation des Implantats (100), indem beispielsweise verhindert wird, dass sich Teile des Implantats (100) am distalen Ende einhaken, verkanten und/oder blockieren. Die Aufweitung kann weiter dazu dienen, dass das Implantat (100) beim Einziehen in das Explantationsrohr (701) definiert komprimiert wird. Der Öffnungswinkel der radialen Aufweitung kann zwischen 0° und 150° liegen, zwischen 10° und 90° oder zwischen 20° und 60° liegen. Die Länge des Explantationsrohrs (701) kann zwischen 4 cm und 40 cm liegen, sie kann zwischen 6 cm und 25 cm liegen. Die Länge des Explantationsrohrs (701) kann so gewählt sein, dass das Implantat (100) im komprimierten Zustand teilweise oder vollständig im Rohr aufgenommen werden kann. Der Innendurchmesser des Explantationsrohrs (701) kann zwischen 2 cm und 5 cm liegen, er kann zwischen 3 cm und 4 cm liegen. Die Wandstärke des Explantationsrohrs (701) kann zwischen 0.1 mm und 3 mm liegen, sie kann zwischen 0.5 mm und 1.5 mm liegen. Die Wandstärke kann zum distalen Ende des Explantationsrohrs (701) abnehmen, um den Einzug des Implantats (100) in das Explantationsrohr (701) zu vereinfachen. Die Klemme (705) kann zumindest einteilig ausgeführt sein. Sie kann jedoch auch mehrteilig ausgeführt sein, damit sie um den Kabelbaum (4) oder die Fixierhülse gelegt werden kann. Der Innendurchmesser der Klemme (705) kann mindestens so groß sein wie der Außendurchmesser des Kabelbaumes oder der Fixierhülse. Der Innendurchmesser der Klemme (705) kann veränderlich sein. Die Klemme (705) kann ähnlich einer Schlauchschelle gestaltet sein. Beispielsweise kann eine Klemme (705) verwendet werden, deren Innendurchmesser größer ist als der Außendurchmesser des Kabelbaums (4) oder der Fixierhülse, so dass die Klemme (705) einfach über den Kabelbaum (4)/die Fixierhülse geführt werden kann. Sobald die Klemme (705) dann an der angestrebten Position sitzt kann dann der Innendurchmesser verkleinert werden, um die Klemme (705) zum Kabelbaum (4)/zur Fixierhülse zu koppeln, beispielsweise ähnlich dem Funktionsprinzip einer Schlauchschelle. Durch eine Durchmesserverringerung kann der Sitz und die Rutschfestigkeit der Klemme (705) in Bezug auf den zu greifenden Kabelbaum (4) / die zu greifende Fixierhülse erhöht werden. Eine zusätzliche Verbesserung von Sitz und Rutschfestigkeit kann durch eine Strukturierung der Innenwand der Klemme (705) erzielt werden, etwa in Form von Zähnen, Zacken oder Rillen. Die Wandstärke der Klemme (705) kann zwischen 0.1 mm und 2 mm liegen, sie kann zwischen 0.3 mm und 1 mm liegen. In der gezeigten Ausführungsform in Figur 18 sind die Klemme (705) und das Zugelement (704) als ein Teil ausgeführt. Alternativ kann das Zugelement (704) mit der Klemme (705) ähnlich einer Spannzange ausgeführt sein durch die der Kabelbaum (4) hindurch geführt werden kann. Das längliche Zugelement (704) mit Klemme (705) kann dabei beginnend am distalen Ende zumindest einen Schlitz in Längsrichtung aufweisen, der sich zumindest über ein Viertel der Gesamtlänge des Zugelements (704) erstreckt. Der Außendurchmesser des Zugelements (704) im geschlitzten Bereich kann dabei zum distalen Ende hin zunehmen, so dass der geschlitzte Bereich des Zugelements (704) beim Einschrauben in das Endstück (702) zusammengedrückt wird und beispielsweise der Kabelbaum (4) oder die Fixierhülse geklemmt wird. Diese Ausführungsform hat den Vorteil, dass die Klemme (705) nicht manuell vom Bediener an einen Teil des Implantats (100) gekoppelt werden muss, da sich eine Klemme (705) in Form einer Spannzange automatisch zusammen zieht, sobald der geschlitzte Bereich des Zugelements (704) mit sich vergrößerndem Außendurchmesser vom Endstück (702) der Explantationsvorrichtung (70) komprimiert wird. Je weiter das geschlitzte Zugelement (704) dabei in das Endstück (702) geschraubt wird, desto größer kann die Klemmkraft werden.

Das Endstück (702) hat unter anderem die Aufgabe, eine relative Verdrehung von Explantationsrohr (701) und Zugelement (704) zueinander zu verhindern und eine Drehung an der Mutter (703) in eine translatorische Bewegung des Zugelements (704) umzuwandeln. Beispielsweise mittels einer Nut-Feder-Verbindung, wie in Figur 18 dargestellt, in welcher das Zugelement (704) eine Längsnut aufweist, in welche eine Nase des Endstücks (702) eingreift. Alternativ kann das Endstück (702) statt einer Nase auch einen Nutenstein oder ein anderes Führungselement aufweisen. Das Endstück (702) kann sich auch nicht gegen das Explantationsrohr (701) verdrehen. Ohne das Endstück (702) könnte eine Drehung an der Mutter (703) eine Verdrehung des Zugelements (704) hervorrufen, was eine Verdrehung des Implantats (100) und ggf. eine Verletzung des Patienten verursachen könnte. Das Endstück (702) ist zumindest einteilig ausgeführt. Es kann jedoch auch mehrteilig ausgeführt sein, damit es einfacher um den Kabelbaum (4) gelegt werden kann. Das Endstück (702) kann an das proximale Ende des Explantationsrohrs (701) gekoppelt werden, beispielweise durch zentriertes Aufsetzen, Verschrauben oder einen Bajonettverschluss. Die Klemme (705) mit dem Zugelement (704) ragt in der gezeigten Ausführungsform der Explantationsvorrichtung (70) durch die zentrische Axialbohrung des Endstücks (702) hindurch. Die Kopplung des Endstücks (702) mit dem Explantationsrohr (701) kann gegen die relative Verdrehung von Explantationsrohr (701) und Endstück (702) gesichert sein, bspw. durch einen Zentrierdorn, einen Splint, eine Schraube, einen Bolzen oder eine Nut in die ein Eingreifelement gekoppelt werden kann. Der Durchmesser der zentrischen Axialbohrung kann von der Größenordnung des Außendurchmessers des Zugelements (704) sein. Die in der Zeichnung aufgezeigten Außendurchmesser des Endstücks (702) sind ähnlich den Durchmessern des Explantationsrohrs (701) in der Art, das ein sicherer Sitz des Endstücks (702) auf dem Explantationsrohr (701) gewährleistet ist. Die drehbare Mutter (703) kann nun auf die Kabelbaumklemme aufgeschraubt werden. Sobald die Mutter (703) mit dem Endstück (702) in Kontakt tritt, kann durch sukzessives Drehen der Mutter (703) die implantierte Vorrichtung (bestehend aus der Fixierhülse, der Rahmenstruktur, der darin befindlichen Hülle, der expandierbaren Einheiten, Leitungen, Sensoren, etc.) aus dem Perikard herausgezogen werden. Der Außendurchmesser der Mutter (703) soll die bequeme Bedienung der Explantationsvorrichtung (70) ermöglichen, so dass er mit einer Hand eines Erwachsenen zumindest zur Hälfte umgriffen werden kann. Der Außendurchmesser der Mutter (703) kann zwischen 3 cm und 15 cm liegen, er kann zwischen 6 cm und 12 cm liegen. Die Höhe der Mutter (703) kann zwischen 5 mm und 50 mm liegen, sie kann zwischen 10 mm und 30 mm liegen. Das Gewinde, welches die Explantation ermöglicht, sollte derart ausgelegt sein, dass eine Ausbringung des Implantats (100) mit einer für den Operateur vertretbaren Anzahl Drehungen der Rändelmutter stattfinden kann. Das Gewinde kann ein eingängiges oder ein mehrgängiges Gewinde sein. Durch die Verwendung eines mehrgängigen Gewindes kann die Gewindesteigung erhöht werden, womit pro Umdrehung der Mutter (703) eine größere translatorische Bewegung am Zugelement (704) umgesetzt wird. Das Gewinde kann ein Bewegungsgewinde sein, beispielsweise ein Trapezgewinde. Bewegungsgewinde haben im Gegensatz zu Befestigungsgewinden den Vorteil, dass die Reibungskräfte geringer sind und/oder keine Selbsthemmung auftritt, womit die Mutter (703) vom Bediener leichter gedreht werden kann als bei einem Befestigungsgewinde (wie z.B. metrisches oder zölliges Regelgewinde). Die Explantation mit der vorliegenden Ausführungsform einer Explantationsvorrichtung (70) hat den Vorteil, dass der Schraubmechanismus eine dosierbare und nicht auf großen translatorischen Bewegungen der Explantationsvorrichtung (70) beruhende Explantation ermöglicht, was wiederum das Trauma für den Patienten reduziert.

Alternativ zum oben beschriebenen Explantationsvorgang kann der Kabelbaum (4) auch in Höhe der Enden der Verlängerungsstreben abgeschnitten werden. Im Falle von Ausführungsformen des Implantats (100), bei welchen die Verlängerungsstreben, wie in einem vorangehenden Abschnitt beschrieben, an den Enden Haken, Ösen, Schlaufen oder abgewinkelte Enden aufweisen, kann das Implantat (100) an diesen herausgezogen werden. Hierfür kann eine Ausführungsform der Explantationsvorrichtung (70) verwendet werden, welche alternativ oder zusätzlich zur Klemme (705) am Zugelement (704) noch Seilzüge, Drähte und/der Gestänge aufweist. Die Seilzüge, Seile, Gestänge können Teil des Zugelements (704) sein oder mit dem Zugelement (704) gekoppelt sein. Am anderen Ende können die Seilzüge, Seile und/oder Gestänge der Explantationsvorrichtung (70) an die Haken, Ösen, Schlaufen oder abgewinkelten Enden der Verlängerungsstreben gekoppelt werden können. Damit kann das Implantat (100) wie zuvor in den vorangegangenen Ausführungsformen zumindest teilweise in das Explantationsrohr (701) hineingezogen werden und aus dem Perikard und aus dem Körper des Patienten herausbefördert werden.

Allgemein können Ausführungsformen der Explantationsvorrichtung (70) so geartet sein, dass sie eine Ausführungsform des Implantats (100) durch Rückführung in dessen nicht expandierten Zustand einfacher aus dem Perikard explantierbar machen.

Im Falle von Ausführungsformen des Implantats (100), bei welchen das Implantat (100) ohne Perikardschleuse (3), Kabelbaum (4) und Versorgungseinheit vollständig in die Perikardhöhle (902) eingesetzt wird, kann das zumindest eine Zugelement (704) anstelle einer Klemme (705) an seinem distalen Ende eine Kopplungsmöglichkeit aufweisen, welche komplementär ist zu der für die Explantation vorgesehenen, zumindest einen Kopplungsmöglichkeit an der Rahmenstruktur (1) des Implantats (100). Die Kopplung kann bspw. als Haken-Öse-Verbindung ausgeführt sein. Auch andere Ausführungsformen der Kopplung sind denkbar. Eine genauere Beschreibung eines vollständig in die Perikardhöhle (902) implantierbaren Implantats (100) ist in einem vorangegangenen Abschnitt näher beschrieben.

## Patentansprüche

1. Vorrichtung zur Applikation einer Substanz auf eine Herzoberfläche umfassend:
eine Rahmenstruktur (1),
eine Hülle (2),
ein Substanzträger (40) und
eine zu applizierende Substanz (403),
wobei der Substanzträger (40) eine Tasche (50) ist und die zu applizierende Substanz (403) in die Tasche (50) eingebracht ist,
**dadurch gekennzeichnet, dass** die Tasche (50) einen entfernbaren Bereich (502) auf der herzzugewandten Fläche der Tasche (50) hat.

2. Vorrichtung nach Anspruch 1, wobei der Substanzträger (40) auf der Hülle (2) angebracht ist.

3. Vorrichtung nach einem der vorhergehenden Ansprüche, wobei die zu applizierende Substanz (403) auf einem Träger in die Tasche (50) eingebracht ist.

4. Vorrichtung nach einem der vorhergehenden Ansprüche, wobei der entfernbare Bereich zumindest teilweise biodegradierbar ist.

5. Vorrichtung nach einem der vorhergehenden Ansprüche, wobei die Tasche (50) einen permeablen Bereich umfasst.

6. Vorrichtung nach einem der vorhergehenden Ansprüche, wobei der Substanzträger (40) eine Zuleitung (408) hat.

7. Vorrichtung nach einem der vorhergehenden Ansprüche, wobei der Substanzträger (40) eine schwammartige oder poröse Struktur umfasst.

8. Vorrichtung nach einem der vorhergehenden Ansprüche, wobei die Vorrichtung minimal-invasiv implantiert werden kann.

9. Vorrichtung nach einem der vorhergehenden Ansprüche, wobei die Rahmenstruktur (1) aus Draht gefertigt ist, insbesondere aus einem Draht umfassend eine Formgedächtnislegierung.

10. Vorrichtung nach einem der vorhergehenden Ansprüche, wobei die zu applizierende Substanz (403) ein pharmazeutischer Wirkstoff oder wobei die zu applizierende Substanz (403) Zellen umfasst.

11. Vorrichtung zum Implantieren der Vorrichtung gemäß einem der vorhergehenden Ansprüche, wobei die Vorrichtung zum Implantieren ein rohrförmiges Zuführsystem (9) umfasst.

12. Verfahren zum Herstellen einer Vorrichtung zur Applikation einer Substanz auf eine Herzoberfläche umfassend die Schritte:
Bereitstellen einer Rahmenstruktur (1);
Anbringen einer Hülle (2) an der Rahmenstruktur (1);
Anbringen eines Substanzträgers (40, 211); und
Beladen des Substanzträgers (40, 211) mit einer zu applizierenden Substanz (212, 403)
wobei der Substanzträger (40) eine Tasche (50) ist und die zu applizierende Substanz (403) in die Tasche (50) eingebracht ist,
**dadurch gekennzeichnet, dass** die Tasche (50) einen entfernbaren Bereich (502) auf der herzzugewandten Fläche der Tasche (50) hat.

## Claims

1. Device for the administration of a substance to a surface of the heart comprising:
a frame structure (1),
a sleeve (2),
a substance carrier (40) and
a substance to be administered (403),
where the substance carrier (40) is a pocket (50) and the substance to be administered (403) is inserted into the pocket (50), wherein the pocket (50) has a removable area (502) on the surface of the pocket (50) facing the heart.

2. Device according to Claim 1, wherein the substance carrier (40) is mounted on the sleeve (2).

3. Device according to one of the preceding claims, wherein the substance to be administered (403) is inserted into the pocket (50) on a carrier.

4. Device according to one of the preceding claims, wherein the removable area is at least partially biodegradable.

5. Device according to one of the preceding claims, wherein the pocket (50) comprises a permeable area.

6. Device according to one of the preceding claims, wherein the substance carrier (40) has a delivery line (408).

7. Device according to one of the preceding claims, wherein the substance carrier (40) comprises a spongy or porous structure.

8. Device according to one of the preceding claims, wherein the device may be implanted minimally invasively.

9. Device according to one of the preceding claims, wherein the frame structure (1) is made out of wire, in particular out of a wire comprising a shape memory alloy.

10. Device according to one of the preceding claims, wherein the substance to be administered (403) is a pharmaceutical active agent or wherein the substance to be administered (403) comprises cells.

11. Device for implanting the device according to one of the preceding claims, wherein the device for implanting comprises a tubular delivery system (9).

12. Method for producing a device for the administration of a substance onto a surface of the heart comprising the following steps:
Provision of a frame structure (1);
Mounting of a sleeve (2) on the frame structure (1);
Mounting of a substance carrier (40, 211); and
Charging of the substance carrier (40, 211) with a substance to be administered (212, 403),
where the substance carrier (40) is a pocket (50) and the substance to be administered (403) is inserted into the pocket (50), wherein the pocket (50) has a removable area (502) on the surface of the pocket (50) facing the heart.

## Revendications

1. Dispositif pour l'application d'une substance sur une surface du coeur, comprenant :
une structure-cadre (1),
une enveloppe (2),
un support de substance (40) et
une substance à appliquer (403),
le support de substance (40) étant une poche (50) et la substance à appliquer (403) étant introduite dans la poche (50),
**caractérisé en ce que** la poche (50) présente une zone amovible (502) sur la surface de la poche (50) tournée vers le coeur.

2. Dispositif selon la revendication 1, dans lequel le support de substance (40) est appliqué sur l'enveloppe (2).

3. Dispositif selon l'une quelconque des revendications précédentes, dans lequel la substance à appliquer (403) est introduite dans la poche (50) sur un support.

4. Dispositif selon l'une quelconque des revendications précédentes, dans lequel la région amovible est au moins en partie biodégradable.

5. Dispositif selon l'une quelconque des revendications précédentes, dans lequel la poche (50) comprend une zone perméable.

6. Dispositif selon l'une quelconque des revendications précédentes, dans lequel le support de substance (40) présente une conduite d'alimentation (408).

7. Dispositif selon l'une quelconque des revendications précédentes, dans lequel le support de substance (40) comprend une structure de type éponge ou une structure poreuse.

8. Dispositif selon l'une quelconque des revendications précédentes, dans lequel le dispositif peut être implanté par une technique mini-invasive.

9. Dispositif selon l'une quelconque des revendications précédentes, dans lequel la structure-cadre (1) est fabriquée en fil métallique, en particulier en fil métallique comprenant un alliage à mémoire de forme.

10. Dispositif selon l'une quelconque des revendications précédentes, dans lequel la substance à appliquer (403) comprend une substance active pharmaceutique ou la substance à appliquer (403) comprend des cellules.

11. Dispositif pour implanter le dispositif selon l'une quelconque des revendications précédentes, le dispositif d'implantation comprenant un système d'alimentation de forme tubulaire (9).

12. Procédé de fabrication d'un dispositif pour l'application d'une substance sur une surface du coeur, comprenant les étapes suivantes :
fourniture d'une structure-cadre (1) ;
application d'une enveloppe (2) sur la structure-cadre (1) ;
application d'un support de substance (40, 211) ; et
chargement du support de substance (40, 211) avec une substance à appliquer (212, 403),
le support de substance (40) étant une poche (50) et la substance à appliquer (403) étant introduite dans la poche (50),
**caractérisé en ce que** la poche (50) présente une région amovible (502) sur la surface de la poche (50) tournée vers le coeur.
